# EUROPEAN PATENT APPLICATION

(11) **EP 2 090 588 A1**
(43) Date of publication of application: **19.08.2009**
(21) Application number: 07823071.1
(22) Date of filing: 23.10.2007
(51) Int. Cl.: C07K 14/72, C12Q 1/68

(54) **IN VITRO METHOD FOR PROGNOSIS AND/OR DIAGNOSIS OF HYPERSENSITIVITY TO OOESTROGENS OR TO SUBSTANCES WITH OOESTROGENIC ACTIVITY**

(30) Priority: 23.10.2006 ES 200602692
(71) Applicant: Neocodex, S.L., 41092 Sevilla (ES)
(72) Inventor: RIVERO, María del Carmen, 41092 Sevilla (ES); SALINAS, Ana, 41092 Sevilla (ES); SAEZ GOÑI, María Eugenia, 41092 Sevilla (ES); MORÓN CIVANTOS, Francisco Jesús, 41092 Sevilla (ES); REAL, Luis Miguel, 41092 Sevilla (ES); GALÁN, José Jorge, 41092 Sevilla (ES); RUIZ LAZA, Agustín, 41092 Sevilla (ES); ROYO, José Luis, 41092 Sevilla (ES)
(74) Representative: Illescas, Manuel
(86) International application number: PCT/ES2007/070177
(87) International publication number: WO 2008/049953

(57) **Abstract**

*In vitro* method for prognosis and/or diagnosis of hypersensitivily to oestrogens or to substances with oestrogenic activity. The method is based on the detection of the presence or absence of a deletion of 2244 nucleotides at intron 6 of the human gene of the alpha oestrogen receptor *(ESRl),* which deletion, if present, signifies an increase in the probability that an individual is hypersensitive to oestrogens or to substances with oestrogenic activity, and/or the determination of the nucleotide present in the location corresponding to at least one polymorphism of said gene selected from rs851995, rs3020314 and rs910416. The invention also relates to oligonucleolides that are useful for detecíing variants in said gene, kits with said oligonucleotides that enable the method of the invention to be implemented and to the use of a variant of the *ESRl* gene that exhibits the deletion for the *in vitro* prognosis and/or diagnosis of hypersensitivity to oestrogen.

## Description

### FIELD OF THE INVENTION

This invention relates to the prognosis or diagnosis of genetic diseases the etiology whereof is based on an alteration or change in the genetic information carried by a subject, such as mutations or polymorphisms. Specifically, this invention relates to a method for the *in vitro* prognosis and/or diagnosis of hypersensitivity to oestrogens or to substances with oestrogenic activity and, therefore, to oestrogen-related diseases (ERDs), in subjects who carry said mutations or polymorphisms.

### BACKGROUND AND STATE OF THE ART

Oestrogens are a group of sexual hormones produced from androgenic precursors by the action of the cytochrome P450 aromatase protein complex. In humans, up to six different oestrogens have been described; however, only three of them: Estradiol (the most potent), Estrone and Estriol (a product of the oxidation of the other two), are found in significant quantities at the systemic level.

The cytochrome P450 aromatase complex is formed by two proteins: ubiquitous NADPH-cytochrome P450 reductase and cytochrome P450 aromatase, which contains the haemo group and the steroid-binding pocket. In humans, cytochrome P450 aromatase is expressed in several tissues: ovaries, testicles, placenta, foetal liver, adipose tissue, chondrocytes and bone osteoblasts, and numerous sites in the brain, including several areas of the Hypothalamus, the hypophysis, the limbic system and the cerebral cortex.

The classical action mechanism of oestrogens takes place through their interaction with two types of receptors, Oestrogen Receptor α (ORα) and Oestrogen Receptor β (ORβ). The binding of oestrogens to their receptors ultimately causes the modulation of the transcription of oestrogen-dependent genes (O'Donnell et al., 2001).

The classical or genomic action mechanism of oestrogens takes several hours. The ORα and ORβ receptors may be found at the cytoplasmic or nuclear level, bound to certain proteins that act as chaperones, stabilising the receptors in their inactivated state or masking the receptors' DNA-binding domain. Other proteins associated with the receptors may contribute to the interaction between different signalling pathways. When oestrogens diffuse to the cells, they bind to the receptor's ligand-binding domain. The receptor then becomes dissociated from the cytoplasmic chaperones; the oestrogen-receptor complex passes on to the cell nucleus and binds to specific DNA sequences called oestrogen response elements (EREs), such as homo- (ORα-ORα or ORβ-ORβ) or heterodimers (ORα-ORβ), to form a platform whereto co-activator complexes and transcription factors necessary for transcriptional activation bind. Oestrogens also regulate the expression of genes that do not carry EREs by the modulation of the activity of other transcription factors (O'Donnell et al., 2001; Gruber et al., 2002).

On the other hand, other "non-classical" or "non-genomic" action mechanisms have been described for oestrogens, which take place in time intervals that range from minutes to seconds. ORα and ORβ receptors that are adjacent or located in the plasma membrane, or oestrogen-binding proteins associated with the plasma membrane, participate in these alternative action mechanisms (Deroo and Korach, 2006). Examples of effects mediated by these alternative action mechanisms are rapid vasodilation of coronary arteries, the rapid insulinotrophic effect of estradiol on pancreatic beta cells and the rapid activation of growth-factor-mediated signalling pathways in neuronal cells (Gruber et al., 2002).

The main functions of oestrogens are the development of secondary sexual characteristics in women and control of the female reproductive cycle. In addition to these functions, oestrogens exhibit a large number of additional physiological activities on different tissues, in both men and women:
- Bone growth and epiphyseal closure
- Regulation of the hypothalamus-hypophysis-gonad axis
- Increased retention of Nitrogen
- Retention of sodium, chlorine and water: mineralcorticoid action
- Improved lipid profile: increase in HDL-cholesterol and triglycerides and decrease in LDL-cholesterol and alpha-lipoprotein
- Beneficial effects on the cardiovascular system: increase in prostacyclins and nitric oxide in the vascular endothelium; decreased production of Thromboxane A2 and endothelin; antioxidant action; Calcium channel blocking and Potassium channel opening.

A large amount of data obtained from clinical observations, cell cultures and animal models have supplied very valuable information about the action of oestrogens on health and diseases.

In this regard, it has been observed that there is a group of diseases the common characteristic whereof is an alteration of the levels or action mechanisms of oestrogens. This group is called Oestrogen-Related Diseases (ERDs) and includes a heterogeneous group of clinical entities, such as breast cancer, colorectal cancer, endometrial cancer, prostate cancer, osteoporosis, Alzheimer's disease, Parkinson's disease, cardiovascular diseases, obesity, endometriosis, and male and female infertility, amongst others (Morón et al., 2007).

This fact has encouraged various researchers to study the role of discrete genes related to the synthesis, the action mechanism and the metabolism of oestrogens in ERDs. For example, some genetic variants located in the ESR1 and ESR2 genes (which encode receptors ORα and ORβ, respectively) have been studied in practically all ERDs. These variants have been associated with clinical characteristics of osteoporosis, such as the risk of bone fracture or low bone mineral density in post-menopausal women, the risk of suffering breast cancer, endometriosis, Alzheimer's disease or male infertility, amongst others (Morón et al., 2007) (bttp://geneticassociationdb.nih.gov).

The ERD diseases located in the state of the art include the following:

### 1. Male infertility

The term infertility is defined by the World Health Organisation (WHO) (www.who.int) as the incapacity to achieve pregnancy after 12 months of sexual relationships without using any type of contraceptive method. It is primary infertility when the couple has never achieved a pregnancy and secondary infertility when the couple has a history of one or several pregnancies and over one year of exposure without conception elapses after the last one. According to the same organisation, sterility is a subject's permanent incapacity to have offspring.

In general, one in every ten couples experiences primary or secondary infertility, but the incidence of infertility varies between countries from less than 5% to over 30%. In 51.2% of infertile couples, the incapacity to conceive originates in the male (Tournaye, 2002).

In recent years, a considerable number of articles have been published which show a trend toward a reduction in sperm quality in several developed countries (Carlsen et al., 1992; Swan et al., 1997; Swan et al., 2000). In general, it may be affirmed that sperm quality has decreased in recent years in some countries, but not in others; that, when said decrease occurs, it is accompanied by a deterioration of the motility and the morphology of spermatozoids; and that, where sperm quality has decreased, not only a decrease therein has been observed in time, but also what is known as a "birth cohort effect", since men born in the 1940s have a higher sperm quality than those born in the 1960s (Joffe 2003).

The most recent study of the Spanish population in this regard compared the sperm quality of close to 16,000 semen samples collected between 1986-1996 and 1997-2004. A decrease from an average 244 million mobile spermatozoids per ejaculation recorded during the first period to 183 million between 1997-2004 in the second period was observed (Núñez, 2005).

In addition to the concern about the decrease in sperm quality in certain countries, the generalised implementation of Assisted Reproduction Techniques (ARTs) is another aspect to be considered in the field of reproductive medicine. It has been estimated that, in some European countries, up to 5% of all births are currently the result of ARTs (Tournaye, 2002). Furthermore, a large number of couples that were incapable of conceiving a few years ago have benefited from the appearance of new ARTs, such as Intracytoplasmatic Sperm Injection (ICSI) (Lissens, 1999).

In relation to the above, there is a great concern about the safety of ARTs, since the transmission of currently unknown genetic defects related to infertility or other pathologies cannot be discarded. Moreover, the cost/effectiveness relationship of ARTs, as well as the rate of multiple pregnancies and the economic and health consequences arising therefrom are currently the subject of intense debate (Collins, 2001; Katz et al., 2002).

As a consequence of all this, the identification of factors that cause or predispose to infertility is one of the main challenges that reproductive medicine currently faces.

Male infertility may be the result of congenital or acquired urogenital anomalies, infections in the male accessory glands, increased scrotal temperature, endocrine disorders, genetic anomalies and immunological factors (WHO, 2000). However, in 40%-60% of infertile males, the only detectable anomalies are found in the main seminal parameters, i.e. the concentration, the mobility and/or the morphology of spermatozoids, whereas no alterations are identified in physical examinations or endocrine tests. Subjects who have these characteristics are said to suffer from idiophathic infertility (Dohle et al., 2005).

In this regard, it is worth stressing that infertility is a term that refers to both members of the couple, whereas oligozoospermia (small number of spermatozoids) and azoospermia (absence of spermatozoids) are specific male characteristics. Therefore, both terms are not synonymous, although it is evident that a subject with a low sperm count will be more likely to be infertile than a normozoospermic subject (one with a normal number of spermatozoids).

Idiopathic infertility is a complex disease that results from the interaction between environmental and genetic factors. Currently, a large number of exogenous products with harmful effects on male reproductive capacity have been identified in both animal models and humans (Joffe, 2003; Lottrup et al., 2006). However, although a considerable number of genetic factors responsible for infertility have been identified in animal models (Cooke et al., 2002), the search for genetic factors that predispose to male idiopathic infertility in humans has been less fruitful.

The most common genetic cause of oligozoospermia and azoospermia (low or zero sperm concentration) are microdeletions in the Y chromosome (Krausz and Degl'Innocenti, 2006). These microdeletions are responsible for the total or partial elimination of the azoospermia regions (AZFa, AZFb and AZFc) located in the long arm of the Y chromosome. This notwithstanding, 85% of azoospermic subjects and 90% of severe oligozoospermic individual do not exhibit said alterations in the Y chromosome (Krausz et al., 2003).

Most genetic association studies published thus far, the purpose whereof is to identify genetic factors that predispose to male idiopathic infertility, have followed a candidate gene strategy. This strategy is based on the selection of genes whose function, expression profile, chromosomal location or involvement in related pathologies make them appealing to be studied in relation to the disease of interest.

In this regard, one of the genes that has been considered by some as a candidate of interest in the study of male infertility is the Oestrogen Receptor Gene (ESR1). This gene encodes Oestrogen Receptor alpha, which is one of the key pieces in the physiological action of oestrogens in both men and women.

Oestrogens play a very important role in the function of the male reproductive tract in humans. For example, Estradiol facilitates the negative feedback mechanism exerted by Testosterone on the Hypothalamus-hypophysis-testicle axis, participates in testicular descent in the foetal age, in the modulation of the function of Leydig cells (the testosterone-producing cells located in the testicles) and Sertoli cells (whose function is to protect, feed and sustain immature spermatozoids until they are released into the tubules), and, furthermore, seems to exert an anti-apoptotic function on the germ cells (O'Donnell et al., 2001).

On the other hand, the disruption of the Esr1 gene in mice leads to infertility in the male, due to the lack of fluid reabsorption in the efferent ducts, which induces a progressive degeneration of the seminiferous tubules (Hess et al., 2000).

In humans, only one case of oestrogen insensitivity has been described that is caused by a homozygosis mutation in exon 2 of the ESR1 gene (Smith et al., 1994). This mutation was found in a male subject the most significant characteristics whereof were incomplete epiphyseal closure at 28 years of age, glucose intolerance with hyperinsulinaemia and bone mass reduction. This subject exhibited normal secondary sexual traits, eugonadism (normal gonadal function, which is generally identified on the basis of a normal testosterone concentration in men), high levels of estradiol, Follicle-stimulating hormone and Luteinising hormone, and normal Testosterone levels. This subject's sperm count is considered to be normal (25 million spermatozoids per millilitre of ejaculated semen), although the spermatozoids had a low viability (18% versus the 50% reference value) (Smith et al., 1994).

Studies of the genomic sequence of the ESR1 gene have made it possible to identify a large number of Single Nucleotide Polymorphisms (SNPs) in this gene. For example, GenBank's public SNP database (www.ncbi.nlm.nih.gov) includes over 1,300 polymorphisms located in the genomic region of ESR1, some of which are discussed below, referring thereto with the name used in said database.

Some of these polymorphisms have been used as markers in various genetic association studies on various oestrogen-related diseases, such as Alzheimer's disease, Parkinson's disease, multiple sclerosis, coronary disease, rheumatoid arthritis, serum LDL levels, bone mass index, precocious menopause, breast cancer, prostate cancer, endometrial cancer and male infertility, amongst others (geneticassociationdb.nih.gov).

Furthermore, different polymorphic variants of the ESR1 gene have been disclosed, as well as methods for the detection thereof, and their use to prognosticate some diseases or the body's response following the administration of various drugs. Patent application US 2002/0187495, for example, discloses the association between ESR1 polymorphisms and the body's favourable response to hormone replacement therapy designed to control hypercholesterolaemia. On the other hand, patent US5834200 mentions the relationship between polymorphisms in said gene and the predisposition to develop osteoporosis. Patent applications JP2000300295 and JP2001333798 relate to probes and methods designed to detect ESR1 polymorphisms, evaluate the sensitivity of a subject with said polymorphisms to an osteoporosis therapeutic agent and, on the basis thereof, decide the most adequate drug to be supplied. Application US 2002/0123095 mentions more diseases wherein oestrogens might be involved, including cardiovascular diseases, erythematous lupus, arthritis, osteoporosis, Alzheimer's disease and breast cancer, and proposes the detection of SNPs in the ESR1 gene to identify subjects with the risk of developing these diseases. None of these documents mentions a possible association between ESR1 variants (said variants being considered to be SNPs and/or polymorphisms) and infertility, or which variants could be involved in the development of said disorder.

Patent application JP2006061128 does refer to the use of genetic markers located in the human ESR1 gene for the "evaluation of the sensitivity to multifactorial diseases in sexual differentiation", which are diseases that evolve with anatomic-physiological alterations, such as the development of micropenises, the absence of testicular descent or the development of a vulva in males, i.e. alterations which, when they cause infertility, it would not be considered to be idiopathic infertility. The inventors cited in said application also published an article where they proposed the association of the markers whereto said application relates with the development of cryptorchidism in the Japanese population (Yoshida et al., 2005). However, the markers cited in both cases are, once again, single-nucleotide polymorphisms (SNPs), not deletions that affect more than about ten base pairs.

Cryptorchidism is a developmental defect characterised by the absence of the descent of one or both testicles to the scrotum. Cryptorchidism is a susceptibility factor for male infertility, since between 2% and 9% of infertile males have a history of cryptorchidism (Baccetti et al., 2002), and, therefore, these cases are not considered to be idiopathic.

Given the relationship between cryptorchidism and male infertility, the group of inventors of this invention developed a study which analysed whether there was an association of five of the SNP markers (rs926779, rs3020364, rs6932902, rs3020371 and rs3020375) included in Yoshida et al. (2005) with cryptorchidism in the Italian population, and with infertility in the Italian population and the Spanish population (Galán et al., 2006). The results obtained did not coincide with those presented by Yoshida et al. (2005), since the combination of these five markers (haplotype) that entailed a risk factor for cryptorchidism in the Japanese population acted as a protective factor against cryptorchidism in the Italian population. Moreover, none of these five markers or the combination thereof are associated with male idiopathic infertility in the Italian or the Spanish population (Galán et al., 2006).

On the other hand, in a total of four studies performed on four independent populations, polymorphisms located in the ESR1 gene were identified which are associated with male idiopathic infertility:
1. Suzuki et al. (2002) studied the rs1801132 polymorphism located in exon 4 of the ESR1 gene in a group of 31 idiopathic azoospermic subjects and in a group of fertile subjects; both groups comprise males in the Japanese population. These authors detected a genetic association between the polymorphic G allele of the rs1801132 marker and idiopathic azoospermia in the Japanese population, since the frequency of said allele was higher in azoospermic patients than in fertile males.
2. Kukuvitis et al. (2002) performed a genetic association study which analysed a group of 64 fertile males (group A) and three groups of infertile subjects: 29 idiopathic infertile males with moderate oligozoospermia (sperm counts between 10 and 20 million spermatozoids per millilitre of ejaculated semen) (group B); 42 idiopathic infertile males with severe oligozoospermia (less than 10 million spermatozoids per millilitre of ejaculated semen) or azoospermia (group C); and 38 non-idiopathic infertile males (group D). All the subjects included in the study belonged to the general Greek population. Kukuvitis et al. (2002) analysed three markers, two SNPs located in intron 1 of the ESR1 gene (rs8179176 and rs9340799) and a microsatellite marker located in the amino-terminal end of the *Androgen Receptor (AR)* gene [OMIM:313700] which consisted of a variable number of repeats of trinucleotide CAG. The authors did not find any statistically significant differences when they compared the allele or genotype frequencies of the rs8179176 marker between group A and the three groups of infertile subjects. However, Kukuvitis et al. (2002) observed that the G allele of the rs9340799 marker appeared in a statistically higher frequency in group C of patients than in group A; for this reason, they concluded that this marker had a genetic association with idiopathic infertility in Greek severe oligozoospermic or azoospermic subjects. On the other hand, the same authors did not find any differences between the means or the range of the number of CAG repeats in the microsatellite marker of the AR gene, although the frequency of alleles with more than 30 CAG repeats was greater in each of the three infertile groups as compared to group A.
3. Guarducci et al. (2006) studied the number of repeats of dinucleotide TA in a microsatellite marker located at the 5'-end of the ESR1 gene in a group of 191 males with idiopathic infertility and in a group of 156 fertile subjects. Both groups belonged to the general Italian population. The authors discovered a correlation between a greater number of TA repeats in the microsatellite marker and lower sperm counts in the group of infertile subjects. Moreover, the presence of alleles with more than 20 TA repeats in said marker, in homozygosis or in heterozygosis, was associated with lower sperm counts in the group of fertile subjects.
4. This group of inventors also performed a genetic association study which analysed the individual and the joint role of five genes involved in the synthesis or the action mechanism of oestrogens in male idiopathic infertility in the Spanish population (Galán et al., 2005), one of which is the ESR1 gene. The results obtained from said study support the idea that the ESR1 gene plays a role in male idiopathic infertility in the Spanish population:
   - The rs8179176 polymorphism, located in intron 1 of the human ESR1 gene, was analysed in a group of 104 male subjects with idiopathic infertility (case group) and in another group composed of 95 unrelated subjects from the same population (control group). Although the frequency of the rs8179176 polymorphism was not statistically different in both groups, the study of the Hardy-Weinberg equilibrium (HWE) (Hardy, 1908; Weinberg, 1908) in these groups showed an excess of homozygotic subjects for rs8179176 in the case group, and not in the control group (Galán et al., 2005). The moderate HWE deviations observed in the case group and not in the control group may be indicative of the association of the gene under study with the disease under study (Hoh et al., 2001).
   - An HWE deviation was also identified for the rs8179176 marker in group C of infertile Greeks studied by Kukuvitis et al. (2002). This HWE deviation is also due to an excess of homozygotic subjects for the rs8179176 marker (Galán et al., 2005).
   - The analysis of a microsatellite marker with a high heterozygoticity, located 35 Kb from the rs8179176 marker, also showed a greater number of homozygotic subjects in the case group than in the control group for the Spanish population. However, the allele and genotype frequencies, and the allele distribution of said marker, are not different in both groups (Galán et al., 2005). This marker is the same that would be subsequently studied by Guarducci et al., (2006) in the Italian population.
   - The multi-locus analysis of the oestrogen pathway indicates that the ESR1 gene could have a dominant role in male infertility (Galán et al., 2005).

Therefore, those studies wherein the variants of the ESR1 gene were related to male infertility focused on the analysis of the SNPs located in said gene and the repeats in microsatellites and their potential relationship with infertility. Thus far, no associations had been established between deletions in said gene and male idiopathic infertility. Given the existing interest in identifying the factors that cause infertility or predispose thereto, the group of inventors proposed the hypothesis that the ESR1 gene may contain a genetic susceptibility factor for male idiopathic infertility. Identifying the nature of this factor, the frequency thereof in infertiles subjects and in the general population, and its potential utility in diagnosing the predisposition to suffer from male idiopathic infertility were the main objectives in the development of this invention.

### 2. Response to Controlled Ovarian Hyperstimulation (COH)

Controlled Ovarian Hyperstimulation (COH) is a procedure designed to achieve a sufficient oocyte quantity and quality, and thus improve pregnancy rates in different assisted reproduction techniques. The induction of ovulation is an important option in reproduction, since it allows for a therapeutic approach to the most frequent causes of female infertility in developed countries. The study of its action mechanism increased the knowledge about the physiology of the "hypothalamus-hypophysis-ovary" axis and, in particular, of hormonal control mechanisms.

Currently, there are various protocols to develop COH, which normally include three stages:
- Hypophyseal braking: The administration of gonadotropin-releasing hormone agonists (GnRHa), such as Triptorelin, allows for the biochemical ablation of the "hypothalamus-hypophysis-ovary axis", thereby suppressing the production of ovarian oestrogens.
- Stimulation of Follicle-genesis: it is performed by the administration of drugs with gonadotrophic effects, such as human menopausal gonadotropin (hMG), highly purified urinary Follicle-Stimulating hormone (FSH) or recombinant FSH (obtained by genetic engineering).
- Stimulation of ovulation: It may be achieved by the administration of drugs with an activity similar to that of human chorionic gonadotropin (hCG).
- The selection of the different existing protocols must be designed on the basis of each patient's needs. However, several studies have shown a great variability in clinical results in patients subject to COH treatment, which depend on factors such as age and ovarian follicle reserve (Kligman and Rosenwaks, 2001).

Hundreds of studies performed in the past decade research, propose and evaluate numerous biochemical and epidemiological markers in relation to the response to COH, and these studies focus on the main risks that appear in these therapeutic procedures in order to prevent them. Amongst the main risks that may appear in a COH protocol, we find:
- A low or null oocyte response, which leads to cancellation of the treatment, with the consequent risk for the patient and the enormous economic cost. One of the main biomarkers of low response to COH is related to the ovarian reserve of the women treated (Tarlatzis et al., 2003).
- Multiple pregnancy (more than two foetuses) is a serious complication, which entails physical risks (uterine overdistension, infection, premature breaking of the membranes, polyhydramnia, cervical incompetence and even foetal prematureness) and psychic risks, in addition to high medical and social costs.
- The ovarian hyperstimulation syndrome (OHS) is an iatrogenic complication triggered after the exogenous administration of drugs in a COH cycle which primarily occurs in the luteal stage of the cycle or at the beginning of pregnancy. Although it is generally considered to be an iatrogenic symptomatology caused by the use of gonadotropins to induce follicular development (Forman et al., 1990), some cases have been described in natural cycles (Zalel et al., 1992). According to different publications, the incidence of OHS varies from 0.6%-14%, and the incidence of the severe stage is estimated to be approximately between 0.5%-5% (Delvigne and Rozenberg, 2002), depending on the identification criteria used. OHS exhibits different degrees of severity; and some lethal cases have even been described (Semba et al., 2000).

Currently, there not many data about the genetic markers involved in the variability of the response to COH. Most of these studies have been performed on the different genes related to follicle-genesis and the production of oestrogens (de Castro et al., 2005b; Greb et al., 2005).

No patents have been identified which include any indication of the identification of genetic factors as predictive factors for the response to COH.

### 3. Couple infertility

As previously discussed, the term "infertility" refers to both members of the couple, since the fertility status of one of the members of the couple decisively influences the other member's reproductive capacity. A mild degree of infertility is not necessarily associated with couple infertility when it appears in only one of the members, but it may reduce the couple fertility when it appears in both members (Dohle et al., 2005). However, the terms "female infertility" or "male infertility" are frequently used when addressing the reproductive capacity of a single subject.

Infertility may be classified into four categories on the basis of its origin: female factor when the cause of infertility is found in the woman (35%), male factor when the couple infertility is due to the male (30%), combined factors when anomalies associated with infertility are detected in both members of the couple (20%) and idiopathic infertility when no diagnosis can be provided following a complete examination of both members of the couple (15%) (Forti and Krausz, 1998).

A large number of studies have been published which warn about a reduction in the reproductive capacity of men and women caused by environmental factors, such as substances with a deleterious activity on the endocrine system (Joffe, 2003), or social factors, such as the generalised increase in the age at which women have their first child (te Velde and Pearson, 2002).

The diagnosis of infertility is usually performed by taking into account each member of the couple separately. Thus, the woman's medical history is usually recorded and she is subject to a physical examination, hormonal evaluation of the ovarian function, evaluation of the tubaric capacity, evaluation of cervical factors, sperm-cervical mucus interaction, examination of anomalies in the uterus and genetic tests. The man's medical history is also recorded, and a physical examination is performed, jointly with the analysis of semen samples, hormonal measurements, bacteriological examination and genetic tests (Forti and Krausz, 1998).

Currently, there are no diagnostic methods that evaluate infertility at the couple level based on the molecular analysis of discrete factors and which are applied in clinical practise.

A patent has been published which exposes the future mother to a Transforming Growth Factor beta (TGF-beta) and an antigen from the couple in order to cause a reduction in the future mother's immune response to one or several antigens from the father. US7204978, EP1743649, AU6284698: "Treatment and diagnosis of infertility using TGFbeta or activin".

### 4. Osteoporosis

The World Health Organisation (WHO) defines osteoporosis as: "Systemic disease characterised by a reduction in bone mass and a deterioration of the bone tissue's microscopic architecture, which leads to increased fragility and a consequent increase in the susceptibility to bone fractures" (www.who.int).

Osteoporosis is due to an increase in bone resorption, which is associated with an oestrogen deficiency. Oestrogens regulate skeletal homeostasis in men and women by a reduction in osteoclast-mediated bone resorption and an increase in osteoblast-mediated bone formation. Both oestrogens and raloxifene (a selective modulator of oestrogen receptors) are currently prescribed for the prevention of osteoporosis in post-menopausal women (Herynk and Fuqua, 2004).

The role of the ESR1 gene in the development of osteopaenia and in skeletal growth has been demonstrated in the clinical description of a male patient with oestrogen resistance caused by a mutation in said gene. This patient showed incomplete epiphyseal closure and reduced bone mineral density at 28 years of age (Smith et al., 1994).

Numerous studies have analysed the influence of genetic ESR1 variants (specially the PvuII and Xbal polymorphisms included in intron 1 of said gene) on various clinical features related to osteoporosis (Ioannidis et al., 2004; Albagha et al., 2005; Choi et al., 2005; Morón et al., 2006; Castellani et al., 2006; Rivadeneira et al., 2006), which supports the hypothesis that these and other variants of the ESR1 gene have a real effect on the development of this disease.

Osteoporosis is usually diagnosed by measuring a patient's bone mineral density by densitometry, which is a radiographic diagnostic method. However, various patents have been published which propose the analysis of variants located in discrete genes as diagnostic methods for osteoporosis, as in the case of:
- WO0216553, related to the Bone Strength and Mineralisation Regulatory Protein (BSMR) gene
- US5922542, related to the Collagen-alpha Type I gene (COL1A1)
- US5998137, related to the Transforming Growth Factor beta gene (TGFB)

Patent KR20030065205, titled "Method for measurement of osteoporosis risk factor by measuring polymorphism of genes involved in osteoporotic process and kit for diagnosis of genetic risk factor of osteoporosis", proposes the study of variants in the Leptin Receptor (LEPR), the Vitamin D Receptor (VDR), the Transforming Growth Factor beta 1 (TGFB1) and the ESR1 genes for the genetic diagnosis of osteoporosis. The variants of the ESR1 gene included in this patent are rs8179176 (PvuII) and rs9340799 (Xbal), both located in the first intron of this gene.

### 5. Breast cancer

Breast cancer is the main cause of death in women with solid tumours. The estimated annual incidence of breast cancer is approximately 200,000 in the USA and 320,000 in Europe (Dumitrescu and Colarla, 2005). Data obtained from clinical observations and animal model studies have demonstrated the involvement of oestrogens in the development of breast cancer.

Oestrogens perform physiological actions on the mammary tissue which are currently well defined. On the one hand, they stimulate the growth and differentiation of the breast duct epithelium, inducing mitotic activity in the cylindrical duct cells and the growth of breast connective tissue. Oestrogens exert effects similar to those of histamine on microcirculation in the breast (Gruber et al., 2002).

Currently, there are two hypotheses that explain the relationship between oestrogens and the development of breast cancer. The first proposes that the binding of oestrogens to their receptors stimulates the proliferation of mammary cells, thereby increasing cell division and DNA synthesis, which increases the risk of errors during the DNA replication process and, in turn, may lead to the acquisition of mutations that alter cell processes such as apoptosis, cell proliferation or DNA repair. The second hypothesis proposes that the oestrogen metabolism leads to the production of genotoxic products that may directly damage the DNA, resulting in occasional mutations. There is evidence that oestrogens may act through mechanisms that initiate or promote breast cancer (Herynk and Fuqua, 2004).

Some epidemiological studies from the 1990s disclosed that both a late menarche age and a precocious menopause were associated with a reduced risk of suffering breast cancer. Thus, an additional source of circulating oestrogens, either exogenous or endogenous, coud increase the risk of developing breast cancer. One example is the result obtained by Dr. Huang et al., which associated women's increased weight in the adult age to an increased risk of suffering breast cancer, since adipose tissue is an endogenous source of oestrogens (Huang et al., 1997).

It has been proposed that the ESR1 gene is directly involved in the development of the mammary gland and in breast cancer. Adult female mice lacking the Esr1 gene (ERKO) have mammary glands similar to those of normal female mice of a prepuberal age, with the absence of development of the ducts and the mammary buttons. On the other hand, in murine models with breast tumours with a long latency period, the absence of the Esr1 gene does not increase the incidence of tumour development. In other murine models, with a constitutive mammary expression of the Neu oncogene, ERKO females developed breast tumours, but with a late beginning (Deroo and Korach, 2006). In other murine models of oestrogen-promoted breast tumourigenesis, the incidence of breast tumours is minimised with the loss of the Esr1 gene (Yoshidome et al., 2000).

In humans, some previously characterised variants of the ESR1 gene are associated with a differential risk of suffering breast cancer (Yaich et al., 1992; Shin et al., 2003; Wedren et al., 2004; Boyapati et al., 2005; Onland-Moret et al., 2005; Shen et al., 2006; González-Mancha, submitted). Various patents have also been published which include the analysis of variants of the ESR1 gene for the molecular diagnosis of breast cancer:
- US2006166231, WO2006052731: Molecular indicators of breast cancer prognosis and prediction of treatment response
- WO2006004597: Association of breast cancer DNA methylation profiles with hormone receptor status and response to tamoxifen
- WO2004065545, US2004058340, WO02103320: Diagnosis and prognosis of breast cancer patients
- W02005033336: Materials and methods relating to breast cancer diagnosis
- US2003186313, WO02057283: Methods and compositions in breast cancer diagnosis and therapeutics
- US2006154267: Diagnosis and treatment of breast cancer

However, these variants alone do not explain all breast cancer cases, which suggest that there are more variants located in the ESR1 gene or in other genes of the oestrogen pathway that may condition the appearance of the disease.

Currently, there are numerous diagnostic techniques available for breast cancer, such as mammography (which is an examination that uses low-potency X-rays to detect anomalous zones in the breast), echography (a technique that uses ultrasounds which are converted into images and is useful to distinguish liquid tumours or solid tumour cysts), Nuclear Magnetic Resonance (NMR) (it uses magnetic fields and the spectra emitted by phosphorus in body tissues and converts them into images, which makes it possible to observe the tumour vascularisation), Positron Emission Tomography (PET) (which consists of injecting a radiodrug combined with glucose that is captured by cancerous cells, should a cancer exist, since they consume more glucose; the radiodrug makes it possible to locate the areas where the tumour is found) or biopsy (extraction of tissue for the subsequent evaluation thereof). Despite the developments in the early detection of breast cancer using these techniques, there is still a great interest in distinguishing those population groups that, due to their genetic condition, have a higher risk of suffering breast cancer. For this reason, identifying genetic patterns that confer susceptibility to the disease may become a very useful healthcare tool to conduct prevention campaigns in the general population.

Once the tumour has developed, molecular assays are usually performed of the tissue extracted in the biopsy in order to detect the presence or absence of specific molecules which allow the physician to perform a molecular diagnosis and estimate the prognosis of the disease, as well as select the treatment that is most suitable for the patient. Currently, the HER-2, p53 and Ki67 molecules, and the progesterone and oestrogen receptors are routinely analysed. These analyses, jointly with the tumour size, the lymph node condition and the histological grade of the tumour allow the clinician to classify and treat the cancer. In particular, the tumour's oestrogen receptor content is a known favourable prognostic factor for breast cancer, due to its important role as a mediator of hormonal response, such as proliferation in oestrogen-sensitive tissues. However, it is well known that tumours which are apparently identical from the immunohistochemical standpoint may respond to the same treatment in a differential manner. Therefore, it is relevant to find genetic patterns that may predict which subgroup of patients should be treated with alternative methods, both in order to ensure the effectiveness of the treatment and to prevent the undesirable effects thereof.

On the other hand, two genes have been identified: Breast Cancer 1 (BRCA1), the mutations whereof are responsible for breast cancer in 45% of families with breast cancer, and in 80% of families with breast and ovarian cancer; and Breast Cancer 2 (BRCA2), which is responsible for 35% of breast cancers in families with multiple cases of this disease. However, it should be stressed that over 90% of breast cancer cases do not have a clear hereditary component; therefore, this disease follows a polygenic behavioural model.

Other patents the object whereof are molecular diagnostic methods for the diagnosis of breast cancer are:
- FR2882754; Detecting cancer cells for diagnosis of breast cancer, comprises detection of expression differences of protein kinase C epsilon and reduction in association of LIV 21 with E2F4.
- US2005019782: Diagnostic test kit for determining a predisposition for breast and ovarian cancer, materials and methods for such determination.

### 6. Blood hipertensión

Oestrogens have been traditionally associated with a cardioprotective effect, since they improve the lipid profile at the systemic level and cause an increase in prostacyclins and nitric oxide in the vascular endothelium; decreased production of Thromboxane A2 and endothelin; antioxidant action; Calcium channel blocking and Potassium channel opening.

Blood hypertension (BH) is the most common cardiovascular disease, with a prevalence of close to 27% worldwide. BH is a risk factor for infarctions, heart diseases and kidney diseases. High blood pressure is a complex, multifactorial disease that results from the interaction of multiple genetic and environmental factors. Genetic factors explain between 30% and 50% of the interindividual variability of blood pressure (BP) (Levy et al., 2000; Laiouel, 2003; DeStefano et al., 2004).

Sexual dimorphism in the regulation of BP has been demonstrated in several population studies (Burt et al., 1995; DeStefano et al., 2004) and in animal models (Reckelhoff, 2001). The age-adjusted BP is higher in men than in women, but these differences decrease when women undergo menopause. These findings suggest the presence of different mechanisms for the regulation of BP in men and women, which stresses the importance of sexual hormone levels in the determination of BP.

For example, inhibitors of the CYP19 aromatase enzyme, responsible for the production of estradiol, exhibit antihypertensive effects in animals with genetic and experimental hypertension (Griffing et al., 1991; Melby et al., 1993). Therefore, variants in genes that participate in the synthesis, the action mechanism or the metabolism of oestrogens may play a significant role in the regulation of BP.

Patent WO8808457 includes the analysis of several variants of the ESR1 gene as a prognostic method for the development of hypertension and osteoporosis.

Other patents that include the analysis of genetic variants as methods for the diagnosis and/or prognosis of hypertension are the following:
- JP2007053995: "Method for judging essential hypertension", affects polymorphisms included in the Follicle-Stimulating Hormone Receptor (FSHR).
- CN1912137: "Method for testing GRK4 gene polymorphic related to human essential hypertension", affects the Ala486Val polymorphism of the G-protein-coupled receptor kinase 4 (GRK4).
- WO2004070057: "Use of a novel polymorphism in the hsgk1 gene for the diagnosis of hypertension and use of the sgk gene family for the diagnosis and therapy of the long q/t syndrome", includes the analysis of the human homologues of the serum/glucocorticoid regulated kinase (sgk) gene family.
- JP2006101790, JP2006101789, US2006099594: "Method for evaluating risk of hypertension", includes the analysis of genetic polymorphisms, but does not specify the names of the genes.
- JP2006067902: "Diagnosis of hypertension risk using combination of genetic polymorphism and lifestyle", includes the analysis of genetic polymorphisms, but does not specify the names of the genes.

### 7. Metabolic Syndrome

The Metabolic Syndrome (MS) is defined as the aggregation of multiple metabolic alterations in a subject, which entail a significant increase in the risk of cardiovascular diseases.

Recently (April 2005), the International Diabetes Federation (IDF) has published a definition of MS reached by consensus from experts around the world (Alberti et al., 2005). In this new definition, the main criterion is the presence of abdominal obesity, defined as a waist circumference > 94 cm in men and 80 cm in women. Moreover, two of the following requirements must be met:
- Triglycerides >150 mg/dl or under specific treatment.
- HDL-cholesterol < 40 mg/dl in men, 50 mg/dl women or under specific treatment.
- Blood hypertension: Systolic blood pressure ≥130 mmHg or Diastolic blood pressure ≥ 85 mmHg, or under antihypertensive treatment.
- Baseline glucose ≥ 100 mg/dl or DMII diagnosis.

The influence of oestrogens on MS is demonstrated by the involvement of these hormones on the clinical characteristics that define the syndrome itself. An example of this is the description of a subject with a deficiency in the CYP19 aromatase gene. This subject showed MS, characterised by abdominal obesity, hyperinsulinaemia, acantosis nigricans and non-alcoholic fatty liver disease (Maffei et al., 2007). This observation shows the role of oestrogens in the etiopathogeny of MS.

On the other hand, a recent study has been published that proposes the association of variants of the ESR1 gene (PvuII and Xbal) with the risk of suffering myocardial infarction in patients with MS (Lazaros et al., 2007).

The only patent located that proposes the analysis of genetic polymorphisms as a diagnostic method for the Metabolic Syndrome is JP2007054002, "Diagnosis and prevention of metabolic syndrome", which comprises the analysis of variants in the Angiopoietin-1 gene (ANGPT1).

This invention, for the first time, and surprisingly, relates the presence or absence of a deletion in the ESR1 gene with oestrogen hypersensitivity. To this end, in this invention, it has unexpectedly been observed that the presence of the ESR1-NCD1 deletion is directly related to endometrial growth, in response to oestrogens, in female carriers thereof. Endometrial growth is a known test that is equivalent to the uterotrophic tests used in animal models to detect xenooestrogenic agents, since the proliferation of endometrial cells is completely dependent on oestrogen: estradiol. Likewise, oestrogens regulate both the female and male reproductive tracts, for which reason a mutation such as that described in this invention determines oestrogen hypersensitivity in female carriers and, when it is detected in men, said hypersensitivity is directly related to male idiopathic infertility. Similarly, the predisposition to suffer a number of oestrogen-related diseases, which include osteoporosis, breast cancer, couple infertility, blood hypertension or metabolic syndrome, is determined, as shown for the first time in this invention, by analysing the presence or absence of the ESR1-NCD1 deletion in subject samples.

### DESCRIPTION OF THE INVENTION

This invention relates to a method for the *in vitro* prognosis and/or diagnosis of hypersensitivity to oestrogens or to substances with oestrogenic activity and, therefore, to ERD diseases, by identifying a variant of the human oestrogen receptor alpha gene (ESR1) in the genome of a subject who carries it.

More specifically, the invention relates to a method to determine the existence, in the DNA of the subject under study, of a 2,224-nucleotide deletion located in intron 6 of said gene and/or to determine the nucleotide present in the location pertaining to at least one polymorphism, selected from the rs851995, rs3020314 or rs910416 polymorphisms of the ESR1 gene, or the rs4986938 polymorphism of the ESR2 gene. Likewise, the invention also relates to the oligonucleotides that are useful to detect variants in said gene, kits that comprise oligonucleotides which facilitate performing the method of the invention and the use of a variant of the ESR1 gene that exhibits the deletion of 2,244 nucleotides in intron 6 in order to manufacture said kits.

This invention expresses the results of the analysis of the ESR1-NCD1 variant in the above-mentioned oestrogen-related diseases, and shows that said deletion causes increased sensitivity to oestrogens and substances with oestrogenic capacity in subjects who carry said variant in their DNA. Therefore, this invention proposes a method for the *in vitro* prognosis and/or diagnosis of oestrogen-related diseases, by identifying the presence of the ESR-NCD1 deletion in the subject's genome.

Thus, male idiopathic infertility is considered to be an oestrogen-dependent disease and, therefore, this invention provides the use of a genetic variant of the human ESR1 gene, called ESR1-NCD1, characterised by the presence of a 2,244-nucleotide deletion located in intron 6 of said gene, to prognosticate and/or diagnose human male idiopathic infertility and/or hypersensitivity to oestrogens or substances with oestrogenic activity *in vitro,* either because the presence of said variant is determined independently, or because the nucleotide present in the location pertaining to at least one polymorphism of the ESR1 gene selected from rs851995, rs3020314 or rs910416, or the rs4986938 polymorphism of the ESR2 gene, is additionally determined.

The identification of the 2,244-nucleotide deletion located in intron 6 of the human ESR1 gene is completely innovative, since there are no previously published works, nor any public-access databases, which specify the existence thereof.

As previously mentioned, one aspect of this invention is based on the diagnosis and/or prognosis of male idiopathic infertility, since the human ESR1 gene decisively influences sperm production in males. The presence of the deletion whereto this invention relates, a 2,244-nucleotide deletion in intron 6 of the ESR1 gene, in males would entail a predisposition to a reduction in the production of spermatozoids; therefore, these subjects would be more likely to be infertile. Consequently, another object of the invention is a method for the *in vitro* prognosis and/or diagnosis of the predisposition to suffer from human male idiopathic infertility, which comprises isolating a genomic DNA sample from the subject and determining the presence or absence therein of at least one variant of the oestrogen receptor alpha gene (ESR1), selected from:
a) at least one allele carrying the ESR1-NCD1 deletion in intron 6 of said gene;
b) at least one allele carrying the form of the rs851995 polymorphism wherein the nucleotide located in the position pertaining to said polymorphism is that which has adenine as the nitrogenated base;
c) at least one allele carrying the form of the rs3020314 polymorphism wherein the nucleotide located in the position pertaining to said polymorphism is that which has cytosine as the nitrogenated base;
d) at least one allele carrying the form of the rs910416 polymorphism wherein the nucleotide located in the position pertaining to said polymorphism is that which has thymine as the nitrogenated base.

It is particularly preferable that at least the presence or absence of the ESR1-NCD1 deletion be determined.

The determination of the presence or absence of the ESR1-NCD1 deletion may be performed by the analysis of fragments of the ESR1 gene, previously amplified by PCR using primers and time and temperature conditions that allow for the amplification of a fragment that comprises all or part of the 2,244 nucleotides of the ESR1-NCD1 deletion from the normal allele, and also allow for the amplification of some fragment of the sequence of the ESR1 gene from the allele carrying the deletion. The primers and the amplification conditions will be selected in such a way that the sequences of the fragments amplified from the normal allele and the allele carrying the deletion are different, but the difference may lie in the fact that the fragment amplified from the allele carrying the deletion is identical to the fragment amplified from the normal allele, except in that the former lacks the nucleotides pertaining to the deletion area, or it is also possible, as explained further below, that the primers and the conditions are selected in such a way that there are other differences in the sequences of the fragments amplified from one or the other allele.

A possibility for the PCR design is to select the primers and the reaction conditions in such a way that the fragment amplified from the normal allele comprises the area pertaining to the entire deletion and, therefore, has a size 2,244 pb larger than the fragment amplified from the allele with the deletion. This may be achieved by using a pair of primers the respective binding sites whereof are located in such a way that the area pertaining to the deletion is between them, i.e. the binding site of one of the primers is closer to one of the endpoints of the deletion, whereas the binding site of the other primer is closer to the opposite endpoint of the deletion. Regardless of this condition, in order for the amplification to be possible, each primer must bind to a different chromosome strand and will be selected in such a way that the orientation thereof allows for the amplification of the chromosome fragment located between them.

Discrimination between the PCR products amplified from the normal allele or from the allele carrying the deletion, taking advantage of the difference in size, may be performed after subjecting them to conventional electrophoresis, such as, for example, in polymerised agarose gels on flat surfaces, wherein the position of the bands is detected by staining with ethidium bromide. In this case, in order to facilitate the detection and discrimination of both amplified fragments, it is preferable to select the amplification primers in such a way that the allele carrying the deletion leads to fragments with a size of at least 100 base pairs, in order to facilitate the detection of the band pertaining to the fragment stained with ethidium bromide in the gel, but that the size does not exceed the order of tens of kilobases, in order to facilitate separation from the band pertaining to the fragment amplified from the normal allele and the discrimination between both bands. The region whereto the 4,800-pb SEQ ID NO:81 pertains, comprised between the areas complementary to oligonucleotides A1 (SEQ ID NO:37) and A2 (SEQ ID NO:40), which comprises the area wherein the deletion may appear, is a suitable area for the selection of the oligonucleotides to be used as primers. Thus, for example, oligonucleotide ED-F (SEQ ID NO:46) may be used as a direct amplification primer, combined with different amplification primers that bind to areas close to the deletion area, but at the endpoint opposite to oligonucleotide ED-F (SEQ ID NO:46), such as reverse primers R2 (SEQ ID NO:44), R3 (SEQ ID NO:45) or A2 (SEQ ID 5 NO:40), which lead, respectively, to 239-pb (∼0.3 kb), 809-pb (∼0.8 kb) and 1,696-pb (∼1.7 kb) bands from the allele carrying the deletion, and to 2,483-pb (∼2.5 kb), 3,053-pb (∼3.0 kb) and 3,940-pb (∼3.9 kb) bands from the normal allele, which may be easily separated from those pertaining to the allele carrying the deletion. Oligonucleotide A1 (SEQ ID NO:37) may also be used as a direct primer, combining it, for example, with oligonucleotides R2 (SEQ ID NO:44), R3 (SEQ ID NO:45) or A2 (SEQ ID NO:40) as reverse primers, leading, respectively, to 1,099-pb (∼1.1 kb), 1,669-pb (∼1.7 kb) and 2,556-pb (∼2.6 kb) bands from the allele carrying the deletion, and to 3,343-pb (∼3.3 kb), 3,913-pb (∼3.9 kb) and 4,800-pb (4.8 kb) bands from the normal allele. Amongst all, the A1-R2 primer pair is preferred, since it leads to bands the sizes whereof (∼1.1 and ∼3.3 kb) are easy to discriminate after applying conventional electrophoresis and easily detectable at first sight once they are stained with ethidium bromide.

As an additional control measure, the bands separated by electrophoresis in agarose gels may be purified and sequenced. Sequencing is understood to mean the determination of the order wherein the units making up a DNA molecule are arranged, which, in this case, means determining the order wherein the nucleotides making up the PCR products or the fragments thereof appear. The analysis of the sequence obtained will make it possible to unequivocally ensure that the PCR products amplified, separated by electrophoresis and purified, correspond to fragments of the normal allele or the allele carrying the deletion.

The sequencing may be performed by any method known in the state of the art, such as the enzymatic method proposed by Sanger (Sanger et al., 1977) or any variant thereof. The Sanger DNA sequencing method is based on the interrupted synthesis of a DNA chain, complementary to the single chain that acts as a template and the sequence whereof we wish to analyse. The synthesis is initiated from a single primer, the sequencing primer, which specifically binds to the region where we wish to initiate the sequencing. The primer allows for the DNA-polymerase-mediated incorporation of nucleotides in the 5'-3' direction. This action is interrupted by the incorporation of nucleotides without the hydroxyl group in the 3' carbon (2'-3' dideoxynucleotide 5' triphosphate: ddNTP), and, therefore, incapable of linking to the following nucleotide. In determining the order of the nucleotides in the sequence analysed, the sequencing reaction products are subsequently separated by electrophoretic methods that discriminate, on the basis of size, products with a single different nucleotide, thereby obtaining the order wherein the nucleotides appear in each PCR product. However, it is preferable to use a modification of said sequencing method wherein the sequencing reaction is performed in the presence of the four ddNTPs, each marked with a different fluorescent molecule, and the reaction products are detected by laser-induced fluorescence in four spectral channels specific for each fluorescent marker, a methodology that is used to identify the deletion in Example 3, which is described further below in this specification.

In order to perform the sequencing, any of the amplification primers used to perform the PCRs in each specific segment may be used, for example, as sequencing primers. For example, if amplification primers A1 and A2 have been used for the PCR reaction, the sequencing of the PCR products pertaining to the allele with the deletion (2,556 pb) and to the normal allele (4,800 pb), once they have been separated by electrophoresis and purified, may be performed in each case either with primer A1 or with primer A2. If the sequencing of the fragment amplified from the normal allele is performed with primer A1, sequence SEQ ID NO:81 will be obtained. If the sequencing of the same fragment is performed with primer A2, the sequence complementary to sequence SEQ ID NO:81 (in the opposite direction) will be obtained. In sequencing the fragment amplified from the allele with the deletion, the same sequences will be obtained as in the preceding case, although the absence of the 2,244 pb pertaining to the ESR1-NCD1 deletion will be observed in each of them.

In the event that a large number of samples are to be processed, as might be the case in clinical practise, it is preferable to use other techniques to discriminate between the products amplified from the normal allele and those amplified from the allele carrying the deletion. In this case, an amplification strategy is preferred wherein the PCR is performed in the presence of three primers, one of which (direct or reverse) hybridises with the genomic DNA in the area corresponding to the deletion and, therefore, will only find a complementary sequence whereon to bind in the case of the normal allele, whereas the other two primers (one direct, complementary to a region located at the 5'-end with respect to the deletion, and the other reverse, complementary to a region located at the 3'-end with respect to the deletion) are complementary to sequences found in both the normal allele and the allele carrying the deletion. In this case, the PCR amplification conditions are selected in such a way that the normal allele may only lead to the amplification of the fragment thereof comprised between the primer bound to the deletion area and the corresponding primer (the direct or the reverse primer, as the case may be) with which it may form an amplification pair, and which binds to an area of the ESR1 gene present in both the normal allele and the allele carrying the deletion. One example of said amplification strategy for the subsequent sequencing by pyrosequencing is disclosed in Example 4, wherein oligonucleotide ED-RW (SEQ ID NO:47), used as the reverse primer, is specific for the normal allele, since it finds a complementary sequence only in the deletion area, whereas oligonucleotides ED-F (SEQ ID NO:46) (direct primer) and R2 (SEQ ID NO:44) (reverse primer), find complementary areas whereto they may bind in both the normal allele and the allele carrying the deletion, the former binding to the area located at the 5'-end with respect to the deletion and the latter binding to the area located at the 3'-end with respect thereto. However, the amplification conditions used in said Example 4 are such that, in the case of the normal allele, the distance between ED-F and R2 is excessive for the complete fragment located between both primers to be amplified, such that, in the case of the normal allele, in the presence of the three primers, only the fragment comprised between ED-F (SEQ ID NO:46) and ED-RW (SEQ ID NO:47) is amplified.

The selection of this amplification strategy makes it possible to obtain fragments with suitable sizes to be analysed using methodologies such as, for example, real-time sequencing of the PCR products by pyrosequencing. Use of this technique is preferred, because it facilitates the processing of a large number of samples, the automation thereof and a reduction in the assay times. The technical foundation for this technique may be found in the website http://www.pysequencing.com. Briefly, the technique is based on the addition, one by one, of deoxynucleotide triphosphates (dNTPs) to the single-chain DNA template previously amplified by PCR whereto an oligonucleotide is bound that acts as the primer in the sequencing reaction, detection of the incorporation of the deoxynucleotide added to the nascent chain complementary to said DNA template by detecting the released pyrophosphate coupling it to a pair of reactions that result in the emission of light and degradation of the non-incorporated dNTPs thanks to the presence of the apyrase enzyme in the reaction mixture.

It is particularly preferred that the three primers used for the amplification prior to the pyrosequencing are precisely those used in Example 4, ED-F (SEQ ID NO:46), ED-RW (SEQ ID NO:47) and R2 (SEQ ID NO:44), which, both in the case of the normal allele and in the case of the allele carrying the deletion, lead to fragments with suitable lengths to be analysed by pyrosequencing. In said Example 4, the sequencing primer used is ED-S (SEQ ID NO:48). This primer binds to the segment complementary to the 5'-area adjacent to the deletion in both the normal allele and the allele carrying the deletion. The 5 nucleotides at the 3'-end of primer ED-S (SEQ ID NCM8) bind to the 5 nucleotides complementary to the 5'-end of the region affected by the deletion, whereas, in the allele with the deletion, the 5 nucleotides of the 3'-end of primer ED-S (SEQ ID NO:48) bind to the 5 nucleotides complementary to the 3'-region adjacent to the region affected by the deletion. Therefore, primer ED-S (SEQ ID NO:48) may be used as a sequencing primer for both the normal allele and the allele carrying the deletion.

Another alternative method of detecting the presence of the ESR1-NCD1 deletion by analysing fragments generated using the amplification strategy described, wherein the PCR is performed in the presence of three primers, consists of discriminating the fragments amplified from the normal allele and the allele carrying the deletion by subjecting them to capillary electrophoresis and detecting them thanks to the fact that they couple to a marker molecule, preferably a fluorescent molecule, which makes it possible to use capillary electrophoresis coupled with fluorescence. To this end, the amplification primer common to both of the fragments amplified, or, alternatively, the two specific primers for each amplified fragment, must be marked with a fluorescent molecule at the 5'-end. Determination of the size of each amplified fragment is performed by comparing the time it takes to detect the signal pertaining to the marker coupled to each amplified fragment and the time it takes to capture the fluorescence of markers with a known size. As in the case of pyrosequencing, it is preferable that the primers used for the amplification of the fragments that are to be analysed by means of this methodology be those used in Example 4, ED-F (SEQ ID NO:46), ED-RW (SEQ ID NO:47) and R2 (SEQ ID NO:44).

Another object of the invention is any or any combination of the oligonucleotides designed for the development and execution of the invention, those oligonucleotides the sequence whereof is specified in SEQ ID NO:1, SEQ ID NO:2, SEQ ID NO:3, SEQ ID NO:4, SEQ ID NO:5, SEQ ID NO:6, SEQ ID NO:7, SEQ ID NO:8, SEQ IDNO:9, SEQ ID NO:10, SEQ ID NO:11, SEQ ID NO:12, SEQ ID NO:13, SEQ ID NO:14, SEQ ID NO:15, SEQ ID NO:16, SEQ ID NO:17, SEQ ID NO:18, SEQ ID NO:19, SEQ ID NO:20, SEQ ID NO:21, SEQ ID NO:22, SEQ ID NO:23, SEQ ID NO:24, SEQ ID NO:25, SEQ ID NO:26, SEQ ID NO:27, SEQ ID NO:28, SEQ ID NO:29, SEQ ID NO:30, SEQ ID NO:31, SEQ ID NO:32, SEQ ID NO:33, SEQ ID NO:34, SEQ ID NO:35, SEQ ID NO:36, SEQ ID NO:37 (A1), SEQ ID NO:38 (A3), SEQ ID NO:39 (A4), SEQ ID NO:40 (A2), SEQ ID NO:41 (F1), SEQ ID NO:42 (F2), SEQ ID NO:43 (R1), SEQ ID NO:44 (R2), SEQ ID NO:45 (R3), SEQ ID NO:46 (ED-F), SEQ ID NO:47 (ED-RW), SEQ ID NO:48 (ED-S), SEQ ID NO:49, SEQ ID NO:50, SEQ ID NO:51, SEQ ID NO:52, SEQ ID NO:53, SEQ ID NO:54, SEQ ID NO:55, SEQ ID NO:56, SEQ ID NO:57, SEQ ID NO:58, SEQ ID NO:59, SEQ ID NO:60, SEQ ID NO:61 and SEQ ID NO:62, or the combinations thereof. Amongst them, those in the group formed by SEQ ID NO:1, SEQ ID NO:2, SEQ ID NO:13, SEQ ID NO:14, SEQ ID NO:27, SEQ ID NO:28, SEQ ID NO:37 (A1), SEQ ID NO:38 (A3), SEQ ID NO:39 (A4), SEQ ID NO:40 (A2), SEQ ID NO:41 (F1), SEQ ID NO:42 (F2), SEQ ID NO:43 (R1), SEQ ID NO:44 (R2), SEQ ID NO:45 (R3), SEQ ID NO:46 (ED-F), SEQ ID NO:47 (ED-RW), SEQ ID NO:48 (ED-S), and the combinations thereof, are preferred, due to their proximity to the areas wherein the rs851995, rs3020314 and rs910416 polymorphisms, and the NCD1-ESR1 deletion, are located, the presence or absence whereof is detected in the method of the invention.

Those compositions that comprise at least one of the oligonucleotides the sequence whereof is specified in SEQ ID NO:1, SEQ ID NO:2, SEQ ID NO:3, SEQ ID NO:4, SEQ ID NO:5, SEQ ID NO:6, SEQ ID NO:7, SEQ ID NO:8, SEQ ID NO:9, SEQ ID NO: 10, SEQ ID NO:11, SEQ ID NO:12, SEQ ID NO:13, SEQ ID NO:14, SEQ ID NO:15, SEQ ID NO:16, SEQ ID NO:17, SEQ ID NO:18, SEQ ID NO:19, SEQ ID NO:20, SEQ ID NO:21 SEQ ID NO:22, SEQ ID NO:23, SEQ ID NO:24, SEQ ID NO:25, SEQ ID NO:26, SEQ ID NO:27, SEQ ID NO:28, SEQ ID NO:29, SEQ ID NO:30, SEQ ID NO:31, SEQ ID NO:32, SEQ ID NO:33, SEQ ID NO:34, SEQ ID NO:35, SEQ ID NO:36, SEQ ID NO:37 (A1), SEQ ID NO:38 (A3), SEQ ID NO:39 (A4), SEQ ID NO:40 (A2), SEQ ID NO:41 (F1), SEQ ID NO:42 (F2), SEQ ID NO:43 (R1), SEQ ID NO:44 (R2), SEQ ID NO:45 (R3), SEQ ID NO:46 (ED-F), SEQ ID NO:47 (ED-RW), SEQ ID NO:48 (ED-S), SEQ ID NO:49, SEQ ID NO:50, SEQ ID NO:51, SEQ ID NO:52, SEQ ID NO:53, SEQ ID NO:54, SEQ ID NO:55, SEQ ID NO:56, SEQ ID NO:57, SEQ ID NO:58, SEQ ID NO:59, SEQ ID NO:60, SEQ ID NO:61 and SEQ ID NO:62, or the combinations thereof, are also included within the scope of this invention.

Oligonucleotides with sequences SEQ ID NO:37 (A1), SEQ ID NO:38 (A3), SEQ ID NO:39 (A4), SEQ ID NO:40 (A2), SEQ ID NO:41 (F1), SEQ ID NO:42 (F2), SEQ ID NO:43 (R1), SEQ ID NO:44 (R2), SEQ ID NO:45 (R3), SEQ ID NO:46 (ED-F), SEQ ID NO:47 (ED-RW) are oligonucleotides complementary to areas of intron 6 that may be used as primers for the amplification of DNA fragments of said intron wherein the ESR1-NCD1 deletion may appear and which, therefore, are useful to perform the prognostic and/or diagnostic method of the invention. Any assay kit that comprises at least one of oligonucleotides A1 (SEQ ID NO:37), A3 (SEQ ID NO:38), A4 (SEQ ID NO:39), A2 (SEQ ID NO:40), F1 (SEQ ID NO:41), F2 (SEQ ID NO:42), R1 (SEQ ID NO:43), R2 (SEQ ID NO:44), R3 (SEQ ID NO:45); ED-F (SEQ ID NO:46), ED-RW (SEQ ID NO:47) or ED-S (SEQ ID NO:48), or combinations thereof, is also an object of the invention. A1 (SEQ ID NO:37), A4 (SEQ ID NO:39), F1 (SEQ ID NO:41), F2 (SEQ ID NO:42), ED-F (SEQ ID NO:46) and ED-S (SEQ ID NO:48) bind to the strand complementary to that whereof a sequence fragment is shown in SEQ ID NO:63 and SEQ ID NO:81, and may be used as direct amplification or sequencing primers for a region that comprises all or part of the area that is lost in the allele carrying the deletion, whereas A3 (SEQ ID NO:38), A2 (SEQ ID NO:40), R1 (SEQ ID NO:43), R2 (SEQ ID NO:44), R3 (SEQ ID NO:45) and ED-RW (SEQ ID NO:47) may be used as reverse amplification or sequencing primers for a region that comprises all or part of the area that is lost in the allele carrying the deletion. The kits of the invention may comprise one or more of the oligonucleotides in the group formed by A1 (SEQ ID NO:37), A4 (SEQ ID NO:39), F1 (SEQ ID NO:41), F2 (SEQ ID NO:42), ED-F (SEQ ID NO:46) and ED-S (SEQ ID NO:48), and/or one or more of the oligonucleotides in the group formed by A3 (SEQ ID NO:38), A2 (SEQ ID NO:40), R1 (SEQ ID NO:43), R2 (SEQ ID NO:44), R3 (SEQ ID NO:45) and ED-RW (SEQ ID NO.47). Kits that comprise at least one oligonucleotide from the group formed by those that may act as direct primers and one oligonucleotide from the group formed by those that may act as reverse primers are preferred, since they make it easier to perform the different embodiments of the method of the invention described above. Possible embodiments of the assay kits of the invention are those that comprise at least one pair of oligonucleotides selected from the following pairs:
a) A1 (SEQ ID NO:37) and R2 (SEQ ID NO:44);
b) A1 (SEQ ID NO:37) and R3 (SEQ ID NO:45);
c) A1 (SEQ ID NO:37) and A2 (SEQ ID NO:40);
d) ED-F (SEQ ID NO:46) and ED-RW (SEQ ID NO:47);
e) ED-F (SEQ ID NO:46) and R2 (SEQ ID NO:44);
f) ED-F (SEQ ID NO:46) and R3 (SEQ ID NO:45);
g) ED-F (SEQ ID NO:46) and A2 (SEQ ID NO:40);
or combinations thereof, and, optionally, oligonucleotide ED-S (SEQ ID NO:48).

The presence of potential markers coupled to the oligonucleotides, the specific markers selected, the remaining components present in the kit and the specific oligonucleotides selected for it will depend on the embodiment of the method of the invention wherein they are to be used. Because they are particularly suitable for the embodiments of the method of the invention disclosed in this specification, kits comprising the following are preferred:
i.i. at least the pair of oligonucleotides A1 (SEQ ID NO:37) and R2 (SEQ ID NO:44), and, optionally, dNTPs, one DNA polymerase I or the Klenow fragment thereof and a buffer, concentrated or at such a concentration that said polymerase may act therein;
i.ii. at least the pair of oligonucleotides A1 (SEQ ID NO:37) and R2 (SEQ ID 25 NO:44), and, optionally, dNTPs, a thermostable DNA polymerase and a buffer, concentrated or at such a concentration that said polymerase may act therein;
i.iii. at least the pair of oligonucleotides ED-F (SEQ ID NO:46) and R2 (SEQ ID NO:44) and the pair of oligonucleotides ED-F (SEQ ID NO:46) and ED-RW (SEQ ID NO:47), i.e. the three oligonucleotides ED-F, R2 and ED-RW, and, optionally, dNTPs, a thermostable DNA polymerase, a buffer, concentrated or at such a concentration that said polymerase may act therein, and, also optionally, oligonucleotide ED-S (SEQ ID NO:48), a kit wherein at least one member of each pair of oligonucleotides is coupled, at the 5'-end, to a marker that allows for the detection thereof. One possibility is that oligonucleotides ED-RW (SEQ ID NO:47) and R2 (SEQ ID NO:44) are marked at the 5'-end; amongst the markers, a possible marker is biotin. Another possibility is that oligonucleotide ED-F (SEQ ID NO:46) is marked at the 5'-end; amongst the markers, possible markers are fluorochromes, such as, for example, Cy5.

Moreover, any of the kits may contain one or more oligonucleotides from the group formed by SEQ ID NO:1, SEQ ID NO:2, SEQ ID NO:13, SEQ ID NO:14, SEQ ID NO:27 and SEQ ID NO:28, which would make them particularly useful for embodiments of the method of the invention wherein, in addition to determining the presence or absence of the allele carrying the deletion, the nucleotide present in the locations pertaining to at least one of the rs851995 (SNP1), rs3020314 (SNP7) or rs910416 (SNP14) polymorphisms of the human ESR1 gene is determined. It is preferable that, when the kit comprises one or more oligonucleotides from the group formed by SEQ ID NO:1, SEQ ID NO:2, SEQ ID NO:13, SEQ ID NO:14, SEQ ID NO:27 and SEQ ID NO:28, it also comprise the corresponding oligonucleotide with which it may form a pair in order to act as primers for the amplification of an area that comprises the polymorphism whereto they are close, i.e. that the kit comprise at least one of the pairs selected from the group formed by:
h) SEQ ID NO: 1 and SEQ ID NO:2;
i) SEQ ID NO:13 and SEQ ID NO:14;
j) SEQ ID NO:27 and SEQ ID NO:28,
or combinations thereof. It is particularly preferable that one of the oligonucleotides in each pair is coupled to a marker at the 5'-end, and the marker may be, for example, biotin.

The utility of the invention is demonstrated by a genetic association study of male idiopathic infertility in 166 idiopathic infertile males and 465 subjects from the general Spanish population, which is explained and illustrated by the Examples and the Figures shown below.

Said study is compatible with observations made in animals carrying mutations in the ESR1 gene (Korach, 1994), as well as with other recently published genetic association studies which describe the association of other variants of the human ESR1 gene with male idiopathic infertility (Kukuvitis et al., 2002; Suzuki et al., 2002; Galán et al., 2005; Guarducci et al., 2006).

In this invention, in addition to the finding related to the influence of the human ESR1 gene in male sperm production, which allowed for the prognosis and/or diagnosis of male idiopathic infertility, or the predisposition to suffer from it, by determining the presence or absence of a 2,244-nucleotide deletion located in intron 6 of the human oestrogen receptor alpha gene (ESR1), it was concluded that the presence and absence of said deletion in the subject's genomic DNA was also a tool for the *in vitro* prognosis and/or diagnosis of hypersensitivity to oestrogens or the predisposition to suffer other oestrogen-related diseases (ERDs), which include, amongst others: breast cancer, osteoporosis, blood hypertension, couple infertility or metabolic syndrome. This invention comprises, moreover, the fact that the prognosis/diagnosis of male idiopathic infertility or, in general, of oestrogen hypersensitivity, is based on the detection of the ESR1-NCD1 deletion, jointly with any of the following polymorphisms of the ESR1 gene: rs851995, rs3020314 and rs910416. Furthermore, the invention also considers, in the case of the prognosis/diagnosis of oestrogen hypersensitivity, that said prognosis/diagnosis is based, in addition to a deletion and the above-mentioned polymorphisms in the ESR1 gene, on the detection of the rs4986938 polymorphism of the ESR2 gene.

### BRIEF DESCRIPTION OF THE FIGURES

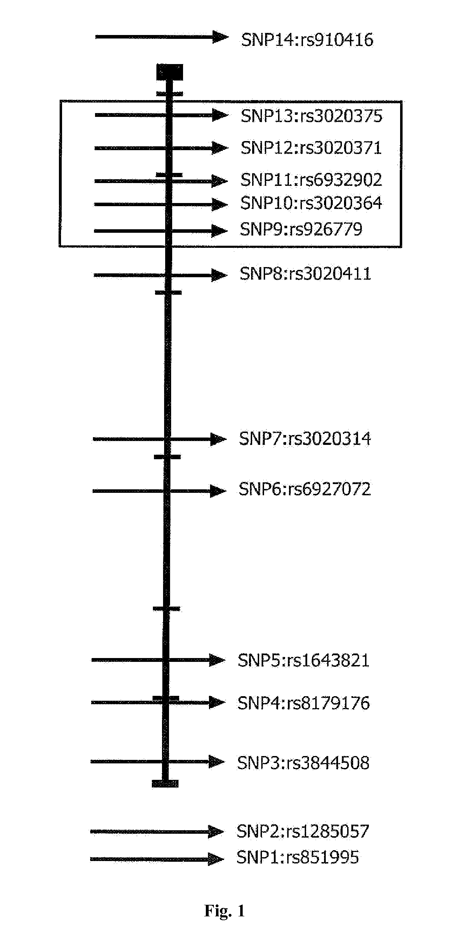

Fig. 1 shows a diagram that represents the human ESR1 gene. The thick vertical lines on the horizontal axis represent the gene's eight exons, whereas the fragments located between them represent the introns. The arrows indicate the location of the polymorphisms that constitute the panel of markers used in the ESR1-NCD1 identification process described further below. The box containing several arrows serves to highlight the markers referred to in document JP2006061128.

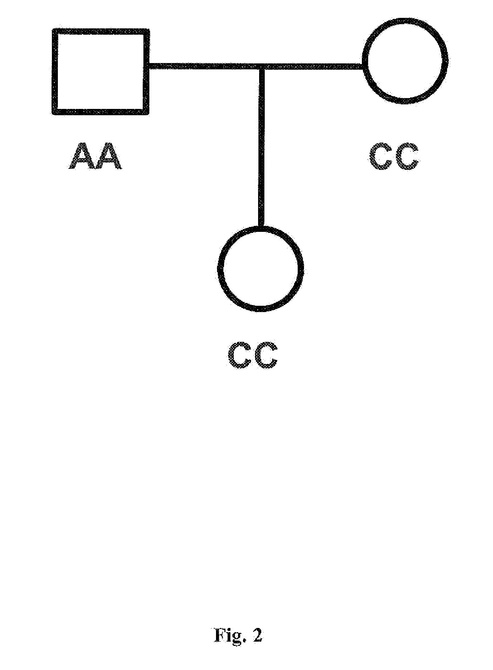

Fig. 2 represents the result of the genotyping of marker SNP13 (rs3020375) in the FN18 family nucleus. The square represents the male parent, the circle to the right represents the female parent and the circle located on the lower part represents the female offspring. The genotype of marker SNP13 of each member of FN18 is indicated in letters below the corresponding symbol.

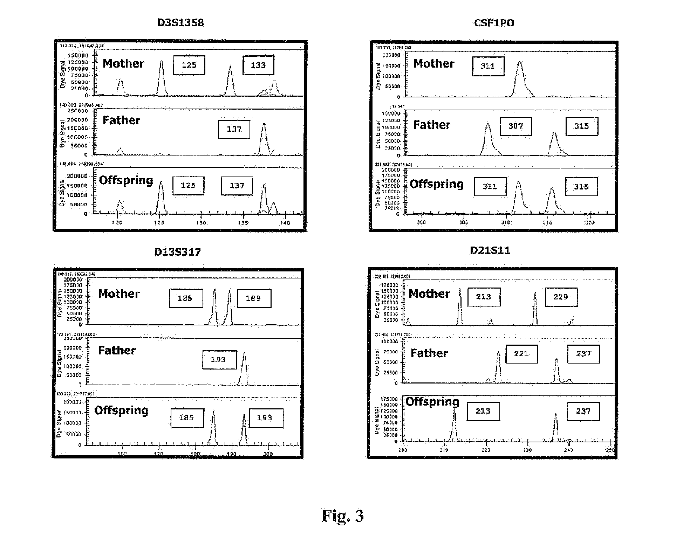

Fig. 3 shows the graphs obtained in the kinship study for different family nuclei. Each box covers the results obtained in a family nucleus for the microsatellite marker whose name is indicated above the box. In each box, the upper graph was that obtained for the mother, the middle graph was that obtained for the father and the lower graph was that obtained for the offspring of both. In each graph, the peaks with the greater height, next to which a boxed number appears, correspond to the alleles of each marker present in each of the subjects analysed, whereas the boxed number corresponds to the size of each allele in nucleotides. The peaks with the lower height, next to which there are no boxed numbers, correspond to the molecular weight marker.

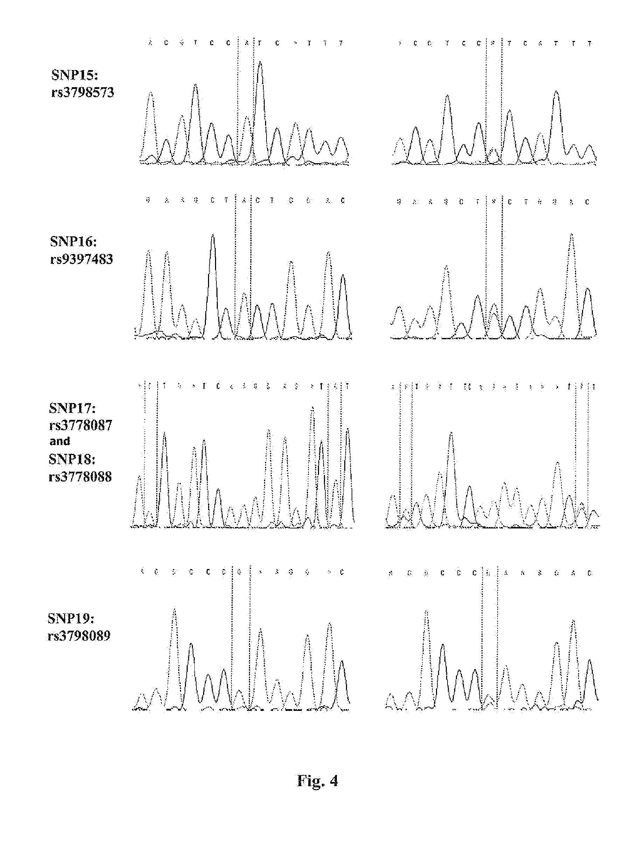

Fig. 4 shows the graphs obtained in the FN18 family nucleus following the sequencing of markers SNP15, SNP16, SNP17, SNP18 and SNP19, which appear, jointly with the corresponding pair of graphs, next to the left-hand edge of the page. The right column of graphs corresponds to the father of FN18, whereas the left column corresponds to the mother. The exact position of each marker analysed is indicated between bars. The letters above the peaks indicate the nucleotides present in each position by the abbreviation of the corresponding nitrogenated base: A; adenine; G: guanine; C: cytosine; T: thymine; R: guanine and adenine; K: guanine and thymine; M: adenine and cytosine; S: guanine and cytosine.

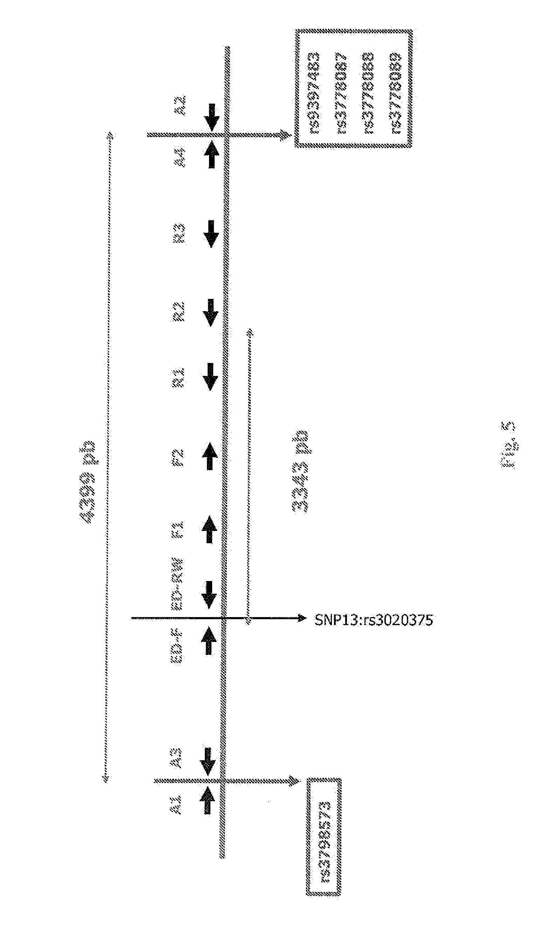

Fig. 5 is a schematic representation of the critical region of intron 6 of the human ESR1 gene comprised between the rs3798573 (SNP15) and rs9397483 (SNP16) polymorphisms, within which the ESR1-NCD1 deletion and the primers useful for the analysis thereof are located. The thick vertical arrows indicate the location of each of said polymorphisms, whereas the thinner vertical arrow indicates the location of rs3020375 (SNP13). The short horizontal arrows, located above the thick horizontal line that represents the intron fragment, indicate the sites whereto the primers indicated above the arrows bind.

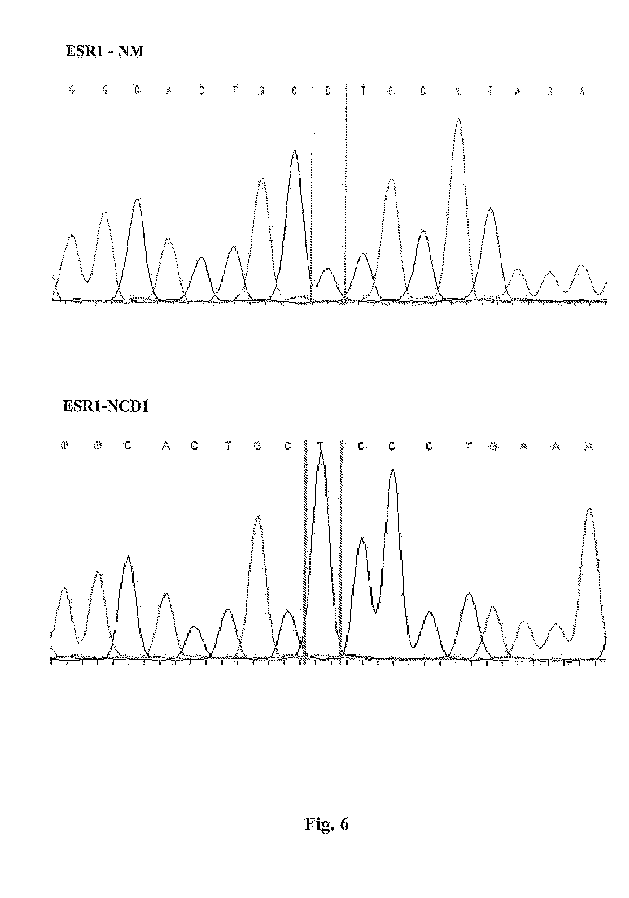

Fig. 6 shows the graphs obtained in the sequencing of the normal reference allele (upper graph, labelled as ESR1-NM) and of the allele that exhibits the deletion (lower graph, labelled as ESR1-NCD1), both obtained from the father of the FN18 family nucleus. The vertical bars delimit the position wherefrom the alleles differ and, therefore, the endpoint of the ESR1-NCD1 deletion.

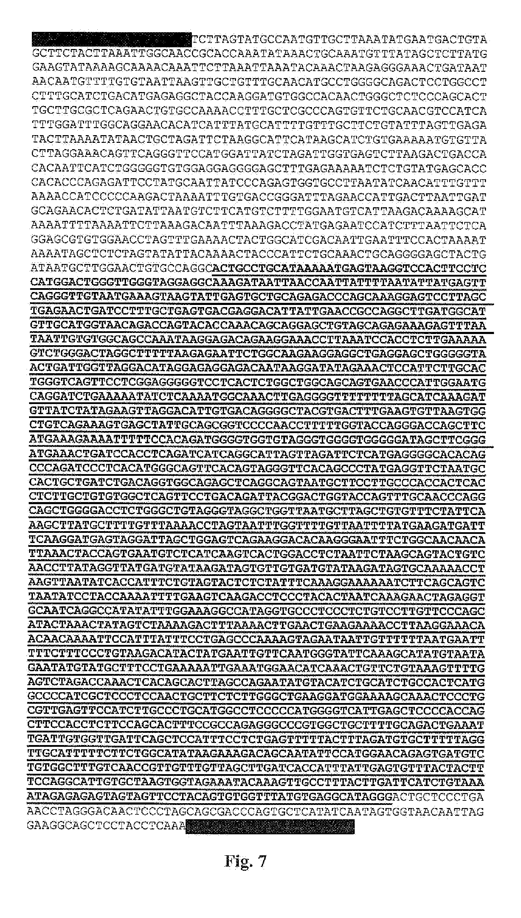

Fig. 7 shows the sequence of the 3,343-nucleotide fragment of intron 6 of the human ESR1 gene comprised between the sites whereto primers A1 (SEQ ID NO:37) and R2 (SEQ ID NO:44) bind. The areas with which said primers hybridise are indicated with a dark background over the corresponding nucleotides. The underlined nucleotides are those lost in the ESR1-NCD1 variant.

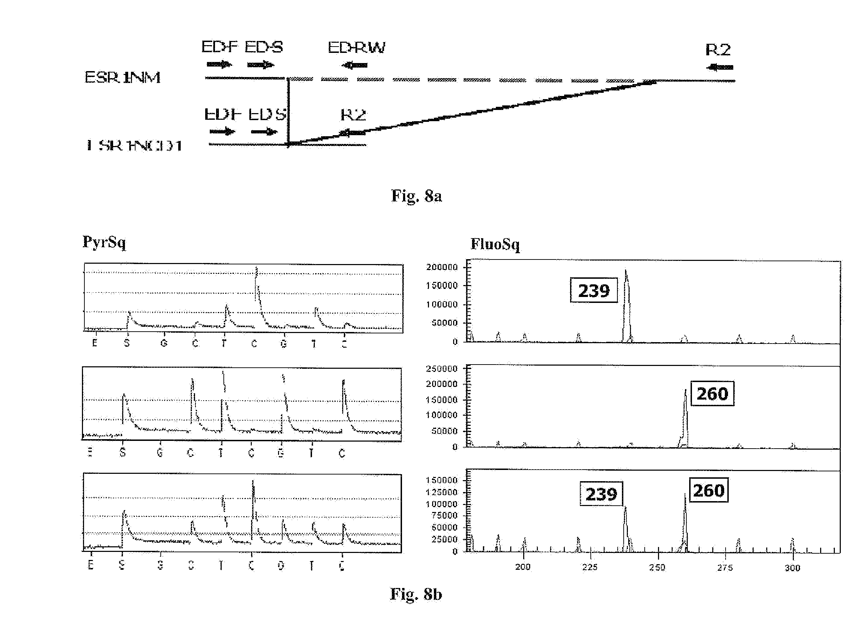

Fig. 8 refers to the genotyping of the ESR1-NCD1 variant. Fig. 8a is a representation of the normal allele (ESR1-NM) and the allele with the deletion (ESR1-NCD1), wherein the solid lines represent the genomic area common to both alleles and the broken line represents the genomic area that comprises the ESR1-NCD1 deletion. The sites whereto amplification primers ED-F (SEQ ID NO:46), ED-RW (SEQ ID NO:47) and R2 (SEQ ID NO:44), and sequencing primer ED-S (SEQ ID NO:48), bind are indicated with arrows above both alleles. Fig. 8b shows graphs obtained in the genotyping of subjects with respect to the alleles corresponding to the absence (ESR1-NM) or presence of said variant (ESR1-NCD1). The graphs on the left, headed by the abbreviation "PyrSq", were obtained using the pyrosequencing technique, whereas the graphs on the right, headed by the abbreviation "FluoSq", were obtained by capillary electrophoresis coupled with fluorescent detection of the alleles. The result obtained in the genotyping is indicated to the left of each pair of graphs obtained by a different technique: Hom_DD: homozygotic subject for the variant wherein the deletion is present; Hom_II: homozygotic subject for the normal allele, i.e. the variant wherefrom the deletion is absent; Het_ID: heterozygotic subject.

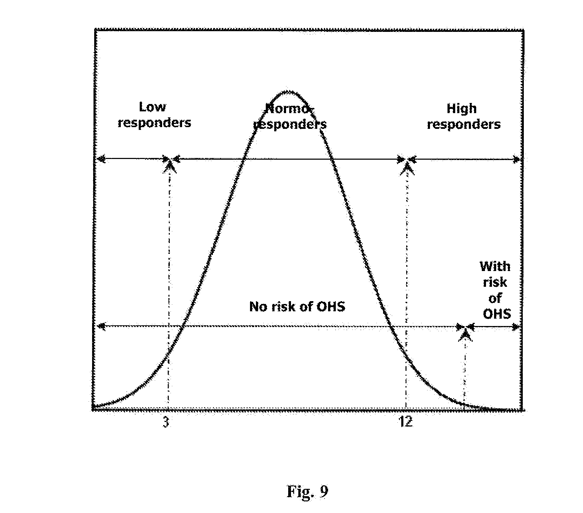

Fig. 9. Representation of the Gaussian bell distribution of the production of follicles in women subject to controlled ovarian hyperstimulation. The x-axis represents the frequency and the y-axis represents the number of follicles.

### DETAILED DESCRIPTION OF THE INVENTION

### Subjects included in the sturdy

In order to perform this invention, several groups of subjects with the diseases under study and other healthy subjects were selected. All the subjects included in the study are Caucasian and belong to the general Spanish population. This selection criterion was applied in order to avoid stratification problems in the genetic analyses performed during the invention, i.e. in order not to have to divide the population into separate subgroups, each of which would have to be sampled independently.

All the subjects included in the study provided an informed consent to participate therein. The ethics committees at the reference centres and Neocodex S.L. approved all the protocols followed in this study. The storage of DNA and the genetic analyses in this study comply with international regulations related to the collection, processing, storage and use of genetic data (International Bioethics Committee, UNESCO SHS-503/2001/CIB-8/3).

### Male idiopathic infertility,

- Group 1: Case group composed of 166 males with idiopathic infertility. All these subjects met the following inclusion criteria for patients in our study: i) azoospermia or severe oligozoospermia (less than 5 million spermatozoids per millilitre of ejaculated semen); ii) absence of any known cause of infertility, such as cryptorchidism, varicocele (presence of varicose veins in the scrotum), obstructive azoospermia, recurrent infections, iatrogenic infertility (caused by medical treatment), hypogonadotrophic hypogonadism, or anomalies in the karyotype; iii) absence of microdeletions in the Y chromosome in accordance with the methodology proposed by Simoni et al. (2004).
- Group 2: Control group composed of 465 unrelated male subjects.
- Group 3: 129 family nuclei (FN) formed by the two parents and at least one offspring. The parents of these FN were subject to the ICSI technique in order to have children. Furthermore, this group included two FN that could serve as negative kinship controls in the paternity studies, since, in these family nuclei, the male parents are anonymous sperm donours wherefrom DNA samples are not available; therefore, in each of those two groups, the member of the group that appeared as "father" was not really the biological father of the corresponding offspring.

### Response to Controlled Ovarian Hyperstimulation (COH)

A total of 366 women subject to assisted reproduction cycles by ICSI or *in vitro* fertilisation (IVF) were analysed. The patients were treated with rFSH for ovarian stimulation, jointly with a GnRHa for hypophyseal braking. Follicle development and growth were evaluated by transvaginal sonography from the sixth day of stimulation and each of the subsequent days. Finally, ovulation is induced by a simple injection (5,000-10,000 IU) of hCG in women with a minimum of 3 follicles and a diameter greater than 18 mm. The oocytes were obtained by previously described conventional methods, through directed transvaginal puncture by means of an echography (Dellenbach et al., 1985). The IVF and ICSI techniques were performed in accordance with the original protocol (Lopata et al., 1980; Edwards et al., 1980).

### Couple-level infertility,

In order to study the effect of the ESR1-NCD1 variant on couple-level infertility, we selected two groups of subjects. The first group is composed of 129 infertile couples that did not achieve pregnancy by natural methods and had to be subject to the Intracytoplasmatic Sperm Injection (ICSI) technique. These infertile couples are those included in the original patent for the identification and characterisation of the ESR1-NCD1 variant. The second group is composed of 100 couples with proven fertility who achieved pregnancy by natural methods.

### Osteoporosis

A total of 949 post-menopausal Caucasian women from the same geographical location were studied. For each woman, Bone Mineral Density (BMD) data were obtained using densitometric techniques. The following definition of osteoporosis is used herein:

T-score values obtained from lumbar spine bone mineral densitometry (L1-L4) below -2.5 or prior diagnosis of osteoporotic fracture (Ettinger et al., 1998). Finally, the group of women analysed were divided into 445 (46.89%) who had osteoporosis and 504 (53.11%) who did not.

The BMD values may be expressed as T-score units. The term "T score" refers to the number of standard deviations from the mean reference value for young adults. The WHO defines the BMD to be normal if it is within one standard deviation from the mean reference value for a young adult (T score > -1). Osteopaenia is defined by a T score between -1 and -2.5, whereas osteoporosis is defined as a T score below -2.5.

We also developed an intense clinical and epidemiological analysis of each woman included in this study. The clinical data were obtained using a questionnaire developed by the clinical researchers participating in this study. Several clinical characteristics previously considered to be risk factos for osteoporosis, such as age, weight, alcohol consumption and smoking, were also collected for each woman. In addition, the cause of menopause (natural, benign or cancer) and the body fat distribution (gynaecoid, android or neither) were also evaluated. The epidemiological results related to the known risk factors support the validity of the selection of the women and the faithfulness of the recording of the clinical and epidemiological data conducted.

### Breast cancer

The subjects analysed consist of 507 women who developed breast cancer between 23 and 88 years of age and underwent surgery for breast cancer between 1989 and 2006. For all these women, biopsies were performed of the tumour where the anatomic-pathological diagnosis was developed, data related to the treatment were obtained and a detailed follow-up was conducted until the first progression event was detected, understanding this to mean the appearance of metastasis or local recurrence.

As the reference population, 715 DNA samples from Spanish women between 34 and 82 years of age, from the same geographical area (Central and Southern Iberian Peninsula), were studied, in order to avoid potential confusions in the results that could be attributed to microstratification phenomena.

Both in the case of the breast cancer patients and in those women included in the study as controls, several epidemiological data associated with the risk of breast cancer were recorded, such as the age of menarche, the age of menopause, parity, etc., in order to adjust the statistical estimators when analysing the genetic data.

It is worth mentioning that the breast cancer study series already had several genetic markers of the oestrogen pathway and other metabolic pathways (ESR1, ESR2, CYP19, BMP 15, RIP140, amongst others), which has thus far allowed us to sub-classify each patient according to their genetic background, and has helped us to determine various genetic risk factors in the general population. A number of recent genetics-based works may be found in the literature which have contributed to a better understanding of the etiology of breast cancer in our population. Therefore, the study series has been previously evaluated by external reviewers and is a reliable source to check the hypotheses generated in the laboratory (Royo et al., 2006).

### Blood hipertensión

In order to analyse the effect of the ESR1-NCD1 variant on blood hypertension, a total of 1,219 women from the general Spanish population were recruited. In order to develop the genotype-phenotype correlations, the subjects included in the study were classified into two groups on the basis of two parameters:
1) Systolic hypertension: Systolic blood pressure greater than or equal to 130 mmHg
2) Diastolic hypertension: Diastolic blood pressure greater than or equal to 90 mmHg

The systolic and diastolic blood pressures were measured three times in the rest position after ten minutes of rest.

### Metabolic Syndrome

In order to analyse the effect of the ESR1-NCD1 variant on the probability of suffering MS, we analysed a total of 1,135 Caucasian subjects from the general Spanish population. These subjects were divided into two groups on the basis of whether they suffered from MS according to the IDF definition, into subjects with MS (n=517) and without MS (n=618).

### Extraction of genomic DNA

All the subjects included in the study provided a 5-ml sample of peripheral blood. Each subject's genomic DNA was extracted from the leukocytes present in each blood sample. The DNA extraction was performed in the MagNa Pure LC automatic station (Roche Diagnostics, Switzerland), using the Large Volume MagNa Pure LC Isolation kit (Roche Diagnostics, Switzerland), in accordance with the manufacturer's instructions. For the genetic tests, aliquots of genomic DNA at a concentration of 10 ng/µl were prepared, and the rest of the stock was cryopreserved at -20°C.

### Nucleotide sequence used

The DNA sequence used to perform the study corresponds to the genomic sequence of the ESR1 gene. This sequence is included in a "Contig", or a set of adjacent genomic segments (Genomic Contig NT_025741), that is available to the general public in the GenBank public database (www.ncbi.nlm.nih.gov). Said "Contig" comprises nucleotides 95.937.265 to 157.582.649 of human chromosome 6 in build 36.2 of the human genome.

The human ESR1 gene is located in the long arm of chromosome 6, in the 6q25.1 region. This gene comprises about 296 kilobases of genomic DNA and is composed of 8 exons separated by seven introns. The information regarding any polymorphism located in this gene used in this work was obtained from the GenBank SNP database (www. ncbi.nlm.nih.gov/SNP) or the HapMap International Project database (www.hapmap.org).

In this specification, we indicate the sequence of the fragments of the region pertaining to Genomic Contig NT_025741 relevant for the understanding of this invention and the development thereof. The chromosomal location of said sequences and of the polymorphisms used for the development of the invention were obtained from the second phase of the HapMap international project (July 2006) in public data release 21. These data are based on the National Centre for Biotechnology Information (NCBI) build 35 of the human genome.

### Statistical calculations

In order to compare the allele frequencies and genotype distributions between the groups of subjects included in the invention, and to analyse the Hardy-Weinberg Equilibrium (HWE) of the markers studied in the same groups, statistical tests adapted from Sasieni (1997) were used. These calculations were developed using the website of the Institute of Human Genetics in Munich, Germany (http://ihg.gsf.de).

In order to analyse the haplotype distributions between cases and controls, the THESIAS software (http://www.genecanvas.org), based on the SEM algorithm (Tregouet et al., 2004), was primarily used. This software performs the haplotype construction and the estimation of frequencies. Furthermore, this programme makes it possible to estimate the haplotype effects by comparison to the reference haplotype, which, in general, is the most frequent haplotype in the population. For the qualitative data, the results are expressed as odds ratios (OR).

In order to analyse the effect of the ESR1-NCD1 deletion on other continuous variables (presence or absence of the disease under study), we used the chi-square test (χ2). Whenever possible, we adjusted these analyses for factors previously known to influence the disease, by means of linear-regression analyses with the General Linear Model (GLM) application of the SPSS 13.0 statistical programme. This is the case with the studies conducted on osteoporosis.

We used the ANOVA test to analyse the influence of dichotomic variables (presence or absence of the ESR1-NCD1 variant) on continuous variables. In those cases when previously known factors that influence the continuous variables were available, we adjusted the analyses for these factors using the linear regression test. For these analyses, we also used the SPSS 13.0 statistical programme.

In order to compare the discrete values observed to those expected, we used the Bitest exact hypothesis test for binomial variables included in the STATA programme (StataCorp LP).

### EXAMPLE 1. Design and genotyping of a panel of SNP-type genetic markers in the ESR1 gene.

In order to perform the positional cloning of the ESR1-NCD1 genomic variant, a panel of markers was designed (in this case, Single Nucleotide Polymorphisms (SNPs)), located along the genomic sequence of the human ESR1 gene. For simplicity purposes, these polymorphisms were renamed SNP1 to SNP14, starting with that located at the gene's 5'-end. The gene location of each of the polymorphisms making up the panel of markers used in the study, as well as the name under which they appear in GenBank, are shown in Fig. 1.

The marker design used offers a mean density of 30.5 kb of genomic DNA per marker (Fig. 1). Markers SNP9 to SNP13 are the five markers included in the study by Yoshida et al. (2005) and in patent application JP2006061128, as previously discussed.

The study of the panel of SNP-type markers in the human ESR1 gene was performed in Group 1 of subjects (166 males with idiopathic infertility) and in 372 subjects from Group 2 (unrelated subjects from the Spanish population).

Genotyping of the 14 polymorphisms was performed by real-time DNA sequencing using Pyrosequencing technology (Biotage AB, Sweden). To this end, we designed amplification primers by Polymerase Chain Reaction (PCR) and specific DNA sequencing primers for each of the polymorphisms making up the panel of markers.

The PCRs were performed in a GeneAmp® PCR System 9700 thermocycler (Applied Biosystems, USA) at a final volume of 20 µl containing 20 ng of initial genomic DNA, 5 pmoles of each amplification primer, 5 nmoles of each deoxynucleotide triphosphate (dNTP Master Mix, Ecogen, Spain), 2 µl of PCR Reaction Buffer 10X (Roche Diagnostics, Switzerland), and 2 IU of Taq DNA polymerase (Roche Diagnostics, Switzerland). The conditions whereunder the PCRs were performed, the sequences of the amplification primers and the sequences of the corresponding sequencing primers are all shown in Table 1, including a "B" at the beginning of those sequences that correspond to biotin-marked primers. Said Table 1 also shows the polymorphisms analysed, their location in chromosome 6 and the two possible nucleotides that may appear in each of said polymorphism locations. Furthermore, the sequence listing in this specification indicates all the genomic sequences amplified by means of these PCR protocols.

In the processing of the PCR products for the subsequent analysis thereof by pyrosequencing, the DNA strand whereto the sequencing primer may bind, by base complementarity, must be preserved, and the strand complementary thereto must be eliminated. The biotin molecule is necessary to isolate the DNA strand of interest and, therefore, the biotin must be bound to the 5'-end of the amplification primer wherefrom said strand will be synthesised. Therefore, in those polymorphisms where the sequencing primer is to be bound to the homologous region of the region that appears in the 5'-direction starting from the polymorphism, the biotin-marked amplification primer must be the reverse one. On the contrary, if the sequencing primer is bound to a region in the 3'-direction starting from the polymorphism, the biotin-marked amplification primer must be the direct one.

Regardless of the primer used for the sequencing, for clarity purposes in the interpretation, the sequences amplified by means of the PCR protocols all refer to the same strand.

**Table 1: SNP-type markers of ESR1, amplification primers, PCR conditions and sequencing primers used in the genotyping thereof**

| Polymorphism | Primers (5 '->3') | PCR Product | | PCR conditions tdi(Tdi)-> [td(Td)->ta(Ta)->te(Te)]XNC-> tef(Tef-) |
|---|---|---|---|---|
| | | Ends of chromosome 6 (size) | SEQ ID NO: | |
| SNP1:rs851995 Genotype: G/A Chromosome 6 position: 152.097.648 | Direct: B-CATTTCGAATCCGTCCAACA (SEQ ID NO:1) Reverse: TGACTGAAGTTGAAGGCTGGA (SEQ ID NO:2) Sequencing: TTTGTGCTTAATTTAATCTT (SEQ ID NO:49) | 152.097.580-152.097.731 (152 pb) | 66 | 5'(95°C) -> [30"(95°C) -> 15"(54°C)-> 15"(72°C)]X50-> 7-(72"C) |
| SNP2: rs! 1285057 Genotype: C/T Chromosome 6 position: 152.195.906 | Direct: B-ATTTCCCTCCAGAAATGTGTGC (SEQ ID NO:3) Reverse: TTTGGCCAGCACTTAATTGTCC (SEQ ID NO:4) Sequencing: CAGCCAGAATAATGAGGA (SEQ ID NO:50) | 152.195.847-152.196.029 (183 pb) | 67 | 5'(95°C) -> [30"(95°C) -> 15"(60°C)-> 15"(72°C)]X50-> 7'(72°C) |
| SNP3: rs3844508 Genotype: T/C Chromosome 6 position: 152.232.156 | Direct: TGAAGAAGAAAGTAGGAACATGCC (SEQ ID NO:5) Reverse: 13-CTAGAGTGTACTACCTAAAGTGTG (SEQ ID NO:6) Sequencing: TGGCCTGAGGTCTTT (SEQ ID NO:51) | 152.232.001 -152.232.293 (293 pb) | 68 | 5'(95°C) -> [30"(95°C} -> 30"(60°C) -> 30"(72°C)]X50 -> 7'(72°C) |
| SNP4:rs8179176 Genotype: T/C Chromosome 6 position: 152.255.448 | Direct: ACTGATATCCAGGGTTATGTGG (SEQ ID NO:7) Reverse: B-CAGAACCATTAGAGACCAATGC (SEQ ID NO:8) Sequencing: TCCAAATGTCCCAGC (SEQ ID NO:52) | 152.255.341 -152.255.528 (188pb) | 69 | 5"(95°C)-> [30"(95°C)-> 30"(64°C) ->20"(72°C)]X70 -> 7'(72°C) |
| SNP5:rs1643821 Genotype: G/A Chromosome 6 position: 152.275.665 | Direct: ATTGTGTTCTGTTGCCTTCTCC (SEQ ID NO:9) Reverse: B-GAATTGTCCTTAACCTTCATGC (SEQ ID NO:10) Sequencing: CTGGAGTTAGTCCTG (SEQ ID NO:53) | 152.275.515-152.275.772 (258 pb) | 70 | 5'(95DC) -> [30"(95°C) ->15"(59°C) ->30"(72°C)]X50 -> 7'(72°C) |
| SNP6: rs6927072 Genotype: G/T Chromosome 6 position: 152.349.801 | Direct: TGCCAGCAAAACTCTAGAAGA (SEQ ID NO: 11) Reverse: B-TCATGTCCAGTGTTTTGTCCA (SEQ ID NO:12) Sequencing: ACAAGGTTAAAGGATT (SEQ ID NO:54) | 152.349.721-152.349.932 (212 pb) | 71 | 5'(95°C) -> [30"(95°C) -> 15"(63°C) ->20"(72oC)]X50 -> 7'(72°C) |
| SNP7:rs3020334 Genotype: T/C Chromosome 6 position: 152.362.786 | Direct: B-AACCCTGCTCTCAAACTGTTG (SEQ ID NO:13) Reverse: AAAGCTGCATATTGCCAGTCC (SEQ ID NO:14) Sequencing: TGTCCTTAAACAGAG (SEQ ID NO:55) | 152.362.683-152.362.913 (231 pb) | 72 | 5'(95DC) -> [30"(95°C) ->15"(63°C) -> 20"(720C)]X50 -> 7'(72°C) |
| SNP8:rs3020411 Genotype: A/G Chromosome 6 position: 152.435.877 | Direct: ATTTGAGAGCCATCTCCGTAG (SEQ ID NO:15) Reverse: B-CCTCCCAAAGTGCTGAGATT (SEQ ID NO:16) Sequencing: GGTTAAGGCAGTTTTTTG (SEQ ID NO:56) | 152.435.710- 152.435.984 (275 pb) | | |
| SNP9: rs926779 Genotype: G/A Chromosome 6 position: 152.448.034 | Direct: B-GCATTGACCAGATTCTTCAGTC (SEQ ID NO: 17) Reverse: ACGTGCGTAGTTAGCAAACATC (SEQ ID NO: 1S) Sequencing: TGCTAGTCGTTAGTAAAAAC (SEQ ID NO:57) | 152.447.899-152.44S.208 (310 pb) | 73 | 5'(95°C) -> [30"(95°C)->15"(60oC) ->30"(72°C)]X35 -> 7'(72°C) |
| SNP10:rs3020364 Genotype: A/G Chromosome 6 position: 152.459.232 | Direct: B-TCATTTGGGAGACTCTTATTGTCC (SEQ ID NO: 19) Reverse: CTACAAAACAGAATCAACGTCCTG (SEQ ID NO:20) Sequencing: GCTGGCCTAGATCTCA (SEQ ID NO:58) | 152.459.159-152.459.379 (221 pb) | 74 | 5'(95°C) -> [30"(95°C) -> 15"(64°C) ->30"(72°C)]X50 -> 7(72°C) |
| SNP11:rs6932902 Genotype: G/A Chromosome 6 position: 152.468.638 | Direct: B-GGATATATACCCAGTAGTGGG (SEQ ID NO:21) Reverse: TAAAAGGGTCTTGGGCATGGA (SEQ ID NO:22) Sequencing: GGGAGTCACCGTTAAG (SEQ ID NO: 59) | 152.468.439-152.46S.7S7 (349 pb) | 75 | 5'(95°C) ->[30"(95°C) -> 15"(60°C) ->30"(72°C)]X35 -> 7'(72°C) |
| SNP12:rs3020371 Genotype: C/T Chromosome 6 position: 152.475.934 | Direct: TTCCACTCACGTGCATTGTGC (SEQ ID NO:23) Reverse: B-CATCCACTGTGTGGATCATAC (SEQ ID NO:24) Sequencing: TGAACCTGTGCCTTCTT (SEQ ID NO:60) | 152.475.785-152.476.101 (317 pb) | 76 | 5'(95°C) -> [30"(95°C) ->15"(59°C) ->30"(72oC)]X50 -> 7'(72°C) |
| SNP13:rs3020375 Genotype: A/C Chromosome 6 position: 152.482.082 | Direct:B-TAATTCTCAGGAGCGTGTGG (SEQ ID NO:25) Reverse: GTCTCTGCAGCACTCAATAC (SEQ ID NO:26) Sequencing: GCACAGTTCCAAGCAT (SEQ ID NO:61) | 152.481.951 -152.482.239 (289 pb) | 77 | 5f(95oC) -> [30"(95°C) -> 15"(60°C) ->30"(72°C)]X35-> 7'(72°C) |
| SNP14:rs910416 Genotype: C/T Chromosome 6 position: 152.525.016 | Direct J3-AGGAACCGTGTCGCTGTCT (SEQ ID NO:27) Reverse: CTCCAATTTTGGCACCATCA (SEQ ID NO:28) Sequencing: CAAGGCACTGAGACCA (SEQ ID NO:62) | 152.524.981 -152.525.129 (149 pb) | 78 | 5'(95°C) -> [30"(95°C) -> 15"(57°C) ->30"(72°C)]X50 -> 7'(72°C) |

| | | | | |
|---|---|---|---|---|
| t: time; T: Temperature; di: initial denaturation; d: denaturation; a: annealing; e: elongation; NC: number of cycles; ef: final elongation | | | | |

The PCR products were genotyped using the PSQ 96 Gold kit (Biotage AB, Sweden) in a Pyrosequencing PSQ 96 instrument (Biotage AB, Sweden), in accordance with the manufacturer's instructions.

Although the panel of markers is composed of 14 polymorphisms, polymorphism SNP8 could not be genotyped in the above-mentioned series of cases and controls, since the results of the genotyping thereof did not show an adequate quality for the analysis designed.

The allele frequencies and genotype distributions of the remaining 13 markers in the subjects analysed are shown in Table 2, where the "Statistical Analysis" column shows the results obtained upon performing four different tests on the data: (a) the Hardy-Weinberg equilibrium (HWE) exact test, which is used to determine whether the observed genotype distribution of the marker under study is similar to that which would be expected according to the allele frequency of the marker in the case and control groups; (b) the allele frequency difference test, which indicates whether the allele frequency of the marker under study is statistically different between cases and controls; (c) the allele positivity test, which compares the genotype frequency of the marker under study in the cases and controls by assuming a dominant inheritance model for the disease, and (d) the Armitage trend test, a statistical test that measures the risk's tendency to exhibit the disease depending on the level of exposure to a factor, in this case, the polymorphism under study.

For each of these tests, the following are shown: the p-value, the odds ratio (OR) and the confidence interval (CI), except in the case of the HWE exact test, where only the p-value is shown, and the Armitage trend test, for which the p-value and the OR are shown. In addition to the p-value, which indicates whether the differences between the groups are statistically significant (when the value is less than 0.05), the two other values are shown because they are very useful to analyse the relationships between sequence variants and diseases: the odds ratio values, for example, reflect the probability that a subject carrying a given allele will suffer the disease (a value of OR>1 means predisposition to suffer the disease and a value of OR<1 indicates protection against the disease, i.e. a lower probability of suffering it), whereas the confidence interval helps to evaluate the precision with which a population parameter has been estimated, since it is the interval that comprises the population with a probability determined by the confidence level, which in this case was established at a value of 95%.

**Table 2.- SNPs of ESR1: Allele frequencies, genotype distributions and statistical studies**

| Polymorphism | Allele frequency (%) | | Genotype distribution | | Statistical analysis p-value [OR (CI)] |
|---|---|---|---|---|---|
| | Controls | Cases | Controls | Cases | |
| SNP1:rs851995 | A: 49.72 | A: 49.10 | 104 GA: 152 AA: 102 | GG: :42 GA:85 AA:39 | 0.003/0.88 (a) 0.85 [0.98 (0.75-1.27)] (b) 0.37 [1.21 (0.80-1.84)] (c) 0.86 [0.96] (d) |
| SNP2:rs1285057 | T: 42.09 | T: 40.00 | CC: 126 CT: 158 TT:70 | CC: 62 CT:74 TT:29 | 0.13/0.42 (a) 0.52 [0.92 (0.70-1.20)] (b) 0.66 [0.92 (0.63-1.35)] (c) 0.54 [0.92] (d) |
| SNP3: rs3844508 | C: 15.25 | C: 12.73 | TT:264 TC:89 CC:11 | TT: 128 TC:32 CC:5 | 0.31/0.15 (a) 0.28 [0.81 (0.55-1.19)] (b) 0.22 [0.76 (0.50-1.18)] (c) 0.30 [0.86] (d) |
| SNP4:rsS179176 | C: 47.70 | C: 42.42 | TT: 104 TC: 157 CC:88 | TT:58 TC:74 CC:33 | 0.07/0.34 (a) 0.11 [0.81 (0.62-1.05)] (b) 0.22 [0.78 (0.53-1.61)] (c) 0.13 [0.82] (d) |
| SNP5:rs1643821 | A: 46.19 | A: 45.45 | GG: 102 GA: 177 AA:75 | GG:51 GA:78 AA:36 | 0.91/0.51 (a) 0.76 [1.04 (0.80-1.35)] (b) 0.90 [0.97 (0.62-1.52)] (c) 0.76 [1.04] (d) |
| SNP6: rs6927072 | T: 27.71 | T: 32.50 | GG: 181 GT: 144 TT:25 | GG:72 GT:72 TT: 16 | 0.69/0.86 (a) 0.12 [1.26 (0.94-1.67)] (b) 0.16 [1.31 (0.90-1.91)](c) 0.11 [1.27] (d) |
| SNP7:rs3020314 | C: 26.20 | C: 34.34 | TT:191 TC: 142 CC: 22 | TT:69 TC:80 CC: 17 | 0.52/0.38 (a) 0.007 [1.47 (1.11-1.95)] (b) 0.009 [1.64 (1.13-2.39)](c) 0.006 [1.49] (d) |
| SNP9: rs926779 | A: 23.29 | A: 27.56 | GG:2I2 GA: 136 AA: 17 | GG:83 GA:60 AA: 13 | 0.47/0.69 (a) 0.14 [1.25 (0.93-1.70)] (b) 0.30 [1.22 (0.84-1.78)](c) 0.14 [1.31] (d) |
| SNP10:rs3020364 | G: 27.61 | G: 30.39 | A A: 187 AG: 153 GG:24 | AA:76 AG:61 GG:16 | 0.36/0.45 (a) 0.37 [1.15 (0.85-1.53)] (b) 0.72 [1.07 (0.73-1.56)] (c) 0.36 [1.20] (d) |
| SN11:rs6932902 | A: 8.96 | A: 11.57 | GG: 286 GA:58 AA:2 | GG:93 GA:28 AA:0 | 1.00/0.36 (a) 0.24 [1.33 (0.83-2.13)] (b) 0.16 [1.43 (0.87-2.38)] (c) 0.22 [1.41] (d) |
| SNP12:rs3020371 | T: 26.27 | T: 29.31 | CC: 188 CT: 146 TT:20 | CC: 74 CT:57 TT: 14 | 0.27/0.55 (a) 0.33 [1.16 (0.86-1.58)](b) 0.67 [1.09 (0.74-1.60)] (c) 0.32 [1.23] (d) |
| SNP13:rs3020375 | C: 19.61 | C: 19.63 | AA: 242 AC:98 CC: 22 | AA: 115 AC:32 CC: 16 | 0.01/<105 (a) 0.99 [1.00 (0.72-1.39)] (b) 0.40 [0.84 (0.56-1.26)] (c) 0.99 [1.09] (d) |
| SNP14:rs910416 | T: 43.16 | T: 49.08 | CC: 122 CT: 155 TT:74 | CC: 42 CT:82 TT:39 | 0.06/1.00 (a) 0.08 [1.27 (0.98-1.65)] (b) 0.04 [1.54 (1.01-2.32)] (c) 0.08 [1.25] (d) |

| | | | | | |
|---|---|---|---|---|---|
| (a) Exact test for HWE in controls/infertile males. (b) Allele frequency difference test. (c) Allele positivity test. (d) Armitage trend test. | | | | | |

The results of the univariant statistical analyses of these polymorphisms in the selected series are summarised below:
- Markers SNP3, SNP4, SNP5, SNP6, SNP9, SNP10, SNP11 and SNP12 do not exhibit any differences between the case and control groups.
- The HWE deviation observed for marker SNP4 in the case group in the previous work by the same inventors is not present in this new analysis.
- Marker SNP7 shows a nominal association with male infertility. Allele C of this polymorphism has an allele frequency of 34.34% in the cases and of 26.20% in the controls, which is statistically significant (see Table 2). This finding might suggest the existence of a genetic susceptibility factor for male infertility in linkage disequilibrium with marker SNP7. The measurement of the effect of this marker on male infertility in the population under study is given by the OR values in each test. Thus, male carriers of allele C of SNP7 would have 64% more possibilities of suffering from male infertility than males who do not carry said allele (OR of the allele positivity test = 1.64). If exposure to the predisposition factor increases, i.e. depending on whether there are zero, one or two copies of allele C of SNP7, the probability of suffering from male infertility increases by 49% for each exposure level (OR of the Armitage trend test = 1.49).
- On the other hand, the allele frequency of marker SNP 14 is not statistically different between the case and control groups. However, a significant p-value is observed in the allele positivity test for this marker. The size of the effect contributed by this test indicates that subjects carrying allele T of marker SNP 14 are 54% more susceptible to suffering from male infertility than subjects who do not carry said allele.
- The frequency of polymorphism SNP1 does not differ between the cases and the controls. However, this marker deviates from the HWE only in the control group. The HWE deviation in the control group is due to an excess of homozygotic subjects for genotypes GG and AA of marker SNP1. The fact that this phenomenon only takes place in the controls and not in the cases suggests that marker SNP1 is in linkage disequilibrium with a protective factor against male infertility.
- Similarly, the allele frequency of polymorphism SNP13 does not differ between the cases and the controls. However, this polymorphism shows a very marked HWE deviation in both the cases and the controls. The HWE deviation is due to an excess of homozygotic subjects for genotypes AA and CC of marker SNP13.

In general, the results derived from the analysis of the panel of markers of the ESR1 gene support the existence of at least one susceptibility factor for male infertility located in said gene. For this reason, it was decided to study these markers in more depth in order to attempt to locate said factor.

### EXAMPLE 2. Study of genetic markers of the ESR1 gene in family nuclei

In order to analyse in depth the polymorphisms with statistically significant values in the univariant studies of the panel of markers (SNP1, SNP7, SNP13 and SNP14), a segregation analysis thereof was performed in a group of 50 Family Nuclei (FN). Since marker SNP4 was associated with male infertility in two previous works (Kukuvitis et al., 2002; Galán et al., 2005), it was decided to also include this marker in the segregation analyses of the FN.

All the FN initially analysed exhibited Mendelian segregation of markers SNP4 SNP1, SNP4, SNP7 and SNP14. However, four FN (8%) showed non-Mendelian segregation for marker SNP13. Fig. 2 shows the segregation of SNP 13 in FN 18. There, it may be observed that the genotype of the male parent is AA, whereas that of the female parent is CC. According to Mendel's laws of inheritance, the offspring should inherit one copy of marker SNP13 from each parent and, therefore, should show genotype AC, instead of CC, which is the marker she really shows. For this reason, the segregation of marker SNP13 in this FN was considered to be non-Mendelian.

In order to ensure that these observations were not due to genotyping errors, genomic DNA was again extracted from the blood samples of each member of these 4 FN, and the genotyping of marker SNP13 was repeated in these new DNA samples. The results of the genotyping of these new samples showed a 100% coincidence with the results initially obtained for them.

Subsequently, the segregation study of marker SNP13 was performed in the remaining 79 FN included in the invention. Non-Mendelian segregation of marker SNP13 was observed in four new FN. Therefore, 8 of the 129 FN analysed (6.20%) exhibited non-Mendelian segregation of marker SNP 13.

In order to discard the possibility that the non-Mendelian segregations observed in the 8 FN for marker SNP13 were a consequence of a paternal or maternal discordance, a study of the kinship between the members of these 8 FN was performed, by analysing 4 microsatellite markers. In the kinship analyses, two FN were included as negative kinship controls and, in these FN, the mother, the offspring and the putative (non-biological) father were analysed. In order to perform the study, four very informative microsatellite markers were selected and the segregation thereof was observed. The information regarding the sequence and the different alleles of these four microsatellite markers was obtained from the GenBank STS database (www.ncbi.nlm.nih.gov). The four microsatellite markers used in the invention will be named according to their access number in this database (D3S1358, CSF1PO, D13S317 and D21 S 11). These four markers are composed of a variable number of repeats of a four-nucleotide sequence, which is specific for each marker; these repeats are indicated in Table 3. The study of the microsatellite markers in the subjects of interest was performed by conventional PCRs, allele discrimination of the different markers by capillary electrophoresis of the PCR products and subsequent detection of the alleles by fluorescence. In order to perform the PCRs, the amplification primers included in GenBank for each marker were used, as shown in Table 3.

**Table 3: Microsatellite markers used in kinship studies**

| Microsatellite marker | Primers (5'-> 3') | PCR conditions tdi(Tdi)-> [td(Td)->ta(Ta)->te(Te)]XNC-> tef(Tef) |
|---|---|---|
| Name: D3S1358 Chromosome region: 3p21.3 Repeat motif: AGAT Range of repeats: (8-20) | Direct: Cy5-ACAGAAGTCTGGGATGTGGA (SEQ ID NO:29) Reverse: GCCCAAAAAGACAGACAGAA (SEQ ID NO:30) | 5'(95°C) -> [30"(95°C) -> 30"(62C) -> 30"(72°C)]X35 -> 7'(72°C) |
| Name: CSF1PO Chromosome region: 5q33.3-q34 Repeat motif: AGAT Range of repeats: (6-16) | Direct: Cy5-AACCTGAGTCTGCCAAGGAC (SEQ IDNO:31) Reverse: TTCCACACACCACTGGCCAT (SEQ ID NO:32) | 5'(95°C) -> [30"(95°C) -> 30"(62C) -> 30"(72°C)]X35 -> 7'(72°C) |
| Name: D13S317 Chromosome region: 13q22 Repeat motif: TATC Range of repeats: (5-16) | Direct: Cy5-ACAGAAGTCTGGGATGTGGA (SEQ ID NO:33) Reverse: GCCCAAAAAGACAGACAGAA (SEQ ID NO:34) | 5'(95°C) -> [30"(95LC) -> 30"(60C) -> 30"(72°C)]X35 -> 7'(72°C) |
| Name: D21S11 Chromosome region: 21q2I Repeat motif: TCTG Range of repeats: (24-28) | Direct: Cy5-GTGAGTCAATTCCCCAAGTGA (SEQ ID NO:35) Reverse: GTTGTATTAGTCAATGTTCTCCA (SEQ ID NO:36) | 5'(95°C) -> [30"(95°C) -> 30"(60°C) -> 30"(72°C)]X35 -> 7'(72°C) |

| | | |
|---|---|---|
| t: time; T: Temperature; di: initial denaturation; d: denaturation; a: annealing; e: elongation; NC: number of cycles; ef: final elongation. | | |

The PCRs were performed in a similar manner to those used for the genotyping of the panel of markers described in the preceding section. The separation and identification of the alleles of the microsatellite markers was performed in a CEQ 8000 Genetic Analysis System automatic sequencer (Beckman Coulter, USA), using the CEQ DNA Size Standard-600 molecular weight marker (Beckman Coulter, CA, USA), in accordance with the manufacturer's instructions. The direct primers of the four microsatellite markers were marked with fluorochrome Cy5 at the 5'-end, which makes it possible to detect the different alleles of the marker by fluorescence in the CEQ 8000 system.

The kinship studies confirmed the paternal-filial relationship between all the members of the 8 FN analysed with a 99.9% certainty. As expected, the segregation of the four microsatellite markers proved paternal discordance in the two control FN. Fig. 3 shows the segregation of the four microsatellite markers analysed in different FN, none of which were the FN included as negative controls.

### EXAMPLE 3. Identification of a deletion located in intron 6 of the human ESR1 gene

The non-Mendelian segregation pattern of marker SNP13 in the 8 FN is compatible with the existence of a deletion that covers all or part of the genomic region where said marker is located. As an example, we can use the analysis of marker SNP13 in FN18 (Fig. 2). In this case, the presence of an allele of marker SNP13 deleted in the father which were subsequently inherited by his daughter would easily explain this segregation pattern.

In order to test the presence of the potential deletion, it was decided to initially analyse only the members of FN18. The strategy followed consists of locating two markers as close as possible to marker SNP13 in the 5'- and 3'-direction, for which the father of FN18 (who was assumed to be the carrier of the deletion) is heterozygotic. In this way, the genomic region where the deletion must be located is delimited. Subsequently, the region thus delimited is analysed at the nucleotide level.

Initially, markers SNP12 and SNP14 were analysed in FN18, since the genotyping methodology for both polymorphisms was available. The father of FN18 turned out to be heterozygotic for both markers; for this reason, the critical region was initially set at 49,082 nucleotides.

In order to delimit the critical region to a manageable size for conventional molecular biology techniques, it was decided to analyse additional markers closer to SNP13. To this end, we sequenced two genomic regions; one of these regions is located less than 1 kb from marker SNP13 in the 5'-direction and contains the rs3798573 polymorphism (SNP15); the other region is located about 4 kb from SNP13 in the 3'-direction and contains markers rs9397483 (SNP16), rs3778087 (SNP17), rs3778088 (SNP18) and rs3778089 (SNP19). These five markers were studied exclusively in FN18, by PCR and direct sequencing. The amplification primers and the conditions for both PCRs are shown in Table 4.

**Table 4.- Polymorphisms used to delimit the potential deletion**

| Polymorphism | Distance to marker SNP13 | Primers (5 '-> 3')* | PCR conditions tdi(Tdi)-> [td(Td)->ta(Ta)->te(Te)]XNC-> tef(Tef) |
|---|---|---|---|
| SNP15: rs3798573 | -606 pb | Direct: (A1) CGCAGTGCTGACTTAAAATGT (SEQ ID NO:37) Reverse: (A3) TCACAGATGCTTATGAATGCC (SEQ ID NO:38) | 5'(95°C) -> [30"(95°C) -> 30"(58°C) ->30"(72-C)]X50 -> 7'(72°C) |
| SNP16: rs93 97483 | +3,682 pb | | 5'(95°C) -> [30"(95°C) -> 30"(58°C) ->30"(72-C)]X50 -> 7'(72°C) |
| SNP17: rs3778087 | +3,739 pb | Direct: (A4) GGAGGGTTGATTTGTTGTGA (SEQ ID NO:39) Reverse: (A2) TTTCTGTTGCAGAGAATCTGG (SEQ ID NO:40) | |
| SNP18: rs3778088 | +3,753 pb | | |
| SNP19: rs3778089 | +3,793 pb | | |

| | | | |
|---|---|---|---|
| t: time; T: Temperature; di: initial denaturation; d: denaturation; a: annealing; e: elongation; NC: number of cycles; ef: final elongation. The signs + and - indicate the 5'- and 3'-direction of the markers with respect to SNP13, respectively. | | | |

The PCR products were subject to conventional electrophoresis in agarose gels and purified with the NucleoSpin® Extract kit (Macherey-Nagel, Germany), in accordance with the manufacturer's instructions. Subsequently, the purified PCR products were sequenced using the direct amplification primers by means of the GenomeLab™ DTCS Quick Start kit (Beckman Coulter, USA) in a Tpersonal thermocycler (Whatman Biometra, Germany). The sequence of the PCR product corresponding to the amplification performed with primers A1 (SEQ ID NO:37) and A3 (SEQ ID NO:38) is shown as SEQ ID NO:79, whereas the sequence of the PCR product corresponding to the amplification performed with primers A4 (SEQ ID NO:39) and A2 (SEQ ID NO:40) is shown as SEQ ID NO:80.

The sequencing products were purified by precipitation with ethanol and introduced into the CEQ 8000 Genetic Analysis System automatic sequencer (Beckman Coulter, USA), where capillary electrophoresis was performed in accordance with the manufacturer's instructions. In order to analyse the raw data and compare them to the reference sequences, the CEQ 8000 Genetic Analysis System computer programme (Beckman Coulter, USA) was used.

Fig. 4 shows the graphs derived from the genotyping by automatic sequencing of the five markers in the father and the mother of FN18. As may be observed in said Figure, the analysis of the two genomic regions considered revealed that the father of FN18 was heterozygotic for the five markers studied, whereas the mother was homozygotic for all of them. The critical homozygotic region was reduced to the region comprised between markers SNP15 and SNP16 (4,399 nucleotides).

Currently, no SNP-type polymorphisms have been described which are sufficiently informative and are located in a position closer to SNP13 than those analysed up to date. For this reason, we decided to study the critical region by sequencing. To this end, as shown in Fig. 5, amplification primers were designed (F1, F2, R1, R2 and R3) which divided the critical region into overlapping segments, also using the primer previously used for the genotyping of marker SNP15 (SEQ ID NO:37), indicated as A1 in Fig. 5, as the direct primer. The rest of the markers used were the following:
Direct:
   - F1: 5'-TCCACAGATGGGGTGGTGTAG-3' (SEQ ID NO:41)
   - F2: 5'-GTCTCATCAAGTCACTGGACC-3' (SEQ ID NO:42)
Reverse:
   - R1: 5'-GGCTAAGTGCTGTGAGTTTGG-3' (SEQ ID NO:43)
   - R2: 5'-TTTACCATCAGATCATGAGGTC-3' (SEQ ID NO:44)
   - R3: 5'-CACTTACGTGTTTAACAGCATC-3' (SEQ ID NO:45)

Therefore, using conventional PCRs and the combination of specific primer pairs, we attempted to amplify discrete region segments. This process was performed using the genomic DNA samples from the members of FN18.

The genomic region delimited by primers A1 and R2 was amplified by PCR in a GeneAmp® PCR System 9700 thermocycler (Applied Biosystems, USA) at a final volume of 20 µl containing 20 ng of initial genomic DNA, 5 pmoles of each amplification primer (Al : SEQ ID NO:37 and R2: SEQ ID NO:44), 10 nmoles of each deoxynucleotide triphosphate (dNTP Master Mix, Ecogen, Spain), 2 µl of PCR Reaction Buffer 10X (Roche Diagnostics, Switzerland), and 2 IU of Taq DNA polymerase (Roche Diagnostics, Switzerland). The mixture was subject to 95°C for 5 minutes; 50 cycles of 95°C for 1 minute, 62°C for 30 seconds and 72°C for 1 minute, and a final extension phase of 7 minutes at 72 °C.

After performing conventional electrophoresis in 1% agarose gels with the products of the PCR delimited by primers A1 and R2, marked in Fig. 5 by a line located beneath the line representing the gene, a DNA band corresponding to the expected size for the amplified 3,343-nucleotide segment was observed in all the members of FN18. However, in the samples from the father and the daughter of said FN, an additional smaller-size band (1,099 nucleotides) was observed.

Both PCR products were separately purified, sequenced and analysed, in a similar manner to that used for the genotyping of markers SNP15-SNP19. Oligonucleotides A1 (SEQ ID NO:37) and R2 (SEQ ID NO:44) were used as primers for the sequencing reaction. The graphs pertaining to the sequencing of both PCR products are indicated in Fig. 6. The analysis of the sequences of both PCR products revealed that the larger-size PCR product corresponded to the reference sequence for this segment included in GenBank, whereas the smaller-size PCR product was the result of a 2,244-nucleotide deletion in said region. Thanks to the analysis of the sequence of this PCR product, the deletion endpoints were delimited at the nucleotide level. The sequence of the 3,343-nucleotide segment delimited by primers A1 and R2, which is also indicated as SEQ ID NO:63, may be observed in Fig. 7. In said Fig. 7, the 2,244 nucleotides affected by the deletion, which are absent from one of the copies of the ESR1 gene in the father and the daughter of FN18, are underlined. The nucleotide sequence of said 3,343-nucleotide fragment (the fragment of intron 6 of the human ESR1 gene, comprised between the binding sites of primers A1 and R2, wherein the ESR1-NCD1 deletion may appear) is indicated as SEQ ID NO:63.

According to the nomenclature proposed by den Dunnen and Antonarakis (2001), the deletion identified in this invention should be named as: **g.IVS6+7731_IVS6+9974del.** As the authors of the discovery of this deletion and of this invention, the authors of the invention propose the alternative name **"ESR1 Non Coding Deletion 1**", with the abbreviation **"ESR1-NCD1".**

### EXAMPLE 4. Selection of the protocol for the protocolised detection of the deletion

Following the identification of the ESR1-NCD1 deletion in the father and the daughter of FN18, two alternative, high-performance genotyping protocols for said deletion were designed and prepared, in order to determine which was more adequate for the protocolised detection of the identified deletion.

One of the protocols was based on real-time sequencing using the pyrosequencing technology with the Pyrosequencing system (Biotage AB, Sweden). The technical foundations of this technology may be found in the website http://www.pysequencing.com. Briefly, the technique is based on the addition, one by one, of deoxynucleotide triphosphates (dNTPs) to a reaction mixture containing the single-chain DNA template previously amplified by PCR, which is bound to an oligonucleotide that acts as a primer in the sequencing reaction, in order to determine the incorporation of the deoxynucleotide added to the complementary nascent chain of said DNA template by detecting the pyrophosphate released, coupling it to a pair of reactions that lead to the emission of light and the degradation of the non-incorporated dNTPs thanks to the presence of the apyrase enzyme in the reaction mixture.

The second protocol is based on capillary electrophoresis coupled with the detection, by fluorescence, of the normal allele and the allele carrying the ESR1-NCD1 deletion, a process which, in this case, was performed in the CEQ 8000 Genetic Analysis System automatic sequencer (Beckman Coulter, USA). Capillary gel electrophoresis is the adaptation of traditional gel electrophoresis applied to capillaries, using polymer solutions to create a molecular sieve also known as replaceable physical gel. This allows for the analytes (in our case, the alleles to be analysed) to have similar charge/mass ratios, so that they may be resolved by size. In order to detect the alleles by fluorescence, the system excites a fluorescent molecule bound to the alleles by means of a diodic laser. Following this excitation, the fluorescent molecule will emit fluorescence in a wavelength that will be captured by the system. The time the system takes to record the fluorescence from each allele is directly proportional to the size thereof. Thus, by comparing the time the system takes to capture the fluorescence of each allele and the time it takes to capture the fluorescence of markers with a known molecular weight, the system may automatically determine the size of the alleles of interest.

Both protocols require a prior PCR reaction wherein the desired fragment is amplified. To this end, three amplification primers were used, which are indicated below:
- Direct:
   ED-F: 5'-TGAAAACTACTGGCATCGAC-3' (SEQ ID NO:46)
- Reverse:
   ED-RW: 5'-GTCTCTGCAGCACTCAATAC-3' (SEQ ID NO:47)
   R2: 5'-TTTACCATCAGATCATGAGGTC-3' (SEQ ID NO:44)

Primers ED-F and R2 are common to both alleles, whereas primer ED-RW is specific for the normal allele. The areas where the primers bind to each of the alleles of the gene are shown in Fig. 8a.

In both protocols, the normal allele is amplified by primers ED-F and ED-RW, whereas the allele carrying the ESR1-NCD1 deletion is amplified by the primer pair formed by ED-F and R2. Despite the fact that primer R2 binds to both alleles, it is not possible to amplify the normal allele with this primer under the PCR conditions used, since the distance between ED-F and R2 (1,648 nucleotides) in the normal allele is excessive for the PCR conditions included in both protocols.

The PCR conditions are identical for both genotyping protocols. The PCR is performed in a GeneAmp® PCR System 9700 thermocycler (Applied Biosystems, USA) at a final volume of 20 µl containing 20 ng of initial genomic DNA, 2.5 pmoles of each amplification primer (ED-F, ED-RW and R2), 5 nmoles of each deoxynucleotide triphosphate (dNTP Master Mix, Ecogen, Spain), 2 µl of PCR Reaction Buffer 10X (Roche Diagnostics, Switzerland), and 2 IU of Taq DNA polymerase (Roche Diagnostics, Switzerland). The mixture is subject to 95°C for 5 minutes; 35 cycles of 95°C for 30 seconds, 62°C for 30 seconds and 72°C for 30 seconds, and a final extension phase of 7 minutes at 72°C.

Under these conditions, the amplified sequences are the following:
- Normal allele: 
TGAAAACTACTGGCATCGACAATTGAATTTCCACTAAAATAAAATAGCTCTC
TAGTATATTACAAAACTACCATTCTGCAAACTGCAGGGGAGCTACTGATAA
TGCTTGGAACTGTGCCAGGCACTGCCTGCATAAAAATGAGTAAGGTCCACT
TCCTCCATGGACTGGGTTGGGTAGGAGGCAAAGATAATTAACCAATTATTTT
AATTTATGAGTTCAGGGTTGTAATGAAAGTAAGTATTGAGTGCTGCAGAGA
C (SEQ ID NO:64)
- Allele with the deletion:

TGAAAACTACTGGCATCGACAATTGAATTTCCACTAAAATAAAATAGCTCTC
TAGTATATTACAAAACTACCCATTCTGCAAACTGCAGGGAGCTACTGATA
ATGCTTGGAACTGTGCCAGGCACTGCTCCCTGAAACCTAGGGACAACTCC
CTAGCAGCGACCCAGTGCTCATATCAATAGTGGTAACAATTAGGAAGG
CAGCTCCTACCTCRAAGACCTCATGATCTGATGGTAAA (SEQ ID NO:65)

In these sequences, the nucleotides underlined at the ends correspond to the areas whereto the amplification primers bind; the nucleotides with double underlining correspond to an area whereto sequencing primer ED-S (SEQ ID NO:48) binds; the nucleotides in italics correspond to nucleotides that form a part of the fragment that is absent from the allele with the deletion; and the nucleotides in bold letters correspond to the area located at the 3'-end with respect to the deletion. The main difference between both protocols is the technology used to detect the deletion:

### - Protocol I: Real-time sequencing using the Pyrosequencing system (Biotage AB, Sweden).

In order to develop this technology, it is necessary for both reverse primers (in this case, R2 (SEQ ID NO:44) and ED-W (SEQ ID NO:47)) to be marked with biotin at the 5'-end. Moreover, a sequencing primer must be used which binds to a genomic region common to both alleles, which, in this case, was primer ED-S: 5'-ACTGTGCCAGGCACTGC-3' (SEQ ID NO:48) (see Fig. 8a). The nucleotide dispensing order was established as follows: GCTCGTC. In order to perform the real-time sequencing of the PCR products, the manufacturer's instructions were followed.

### - Protocol II: Genotyping by capillary electrophoresis coupled with detection of the alleles by fluorescence in the CEQ 8000 Genetic Analysis System automatic sequencer (Bechman Coulter, USA).

For this method, the direct primer (in this case, ED-F (SEQ ID NO: 46)) must be marked with fluorochrome Cy5 at the 5'-end. Capillary electrophoresis was performed in a CEQ 8000 Genetic Analysis System automatic sequencer (Beckman Coulter) using the CEQ DNA Size Standard-600 molecular weight marker (Beckman Coulter, CA, USA), in accordance with the manufacturer's instructions. In order to analyse the alleles detected by the system, the CEQ 8000 Genetic Analysis System computer programme (Beckman Coulter) was used. The conditions used in the capillary electrophoresis were as follows:
- Denaturation: 90°C for 2 minutes
- Sample injection: 2 kV for 30 seconds
- Separation phase: 50°C, 8 kV for 35 minutes

Fig. 8b shows the graphs pertaining to the genotyping of the deletion using both protocols. The left-hand side, labelled as "PySq", corresponds to the results obtained using the pyrosequencing technique; the right-hand side, labelled as "FluoSq", corresponds to the results obtained by capillary electrophoresis coupled with detection of the alleles by fluorescence. Both techniques seem to be valid for the protocolised detection of the deletion.

### EXAMPLE 5. Analysis of the effect of ESR1-NCD1 on male idiopathic infertility

In order to study the role of ESR1-NCD1 in male idiopathic infertility, the genotyping thereof was performed in Groups 1 and 2 of subjects, previously defined. The genotyping of these subjects was performed using pyrosequencing technology (Pyrosequencing / Biotage AB, Sweden), since this protocol is less expensive and faster than the alternative protocol using capillary electrophoresis coupled with fluorescence tested in Example 4. The PCR and sequencing conditions were those described in said Example 4.

The results of the genotyping de ESR1-NCD1 are included in Table 5. As was the case in Table 2, the "Statistical Analysis" column shows the results obtained upon performing four different tests on the data: the Hardy-Weinberg equilibrium (HWE) exact test in controls/infertiles males (a), the allele frequency difference test (b), the allele positivity test (c) and the Armitage trend test (d). For each of them, the following is shown: the p-value, the odds ratio (OR) and the confidence interval (CI) at the 95% level, except in the case of the HWE exact test, for which only the p-value and the OR are shown.

**Table 5.- Allele frequencies, genotype distributions and statistical studies related to the presence of the ESR1-NCD1 deletion in Groups 1 and 2**

| Group | Allele frequency | Genotype distribution* | Statistical analyses |
|---|---|---|---|
| | | II: 349 | 0.10/0.31 (a) 0.04 [1.48 (1.00-2.19)] (b) 0.04 [1.55 (1.01-2.36)] (c) 0.04 [1.55] (d) |
| 1 | 13.75 | ID: 82 | |
| | | DD: 1 | |
| | | II: 117 | |
| 2 | 9.72 | ID: 42 | |
| | | DD: 1 | |

| | | | |
|---|---|---|---|
| (a) HWE exact test in controls/infertile males. (b) Allele frequency difference test. (c) Allele positivity test. (d) Armitage trend test. II: homozygotic for the normal allele. ID: heterozygotic. DD: homozygotic for the deletion | | | |

The allele frequency of ESR1-NCD1 in the general Spanish population is 9.72%, whereas in the group of subjects with idiopathic infertility included in the invention it is 13.75%. The difference in the frequency of ESR1-NCD1 in both groups is statistically significant. The increased predisposition to suffer from idiopathic infertility due to the presence of the ESR1-NCD1 deletion is defined by the odds ratio (OR) values obtained in the statistical analyses. Thus, a male carrying ESR1-NCD1 has 55% more possibilities of being infertile than a subject who does not carry said deletion (OR of the allele positivity test = 1.55). Moreover, the OR obtained in the Armitage trend test (1.55) suggests that, as exposure to the predisposition factor increases (upon going from the ESR1-NCD1 deletion being absent from the genome to having a copy thereof, and, subsequently, having two copies), the probability of being infertile increases by 55% at each exposure level. The OR of the allele difference test considers the alleles individually, not in pairs. As a consequence, this test's OR is not adequate to measure the actual effect of the deletion on a diploid population.

It is worth noting that the ESR1-NCD1 deletion does not deviate from the HWE in the case and control groups, contrary to what occurred with marker SNP13, which is located only 21 nucleotides from the beginning of the ESR1-NCD1 deletion.

In fact, this deletion includes the genomic region where the reverse amplification primer designed for the genotyping of polymorphism SNP13 binds. Therefore, heterozygotic subjects for marker SNP13 who carry a copy of the ESR1-NCD1 variant are genotyped as "false homozygotes", since the allele carrying ESR1-NCD1 could not be genotyped for marker SNP13 in accordance with our protocol.

Finally, if only the subjects genotyped for marker SNP13 who, in turn, do not carry any alleles with the ESR1-NCD1 variant, are considered, no deviations from the HWE are observed for marker SNP13 in the cases (p=0.15) or in the controls (p=0.84). For this reason, it was concluded that the HWE deviation initially observed for marker SNP13 is due to the presence of the ESR1-NCD1 deletion, which caused heterozygotic subjects for SNP13 to be genotyped as homozygotic for the same marker, thereby excessively increasing the total number of homozygotes for marker SNP13.

These results show that the ESR1-NCD1 variant of the human ESR1 gene may cause reduced sperm production in humans. This fact stresses the importance of oestrogens in general, and of Oestrogen receptor alpha in particular, on the male reproductive function in humans. Since this variant also appears in the general population, it is possible that ESR1-NCD1 may require other genetic or environmental factors to show its deleterious effects on human spermatogenesis. Therefore, these findings do not exclude the presence of another genomic variant in the ESR1 gene that may also influence sperm production in humans.

In any event, these data show that the presence of the ESR1-NCD1 deletion increases the probability that a male is infertile and, therefore, support the utility of determining the presence or absence of this deletion to assess a subject's predisposition to suffer from oligozoospermia or azoospermia and infertility related thereto.

### EXAMPLE 6. Relationship between ESR1-NCD1 and the SNP1, SNP7 and SNP14 polymorphisms

In order to analyse the relationship between the ESR1-NCD1 variant and the polymorphisms that showed statistically significant values in the study of the panel of markers (SNP1, SNP7 and SNP14), we performed a genetic linkage study with these markers using the Thesias statistical programme. This study was performed on the 166 infertiles subjects and the 372 controls for which the panel of markers of the ESR1 gene was studied.

The term "linkage disequilibrium" (LD) between markers refers to the tendency of markers to be jointly transmitted as a consequence of the physical distance between them in the same chromosome. Although the distance between the markers is the main determining factor of the degree of LD between them, the recombination rate in the region between both may modify said relationship. Occasionally, markers that are physically very distant are jointly transmitted with a higher frequency than would be expected on the basis of the distance between them; on the other hand, the existence of recombination hotspots in the genome may determine which markers located very close to one another in the chromosome are independently transmitted with a high frequency. The degree whereto two markers tend to be jointly transmitted is quantified by means of Lewontin's parameter (D'), the values whereof range from -1 to +1. A value of D'= ± 1 indicates complete linkage disequilibrium between both markers, i.e. they are always jointly transmitted. The sign is positive when the less frequent alleles in each polymorphism are jointly transmitted, and it is negative when the ones jointly transmitted are the most frequent in one and the less frequent in the other (Lewontin and Kojima, 1960).

The results of this analysis show that none of the three markers analysed exhibit linkage disequilibrium with the ESR1-NCD1 variant, or between one another. That is, the transmission of the alleles of markers SNP1, SNP7 and SNP14 from one generation to the next is independent from the presence or absence of the ESR1-NCD1 variant, as well as from the allele that is present in each marker. Table 6 shows the D' values for the different pairs of markers analysed in the cases and the controls.

**Table 6.- Linkage study between markers SNP1, SNP7 and SNP14 and the ESR1-NCD1 variant.**

| | Controls | | | Cases | | |
|---|---|---|---|---|---|---|
| | SNP7 | ESR1-NCD1 | SNP 14 | SNP7 | ESR1-NCD1 | SNP 14 |
| SNP1 | 0.10 | -0.12 | 0.10 | 0.04 | 0.04 | 0.04 |
| SNP7 | | 0.45 | -0.14 | | 0.48 | -0.04 |
| ESR1-NCD1 | | | 0.11 | | | 0.07 |

The Thesias programme also made it possible to analyse the allele combinations of markers SNP1, SNP7 and SNP14 with the presence or absence of the ESR1-NCD1 variant, and the effect thereof on male idiopathic infertility. Of the 16 possible allele combinations (combinations of four elements with two possible values, 24=16), only six of them exceeded a 5% frequency in the cases and the controls.

Table 7 indicates the frequencies of the six allele combinations analysed in the case and the control groups, as well as the statistical analysis performed thereon.

**Table 7.- Statistical analysis of the frequencies of the haplotypes resulting from the allele combinations of markers SNP1, SNP7, SNP14 and the ESR1-NCD1 variant in the case and control groups.**

| Haplotype | Frequency in controls (%) | Frequency in cases (%) | Statistical analyses p-value [OR (95% CI)] |
|---|---|---|---|
| GTIC | 20.98 | 16.78 | 1* |
| GTIT | 13.20 | 15.15 | 0.68 [1.12 (0.65-1.93)] |
| GCIC | 5.45 | 7.76 | 0.37 [1.38 (0.68-2.82)] |
| ATIC | 14.60 | 13.54 | 0.78 [0.92 (0.53-1.60)] |
| ATIT | 17.41 | 15.06 | 0.53 [0.86 (0.55-1.36)] |
| ACIC | 7.34 | 5.68 | 0.51 [0.76(0.34-1.71)] |

| | | | |
|---|---|---|---|
| Combination used as a reference. For each combination, positions 1, 2, 3 and 4 indicate the allele corresponding to markers SNP1 (G or A), SNP7 (T or C), ESR1-NCD1 (I for the absence of the deletion or D for the presence of the deletion) and SNP14 (C or T). Note that the ESR1-NCD1 deletion is not present in any of the six haplotypes with a frequency greater than 5% in the cases or the controls. | | | |

The statistical analysis revealed that the frequencies of the allele combinations studied do not differ between the cases and the controls (none of the p-values are less than 0.05). The effect of the allele combinations on male infertility, measured in each case by the ORs, with respect to the most common allele combination (GTIC), showed values very close to 1, which led us to conclude that none of these six combinations seem to be associated with male infertility in the population analysed.

Therefore, the effect observed for markers SNP1, SNP7 and SNP14, and the effect of the ESR1-NCD1 variant on male idiopathic infertility in the Spanish population are independent from one another.

### Example 7. Study of the ESR1-NCD1 variant on the response to Controlled Ovarian Hyperstimulation (COH)

Below we list the parameters recorded during the COH cycles which are affected by the presence of the ESR1-NCD1 variant:

### • Endometrial growth:

Endometrial growth is a consequence of COH that depends, to a large extent, on the production of oestrogens derived from follicle-genesis. In order to test the effect of the ESR1-NCD1 variant on endometrial growth, we used a linear-regression test, making adjustments for the levels of estradiol in patients subject to COH following the stimulation of follicle-genesis.

The statistical analyses show that the presence of the ESR1-NCD1 variant causes those women who carry it to exhibit greater endometrial growth (mean growth of 20%), as compared to women who do not exhibit the ESR1-NCD1 variant, when subject to COH cycles. Thus, the mean endometrial growth of women carrying the ESR1-NCD1 deletion is 13.32 mm and that of women who are not carriers is 11.41 mm. This difference is statistically significant (p=0.035).

This may be considered to be a functional test of the action of the ESR1-NCD1 deletion. Taking into consideration that endometrial tissue responds to oestrogenic substances by a thickening thereof, and that women carrying the deletion have a greater proliferation of endometrial tissue when subject to COH cycles, we may conclude that the deletion causes an increase in this tissue's oestrogenic activity through an increase in hypersensitivity to these compounds.

In fact, a bioassay called uterotrophic assay has been proposed which makes it possible to evaluate the oestrogenic capacity of certain substances (endogenous or not). This assay consists of administering different doses of the compound under study, using a compound of known oestrogenic activity (for example, estradiol) as a reference, to an animal model (normally a rat or mouse) and evaluating the uterus weight / body mass ratios in these animals, in order to compare them to those obtained in the animal models that have been received the reference compound (Yamagashi et al., 2003).

Therefore, these data offer functional evidence (endometrial growth in women subject to COH) which confirms that the ESR1-NCD1 deletion acts by increasing the sensitivity to the action of oestrogens or substances with oestrogenic activity by those women who carry it.

### • Follicle production:

As previously discussed, follicle production in the COH cycles is usually stimulated by the administration of substances with gonadotrophic activity. The number of follicles produced following the administration of said substances is a clear indicator of the response to COH. Follicle production in women subject to COH perfectly fits a Gaussian bell distribution, as shown in Figure 9.

Taking this observation into consideration, our group proposed classifying the response to COH on the basis of follicle production into three groups (de Castro et al., 2003):
- Low responders: patients who produce ≤3 follicles (Fridstrom et al., 1997)
- High responders: patients who produce ≥12 follicles and belong to the 90 percentile
- Normoresponders: patients who produce between 4 and 11 follicles.

Upon comparing the frequency of the ESR1-NCD1 deletion between the low-and high-responder groups, a statistically significant difference between both groups (p=0.023; OR=3.709) was observed. This analysis allows us to conclude that the ESR1-NCD1 variant is more frequent in women with a high response to COH at the follicular level and that, consequently, this variant could be used as a predictor of high response to COH. This result once again suggests that carriers of the deletion are more sensitive to oestrogens, since we should not forget that the main, most classical test to measure the response during COH are the levels of estradiol achieved during the induction. It is also well known that the intrafollicular production of estradiol (induced during COH) regulates follicular recruitment mechanisms and the dominance phenomenon in the oestrogenic phase of mammals' ovarian cycle.

### • Hypophyseal braking:

As previously discussed, hypophyseal braking consists of the suppression of the activity of the "hypothalamus-hypophysis-ovary axis". The efficacy of the hypophyseal braking (time elapsed from the beginning of the treatment until the systemic levels of estradiol decrease to below 50 pg/ml) will depend on the patient's age, the quantity and the type of GnRHa administered and the levels of estradiol prior to this treatment. In order to evaluate the effect of the ESR1-NCD1 variant on this parameter, we developed variance analyses (ANOVA) of hypophyseal braking adjusted for the above-mentioned parameters.

These analyses made it possible to verify that the presence of the deletion decisively influences the hypophyseal braking time, such that women carrying said variant achieve hypophyseal braking almost four days earlier on average than those who are not carriers (p=0.05).

This observation once again indicates that the ESR1-NCD1 deletion has a hypersignalling effect for the action of oestrogens, since, for equal levels of endogenous oestrogens, women who carry ESR1-NCD1 achieve ablation of the hypothalamus-hypophysis-ovary axis earlier than those who do not carry said variant. This effect is mediated by negative feedback mechanisms of oestrogens on said axis. Therefore, women with a higher sensitivity to oestrogens at this level will have a more intense negative feedback mechanism and will achieve hypophyseal braking faster than women with a less marked sensitivity to oestrogens (and, consequently, the negative feedback mechanism).

The analyses performed in relation to the response to COH show that the ESR1-NCD1 variant increases the sensitivity of the ESR1 oestrogen receptor to the action of oestrogens and other substances with oestrogenic activity.

### Example 8. Analysis of the ESR1-NCD1 variant on couple-level infertility

The allele frequencies and genotype distribution of the ESR1-NCD1 variant in groups NF-ICSI and NF-FP are not statistically different.

On the other hand, on the basis of the frequency of carriers of the ESR1-NCD1 deletion in men and women, the number of couples wherein both members would be carriers of this variant was calculated.
Frequency of ESR1-NCD1 in infertile men: 28.68%
Frequency of ESR1-NCD1 in infertile women: 22.48%
Expected frequency of infertile couples wherein both members are carriers of ESR1-NCD1: 0.2868 x 0.2248 = 0.0645

The observed frequency of infertile couples wherein both members are carriers of the ESR1-NCD1 deletion is 0.1008 (13/129).

The Bitest hypothesis test indicates that the number of infertile couples wherein both members are carriers of the ESR1-NCD1 deletion is statistically greater than expected (p=0.03).

In order to verify whether the ESR1-NCD1 deletion shows a similar behaviour in couples with proven fertility, we performed the same analysis.
Frequency of ESR1-NCD1 in fertile men: 19.00%
Frequency of ESR1-NCD1 in fertile women: 30.00%
Expected frequency of fertile couples wherein both members are carriers of ESR1-NCD1: 0.19 x 0.30 = 0.057

The observed frequency of infertile couples wherein both members are carriers of the ESR1-NCD1 variant (6%) is practically identical to the expected value (p=1).

Taking these results into consideration, it may be observed that the frequency of infertile couples wherein both members are carriers of the ESR1-NCD1 deletion (10.08%) is higher than in the case of fertile couples (6%). Moreover, the number of infertile couples wherein both members are carriers of the ESR1-NCD1 deletion is statistically greater than expected on the basis of the frequency of this variant in infertile men and women.

The results agree with prior observations from other studies which show an adverse effect of exogenous substances with oestrogenic capacity on the reproductive health of men and women (Joffee, 2003). Therefore, we consider that couples wherein both members are carriers of the ESR1-NCD1 deletion may be more sensitive to the deleterious action of these substances and, therefore, that this variant provides a higher sensitivity to exogenous substances that are harmful for reproductive health.

### Example 9. Analysis of the ESR1-NCD1 variant on osteoporosis

The statistical analyses performed on the ESR1-NCD1 variant show that said variant has a protective effect against osteoporosis in post-menopausal women. The statistical significance p-value is 0.037 and the relative risk value is 0.673, the lower and upper confidence limits being 0.464 and 0.976, respectively. These results indicate that subjects who carry the ESR1-NCD1 variant have a 32.7% lower risk of suffering osteoporosis.

Taking into consideration the protective effect exerted by oestrogens at the bone level, the results of this invention once again indicate that the ESR1-NCD1 deletion has a protective effect against osteoporosis thanks to an increased sensitivity of the receptor to the action of oestrogens or oestrogenic substances also at the bone level.

### Example 10. Analysis of the ESR1-NCD1 variant in breast cancer

After analysing the genetic and epidemiological data in the series, it was observed that the ESR1-NCD1 variant significantly interacted (OR=0.68; p=0.013) with a variant of the ESR2 gene (rs4986938), following a double-dominance model, in accordance with the HFCC analysis software (PCT/EP2007/050264).

**Table 8 represents the interaction model of ESR1-NCD1 with the rs4986938 variant of ESR2 associated with the risk of suffering breast cancer in the Spanish population.**

| | | | | |
|---|---|---|---|---|
| E | | ESR1-NCD1 | | |
| S | | II | ID | DD |
| R | GG | 0 | 1 | 1 |
| 2 | GA | 1 | 1 | 1 |
| | AA | 1 | 1 | 1 |

| | | | | |
|---|---|---|---|---|
| II: Absence of the ESR1-NCD1 variant; ID: one copy of the ESR1-NCD1 variant; DD: two copies of the ESR1-NCD1 variant. | | | | |

HFCC compares the presence of the pattern described in the cases (1) and the controls (0) using a χ2 test with one degree of freedom.

This type of interactions was already previously reported for another disease related to the levels of endogenous oestrogens in the opposite sense, osteoporosis, which fits the established theories that classify breast cancer and osteoporosis as opposite, oestrogen-dependent phenotypes (Morón et al., 2006).

In view of these results, it may be concluded that the presence of the ESR1-NCD1 deletion increases the risk of the appearance of breast cancer in certain groups of women in the general population, probably depending on other factors, both environmental and genetic. As an example of this interaction, the effect of both variants, ESR1-NCD1 and the rs4986938 variant of ESR2, is shown.

Another significant discovery arose from a study performed on different tumour types and the effect that ESR1-NCD1 might exert on each of them. From the 1980s, it has been known that the expression of the oestrogen receptor in the tumour is associated with a histological degree with a higher level of cell differentiation, and this parameter is precisely one of the best indicators with prognostic value used in clinical practise (Fisher et al., 1981). When studying the role that ESR1-NCD1 might be exerting on OR(+) tumours (those that express oestrogen receptor alpha), it was observed that the presence of ESR1-NCD1 alone conditioned the appearance of a subtype of breast cancer with a higher degree of differentiation (Degree 1 (27%), Degree 2 (13%), Degree 3 (13%), χ2=6.5, p=0.04). Table 9 shows the distribution of ESR1-NCD1 according to the histological degree in OR(+) tumours:

| | HISTOLOGICAL DEGREE | | | |
|---|---|---|---|---|
| ESR1-NCD1 | 1 | 2 | 3 | |
| II | 40 | 113 | 78 | 231 |
| ID+DD | 15 | 17 | 12 | 44 |
| Total | 55 | 130 | 90 | 275 |

| | | | | |
|---|---|---|---|---|
| II: Absence of the ESR1-NCD1 variant; ID: one copy of the ESR1-NCD1 variant; DD: two copies of ESR1-NCD1 variant. | | | | |

Using a χ2 test with two degrees of freedom, the presence of allele D is found to be significantly over-represented amongst women with tumours of histological degree 1 (χ2=6.5, p=0.04).

This suggests that cells that express the oestrogen receptor and, moreover, have at least one mutant allele of ESR1-NCD1, have a phenotype of hypersensitivity to oestrogen levels that correlates with a higher degree of cell differentiation.

At the same time, it was shown that ESR1-NCD1 was associated with a lower incidence of debut metastases (presence of metastasis in the first diagnosis) amongst OR(+) tumours. Although 5% of breast cancer cases showed metastasis at the time of diganosis, the presence of only one allele reduced the risk of having debut metastasis by more than half (p=0.05), which is understood to be a better prognosis value.

On the other hand, there were two results that once again suggested a functional role of ESR1-NCD1 in the development and prognosis of the tumour. In the first place, women carrying the ESR1-NCD1 variant had a lower probability of developing a multifocal tumour (8.5% vs 3.4%, p=0.04). Furthermore, women with single tumours who exhibited the ESR1-NCD1 deletion had tumours with a smaller diameter (2.83 cm vs 2.38 cm, p=0.07).

Taking into consideration these data as a whole, it may be affirmed that the presence of the mutant variant of ESR1-NCD1 conditions the proliferation and differentiation capacity of tumoural cells, probably by responding to extracellular oestrogens levels in a differential manner, which ultimately causes a differential progression of the tumour depending on the tumour subtype and the presence of ESR1-NCD1.

It may be concluded that there is clear evidence that the presence of ESR1-NCD1 conditions the risk of suffering breast cancer in a differential manner in a significant portion of the Spanish population. This is probably due to the fact that the cells of women who carry the ESR1-NCD1 variant show a differential sensitivity to circulating oestrogens. Moreover, there is statistically significant evidence that the role of ESR1-NCD1 is not limited to the susceptibility to breast cancer, but also influences the progression of the tumour in a clearly determined subgroup of patients, since it affects the tumour's degree of aggressiveness.

Again, all these findings confirm that carriers of the deletion have a greater sensitivity to oestrogens: on the one hand, it has been proven beyond a doubt that breast carcinogenesis is oestrogen-dependent. On the other hand, independent scientific groups have also shown that breast tumours that acquire a larger number of copies in ESR1 during the tumourigenic process have a better evolution and response to tamoxifen. This type of somatic mutation that appears in tumoural lines would be equivalent to that of carriers of the NCD1 germ-line deletion, but would cause oversignalling of the ESR1 pathway by a radically different molecular mechanism.

### Example 11. Analysis of the ESR1-NCD1 variant on blood hypertension

A comparison of the frequency of the ESR1-NCD1 variant in both groups showed the following.

This variant is not associated with diastolic blood pressure. However, ESR1-NCD1 does have a protective effect on systolic blood pressure (p=0.042; OR= 0.65; 95% CI [0.43-0.98]). These results indicate that subjects who carry the ESR1-NCD1 variant have a 35% lower probability of suffering from systolic hypertension.

On the other hand, the effect of the ESR1-NCD1 variant was also detected in the group of subjects with systolic blood pressure > 130 mmHg and diastolic blood pressure < 85 mmHg (p=0.02; OR= 0.66; 95% CI [0.46-0.94]).

Again, the effect of the ESR1-NCD1 variant on an independent trait (in this case, blood hypertension) indicates that the ESR1-NCD1 variant increases the effects of oestrogens (protection against hypertension), which supports the hypothesis that this variant has a sensitising effect to the actions of oestrogens and/or substances with oestrogenic capacity.

### Example 12. Analysis of the ESR1-NCD1 variant in the Metabolic Syndrome

The statistical analyses show a nominal association of the ESR1-NCD1 variant with MS. According to the results of this invention, said variant acts as a protective factor, reducing the risk of suffering MS by 64% (p=0.007; OR=0.36; 95% CI [0.18-0.76]).

Taking these results into consideration, this invention proposes determining the ESR1-NCD1 variant as a method for the prognosis and/or diagnosis of the predisposition to suffer MS.

As was the case with blood hypertension, the effect observed for the ESR1-NCD1 variant on MS shows that this variant has the effect of increasing oestrogenic activity by increasing the sensitivity to the action thereof.

### REFERENCES

Albagha OM, Pettersson U, Stewart A, McGuigan FE, MacDonald HM, Reid DM, Ralston SH (2005) Association of oestrogen receptor alpha gene polymorphisms with postmenopausal bone loss, bone mass, and quantitative ultrasound properties of bone. J Med Genet 42, 240-246.
Alberti KG, Zimmet P, Shaw J; IDF Epidemiology Task Forcé Consensus Group (2005) The metabolic syndrome-a new worldwide defmition. Lancet, 366, 1059-1062.
Baccetti B, Capitani S, Collodel G, Strehler E, Piomboni P (2002) Recent advances in human sperm pathology. Contraception 65, 283-287.
Boyapati SM, Shu XO, Rúan ZX, Cai Q, Smith JR, Wen W, Gao YT, Zheng W (2005) Polymorphisms in ER-alpha gene interact with estrogen receptor status in breast cancer survival. Clin Cancer Res 11, 1093-1098.
Burt VL, Whelton P, Roccella EJ, Brown C, Cutler JA, Higgins M, Horan MJ, Labarthe D (1995) Prevalence of hypertension in the US adult population. Results from the Third National Health and Nutrition Examination Survey, 1988-1991. Hypertension 25, 305-313.
Carlsen E, Giwercman A, Keiding N, Skakkebaek NE (1992) Evidence for decreasing quality of semen during past 50 years. BMJ 305, 609-613.
Castellani C, Malerba G, Sangalli A, Delmarco A, Petrelli E, Rossini M, Assael BM, Mottes M (2006) The genetic background of osteoporosis in cystic fibrosis: association analysis with polymorphic markers in four candidate genes. J Cyst Fibros 5, 229-235.
Choi JY, Shin A, Park SK, Chung HW, Cho SI, Shin CS5 Kim H, Lee KM, Lee KH, Kang C, Cho DY, Kang D (2005) Genetic polymorphisms of OPG, RANK, and ESR1 and bone mineral density in Korean postmenopausal women. Calcif Tissue Int. 77, 152-159.
Collins J (2001) Cost-effectiveness of in vitro fertilization. Semin Reprod Med 19, 279-289.
Cooke HJ, Saunders PT (2002) Mouse models of male infertility. Nat Rev Genet 3, 790-801.
de Castro F, Ruiz R, Montoro L, Perez-Hernandez D, Sánchez-Casas Padilla E, Real LM, Ruiz A (2003): Role of follicle-stimulating hormone receptor Ser680Asn polymorphism in the efficacy of follicle-stimulating hormone. Fértil Steril 80, 571-576.
de Castro F, Morón FJ, Montoro L, Real LM, Ruiz A (2005) Pharmacogenetics of controlled ovarian hyperstinmlation. Pharmacogenomics 6, 629-637.
Dellenbach P, Nisand I, Moreau L, Feger B, Plumere C, Gerlinger P (1985) Transvaginal sonographically controlled follicle puncture for oocyte retrieval. Fértil Steril 44, 656-662.
Delvigne A, Rozenberg S (2002) Epidemiology and prevention of ovarian hyperstimulation syndrome (OHSS): a review. Hum Reprod Update 8, 559-577.
Deroo BJ, Korach KS (2006) Estrogen receptors and human disease. J Clin Invest 116, 561-570.
DeStefano AL, Larson MG, Mitchell GF, Benjamin EJ, Vasan RS, Li J, Corey D, Levy D (2004) Genome-wide sean for pulse pressure in the National Heart, Lung and Blood Institute's Framingham Heart Study. Hypertension 44, 152-155.
den Dunnen JT, Antonarakis SE (2001) Nomenclature for the descrtption of human sequence variations. Hum Genet 109, 121-124.
Dohle GR, Colpi GM, Hargreave TB, Papp GK, Jungwirth A, Weidner W; The EAU Working Group on Male Infertility (2005) EAU guidelines on male infertility. Eur Urol 48, 703-711.
Dumitrescu RG, Cotarla I (2005) Understanding breast cancer risk -- where do we stand in 2005?. J Cell Mol Med 9, 208-221.
Edwards RG, Steptoe PC, Purdy JM (1980) Establishing full-term human pregnancies using cleaving embryos grown in vitro. Br J Obstet Gynaecol 87, 737-756.
Ettinger B, Pressman A, Sklarin P, Bauer DC, Cauley JA, Cummings SR (1998) Associations between low levéis of serum estradiol, bone density, and fractures among elderly women: the study of osteoporotic fractures. J Clin Endocrinol Metab 83, 2239-2243.
Fisher ER, Osborne CK, McGuire WL, Redmond C, Knight WA 3rd, Fisher B, Bannayan G, Walder A, Gregory EJ, Jacobsen A, Queen DM, Bennett DE, Ford HC (1981) Correlation of primary breast cancer histopathology and estrogen receptor content. Breast Cancer Res Treat 1, 37-41.
Forman RG, Frydman R, Egan D, Ross C, Bariow DH (1990) Severe ovarian hyperstimulation syndrome using agonists of gonadotropin-reieasing hormone for in vitro fertilization: a European series and a proposal for prevention. Fértil Steril 3, 502-509.
Forti G, Krausz C (1998) Clinical review 100: Evaluation and treatment of the infertile couple. J Clin Endocrino! Metab 83, 4177-4188.
Galán JJ, Buch B; Cruz N, Segura A, Morón FJ, Bassas L, Martinez-Pineiro L, Real LM, Ruiz A (2005) Multilocus anaiyses of estrogen-related genes reveal involvement of the ESR1 gene in male infertility and the polygenic nature of the pathology. Fértil Sterii 84, 910-918.
Galán JJ, Guarducci E, Nuti F, González A, Ruiz M, Forti Gf Ruiz A, Krausz C (2006) ESR1 gene ÁGATA haplotype in human cryptorchidism and male infertility. Hum Reprod (sometido).
González-Mancha R, Galán JJ, Crespo C, Iglesias-Pérez L, Gonzalez-Perez A, Morón FJ, Moreno-Nogueira JA, Real LM, Hidalgo-Pascual M, Ruiz A, Royo JL (2007) ERα germline PvuII marker is significantly associated to familial forms of breast cancer and correlates with specific inmunohistochemical parameters.Med Sci20 Monit. Sometido.
Greb RR, Grieshaber K, Gromoll J, Sonntag B, Nicschlag E, Kiesel L, Simoni M (2005) A common single nucleotide polymorphism in exon 10 of the human follicle stimulating hormone receptor is a major determinant of length and hormonal dynamics of the menstrual cycle. J Clin Endocrinol Metab 90, 4866-4872.
Griffing GT, Melby JC, Holbrook M, Johnston ON (1991) Antihypertensive effects of an aromatase inhibitor in inbred salt-sensitive rats. Hypertension 17, 771-775.
Gruber CJ, Tschugguel W, Schneeberger C, Huber JC (2002) Production and actions of estrogens. N Engl J Med 346, 340-352.
Guarducci E, Nuti F, Becherini L, Rotondi M, Balercia G, Forti G, Krausz C (2006) Estrogen receptor alpha promoter polymorphism: stronger estrogen action is coupled with lower sperm count. Hum Reprod 21, 994-1001.
Hardy GH (1908). Mendelian proportion in a mixed population". Science 28:49-50.
Hess RA, Bunick D, Lubahn DB, Zhou Q, Bouma J (2000) Morphologic changes in efferent ductules and epididymis in estrogen receptor-alpha knockout mice. J Androl21, 107-121.
Herynk MH, Fuqua SA (2004) Estrogen receptor mutations in human disease. Endocr Rev. 25,869-898.
uHoh J, Wille A, Ott J (2001) Trimming, weighting, and grouping SNPs in human case-control association studies. Genome Res 11,2115-2119.
Holst F, Stahl PR, Ruiz C, Hellwinkel O, Jehan Z, Wendland M, Lebeau A, Terracciano L, Al-Kuraya K, Janicke F, Sauter G, Simón R (2007) Estrogen receptor alpha (ESR1) gene amplification is frequent in breast cancer. Nat Genet 39, 655-660.
Huang Z, Hankinson SE, Colditz GA, Stampfer MJ, Hunter DJ, Manson JE, Hennekens CH, Rosner B, Speizer FE, Willett WC (1997) Dual effects of weight and weight gain on breast cancer risk. JAMA 278, 1407-11.
Ioannidis JP, Ralston SH, Bennett ST, Brandi ML, Grinberg D, Karassa FB, Langdahl B, van Meurs JB, Mosekilde L, Scollen S, Albagha OM, Bustamante M, Carey AH, Dunning AM} Enjuanes A, van Leeuwen JP, Mavilia C, Masi L, McGuigan FE, Nogues X, Pols HA, Reid DM, Schuit SC, Sherlock RE, Uitterlinden AG;GENOMOS Study (2004) Differential genetic effects of ESR1 gene polymorphisms on osteoporosis outcomes. JAMA 292, 2105-2114.
Joffe M (2003) Infertility and environmental poüutants. Br Med Bull 68, 47-70.
Katz P, Nachtigall R, Showstack J (2002) The economic impact of the assisted reproductive technologies. Nat Cell Biol 4 Suppl, s29-s32.
Kligman I, Rosenwaks Z (2001) Differentiating clinical profiles: predicting good responders, poor responders, and hypenresponders. Fértil Steril 76, 1185-1190.
Korach KS (1994) Insights from the study of animáis lacking functional estrogen receptor. Science 266, 1524-1527.
Krausz C, Forti G, McElreavey K (2003) The Y chromosome and male fertility and infertility. Int J Androl 26, 70-75.
Krausz C, Degl'Innocenti S (2006) Y chromosome and male infertility: update, 2006. FronlBiosci 11,3049-3061.
Kukuvitis A, Georgiou I, Bouba I, Tsirka A, Giannouli CH, Yapijakis C, Tarlatzis B, Bontis J, Lolis D, Sofikitis N, Papadimas J (2002) Association of oestrogen receptor alpha polymorphisms and androgen receptor CAG trinucleotide repeats with niale infertility: a study in 109 Greek infertile men, Int J Androl 25, 149-152. Lalouel JM (2003) Large-scale search for genes predisposing to essential hypertension.Am J Hypertens 16, 163-166.
Lazaros L, Markoula S, Xita N, Giannopoulos S, Gogou P, Lagos G5 Kyritsis AP, Georgiou I (2007) Association of estrogen receptor-alpha gene polymorphisms with stroke risk in patients with metabolic syndrome. Acta Neurol Scand. En prensa.
Levy D, DeStefano AL, Larson MG, O'Donnell CJ, Lifton RP, Gavras H, Cupples LA, Myers RH (2000) Evidence for a gene influencing blood pressure on chromosome17. Genome scan linkage results for longitudinal blood pressure phenotypes in subjects from the framingham heart study. Hypertension 36, 477-483.
Lewontin RC, Kojima K. (1960). The evolutionary dynamics of complex polymorphisms. Evolution 14: 458-472.
Lissens W (1999) Genetics of male reproductive dysfunction. En: Fauser BCJM, 15 Rutherford AJ, Strauss III JF, Van Steirteghen A. Molecular Biology in reproductive medicine. New York: The Parthenon Publishing Group.
Lopata A, Johnston IW, Hoult IJ, Speirs AI (1980) Pregnancy following intrauterine implantation of an embryo obtained by in vitro fertilization of a preovulatory egg. Fertil Steril 33, 117-120.
Lottrup G, Andersson AM, Leffers H, Mortensen GK, Toppari J, Skakkebaek NE, Main KM (2006) Possible impact of phthalates on infant reproductive health. Int J Andro1 29, 172-180.
Maffei L, Rochira V, Zirilli L, Antunez P, Aranda C, Fabre B, Simone ML, Pignatti E, Simpson ER, Houssami S, Clyne CD, Carani C (2007) A novel compound heterozygous mutation of the aromatase gene in an adult man: reinforced evidence on the relationship between congenital oestrogen deficieney, adiposity and the metabolic syndrome. Clin Endocrinol (Oxf) 67, 218-224.
Melby JC, Azar ST, Delaney M, Holbrook M, Griffing GT, Johnston JO. 19-Norcorticosteroids in genetic hypertension (1993) Effects of inhibitors of 11 beta, 18, 19-hydroxylase activity. J Steroid Biochem Mol Biol 45, 13-18.
Morón FJ, Mendoza N, Vázquez F, Molero E, Quereda F, Salinas A, Fontes J, Martinez-Astorquiza T, Sánchez-Borrego R, Ruiz A (2006) Multilocus analysis of estrogen-related genes in Spanish postmenopausal women suggests an interactive role of ESR1, ESR2 and NRIP1 genes in the pathogenesis of osteoporosis. Bone 39, 213-221.
Moron FJ, Galan JJ, Ruiz A (2007) Controlled ovarian hyperstimulation pharmacogenetics: a simplified model to genetically dissect estrogen-related diseases. Pharmacogenomics 8, 775-85.
Nuñez R (2005) Descenso de la calidad seminal en España. XII Congreso Nacional de Andrologia. A Coruña. España.
O'Donnell L, Robertson KM, Jones ME, Simpson ER (2001) Estrogen and spermatogenesis. Endocr Rev 22, 289-318.
Onland-Moret NC, van Gils CH, Roest M, Grobbee DE, Peeters PH (2005) The estrogen receptor alpha gene and breast cancer risk (The Netherlands). Cancer Causes Control 16,1195-1202.
Ramirez-Lorca R, Grilo A, Martinez-Larrad MT, Manzano L, Serrano-Hernando FJ, Moron FJ, Perez-Gonzalez V, Gonzalez-Sanchez JL, Fresneda J, Fernandez-Parrilla R, Monux G, Molero E, Sánchez E, Martinez-Calatrava MJ, Saban-Ruiz J, Ruiz A, Saez ME, Serrano-Rios M (2007) Sex and Body Mass index Specific Regulation of Blood Pressure by CYP19A1 Gene Variants. Hypertension. En prensa.
Reckelhoff JF (2001) Gender differences in the regulation of blood pressure. Hypertension 37, 1199-1208.
Rivadeneira F, van Meurs JB, Kant J, Zillikens MC, Stolk L, Beck TJ, Arp P, Schuit SC, Hofman A, Houwing-Duistermaat JJ, van Duijn CM, van Leeuwen JP, Pols HA, Uitterlinden AG (2006) Estrogen receptor beta (ESR2) polymorphisms in interaction with estrogen receptor alpha (ESR1) and insulin-like growth factor I (IGF1) variants influence the risk of fracture in postmenopausal women. J Bone MinerRes 21, 1443-1456.
Royo JL, Moreno-Nogueira JA, Galán JJ, Gonzalez-Martin A, Ruiz A, González-Mancha R, Real LM (2006) Lack of association between NOS3 Glu298Asp and breast cancer risk: a case-control svudy. Breast Cancer Res Treat 100, 331-333.
Sanger F, Nicklen S, Coulson AR (1977) DNA sequencing with chain-terminating inhibitors. Proc Natl Acad Sci U S A 74, 5463-5467.
Sasieni PD (1997) From genotypes to genes: doubling the sample size. Biometrics 53, 1253-1261.
Semba S, Moriya T, Youssef EM, Sasano H (2000) An autopsy case of ovarian hyperstimulation syndrome with massive pulmonary edema and pleural effusion. Pathol Int 50, 549-52.
Shen Y, Li D.K, Wu J, Zhang Z, Gao E (2006) Joint effects of the CYP1A1 MspI, ERalpha PvuII, and ERalpha Xbal polymorphisms on the risk of breast cancer: results from a population-based case-control study in Shanghai, China. Cancer Epidemiol Biomarkers Prev 15, 342-347.
Shin A, Kang D, Nishio H, Lee MJ, Park SK, Kim SU, Noh DY, Choe KJ, Ahn SH, Hirvonen A, Kim JH, Yoo KY (2003) Estrogen receptor alpha gene polymorphisms and breast cancer risk. Breast Cancer Res Treat 80, 127-31.
Simoni M, Bakker E, Krausz C (2004) EAA/EMQN best practice guidelines for molecular diagnosis of y-chromosomal microdeletions. State of the art 2004. Int J Androl 27, 240-249.
Smith, E. P.; Boyd, J.; Frank, G. R.; Takahashi, H.; Cohen, R. M; Specker, B.; Williams, T. C; Lubahn, D. B.; Korach, K. S (1994) Estrogen resistance caused by a mutation in the estrogen-receptor gene in a man. New Eng. J. Med 331, 1056-1061.
Suzuki Y, Sasagawa I, Itoh K, Ashida J, Muroya K, Ogata T (2002) Estrogen receptor alpha gene polymorphism is associated with idiopathic azoospermia. Fertil Steril 78, 1341-1343.
Swan SH, Elkin EP, Fenster L (1997) Have sperm densities declined? A reanalysis of global trend data. Environ Health Perspect 105, 1228-1232.
Swan SH, Elkin EP, Fenster L (2000) The question of declining sperm density revisited: an analysis of 101 srudies published 1934-1996. Environ Health Perspect 108, 961-966.
Tarlatzis BC, Zepiridis L, Grimbizis G, Bontis J (2003) Clinical management of low ovarian response to stimulation for IVF: a systematic review. Hum Reprod Update 9, 61-76.
te Velde ER, Pearson PL (2002) The variability of female reproductive ageing. Hum Reprod Update 8, 141-154.
Toumaye H (2002) Gamete source and manipulation. En Vayena E, Rowe PJ, Griffin PD (eds). Current practices and controversies in assisted reproduction. World Health Organization, Geneva, Switzerland, pp. 83-101.
Tregouet DA, Escolano S, Tiret L, Mallet A, Golmard JL (2004). A new algorithm for haplotype-based association analysis: the Stochastic-EM algorithm. Ann Hum Genet. 68(Pt 2): 165-77.
Wedren S, Lovmar L, Humphreys K, Magnusson C, Melhus H, Syvanen AC, Kindmark A, Landegren U, Feraier ML, Stiger F, Persson I, Baron J, Weiderpass E (2004) Oestrogen receptor alpha gene haplotype and postmenopausal breast cancer risk: a case control study. Breast Cancer Res 6, R437-49.
Weinberg W. (1908). Über den Nachweis der Vererbung beim Menschen". Jahresh. Verein f. vaterl. Natkd in Wüttemberg 64:368-382.
World Health Organization (WHO) (2000) WHO Manual for the Standardised Investigation and Diagnosis of the Infertile Couple. Cambridge: Cambridge University Press.
Yaich L, Dupont WD, Cavener DR, Parl FF (1992) Analysis of the PvuII restriction fragment-length polymorphism and exon structure of the estrogen receptor gene in breast cancer and peripheral blood. Cancer Res 52, 77-83.
Yoshida R, Fukami M, Sasagawa I, Hasegawa T, Kamatani N, Ogata T (2005) Association of cryptorchidism with a specific haplotype of the estrogen receptor alpha gene: implication for the susceptibility to estrogenic environmental endocrine disruptors. J Clin Endocrinol Metab 90, 4716-4721.
Yoshidome K, Shibata MA, Couldrey C, Korach KS, Green JE (2000) Estrogen promotes mammary tumor development in C3(1)/SV40 large T-antigen transgenic mice: paradoxical loss of estrogen receptoralpha expression during tumor progression. Cancer Res 60, 6901-6910.
Zalel Y, Katz Z, Caspi B, Ben-Hur H, Dgani R, Insler V (1992) Spontaneous ovarian hyperstimulation syndrome concomitant with spontaneous pregnancy in a woman with polycystic ovary disease. Am J Obstet Gynecol 67, 122-124.

## Claims

1. Method for the *in vitro* prognosis and/or diagnosis of hypersensitivity to oestrogens and/or substances with oestrogenic activity, which comprises isolating a genomic DNA sample from the subject and determining the presence or absence therein of at least the ESR1-NCD1 variant of the oestrogen receptor alpha gene, **characterised in that** it is a deletion located in intron 6 of said ESR1 gene.

2. Method for the *in vitro* prognosis and/or diagnosis, as claimed in claim 1, of the predisposition to suffer oestrogen-related diseases (ERD) or to prognosticate a positive response to controlled ovarian hyperstimulation, which comprises isolating a genomic DNA sample from the subject and determining the presence or absence therein of at least the ESR1-NCD1 variant of the oestrogen receptor alpha gene, **characterised in that** it is a deletion located in intron 6 of said ESR1 gene.

3. Method, as claimed in claim 2, where the ERD disease is selected from the group formed by: osteoporosis, breast cancer, couple infertility, male idiopathic infertility, blood hypertension or metabolic syndrome.

4. Method, as claimed in claims 1 or 2, wherein the presence or absence of the ESR1-NCD1 deletion is determined by analysing fragments of the ESR1 gene previously amplified from the genomic DNA sample, using PCR conditions and primers that produce, as the amplification product of the normal allele, a fragment thereof that comprises all or part of the 2,244 nucleotides of the ESR1-NCD1 deletion, and lead to the amplification of some fragment thereof as the amplification product of the allele carrying the deletion; the fragment amplified from the allele carrying the deletion may be identical to the fragment amplified from the normal allele, except for the absence of the nucleotides pertaining to the deletion area, or there may be other differences in the sequence of fragments amplified from both alleles.

5. Method, as claimed in claim 4, wherein the presence or absence of the ESR1-NCD1 deletion is determined by analysing fragments of the ESR1 gene previously amplified from the genomic DNA sample, using PCR conditions and primers that produce, as the amplification product of the normal allele, a fragment thereof that comprises all of the 2,244 nucleotides of the ESR1-NCD1 deletion and, as the amplification product of the allele carrying the deletion, a fragment identical to that amplified from the normal allele, except for the absence of the nucleotides pertaining to the deletion area.

6. Method, as claimed in claim 5, wherein the presence or absence of the ESR1-NCD1 deletion is determined by analysing previously amplified fragments of the ESR1 gene by determining the presence or absence of a PCR product amplified from the allele carrying the deletion, discriminating between it and the PCR product amplified from the normal allele thanks to the different position of the bands obtained from one or the other fragment after subjecting the fragments of the ESR1 gene previously amplified from the genomic DNA sample to conventional electrophoresis.

7. Method, as claimed in claim 6, wherein the fragments of the ESR1 gene subject to electrophoresis have been previously amplified using at least one oligonucleotide selected from A1 (SEQ ID NO:37) and ED-F (SEQ ID NO:46) as the direct primer.

8. Method, as claimed in claim 7, wherein the fragments of the ESR1 gene subject to electrophoresis have been previously amplified using an oligonucleotide selected from R2 (SEQ ID NO:44), R3 (SEQ ID NO:45) and A2 (SEQ ID NO:40) as the indirect primer.

9. Method, as claimed in claim 8, wherein the fragments of the ESR1 gene subject to electrophoresis have been previously amplified using oligonucleotide A1 (SEQ ID NO:37) as the direct primer and oligonucleotide R2 (SEQ ID NO:44) as the reverse primer.

10. Method, as claimed in any of claims 4 to 9, wherein the presence of the ESR1-NCD1 deletion is confirmed by sequencing at least the fragment of the ESR1 gene purified from the electrophoresis band whose location in the gel indicates that the fragment has a size compatible with the absence of the 2,244-nucleotide fragment of the ESR1-NCD1 deletion.

11. Method, as claimed in claim 10, wherein the sequencing is performed using the Sanger method or a variant thereof.

12. Method, as claimed in claim 11, wherein the sequencing is performed using a variant of the Sanger method where the sequencing reaction is performed in the presence of the ddNTPs pertaining to each of the four nucleotides, A, C, G and T, each of the ddNTPs being marked with a different fluorescent molecule.

13. Method, as claimed in claim 12, wherein the sequencing is performed using an oligonucleotide selected from the oligonucleotides used as the pair of primers in the previous amplification of fragments of the ESR1 gene as the sequencing primer.

14. Method, as claimed in claim 4, wherein the presence or absence of the ESR1-NCD1 deletion is determined by analysing fragments of the ESR1 gene previously amplified from the genomic DNA sample using three PCR primers: a pair of primers, the respective binding sites whereof are common to both the normal allele and the allele carrying the deletion, where the direct primer binds to the area located at the 5'-end with respect to the deletion area and the reverse primer binds to the area located at the 3'-end with respect to the deletion area, and a third primer, direct or reverse, which binds to the area of the ESR1 gene that is absent from the allele carrying the deletion, and the amplification of the fragments is performed using amplification conditions such that the normal allele leads to the amplification of only the fragment thereof comprised between the third primer and the corresponding primer common to both alleles wherewith it may form an amplification pair.

15. Method, as claimed in claim 14, wherein the third primer that binds to the area of the ESR1 gene absent from the allele carrying the deletion acts as a reverse primer and the amplification conditions are such that the normal allele leads to the amplification of only the fragment comprised between said reverse primer and the direct primer present.

16. Method, as claimed in claim 15, wherein oligonucleotide ED-RW (SEQ ID NO:47) is used as the reverse primer that binds to the area of the ESR1 gene that is absent from the allele carrying the deletion, oligonucleotide ED-F (SEQ ID NO:46) is used as the direct primer that binds to the area located at the 5'-end with respect to the deletion area, oligonucleotide R2 (SEQ ID NO:44) is used as the reverse primer that binds to the area located at the 3'-end with respect to the deletion area and the amplification conditions are such that the normal allele leads to the amplification of only the fragment comprised between reverse primer ED-RW (SEQ ID NO:47) and direct primer ED-F (SEQ ID NO:46).

17. Method, as claimed in any of claims 14 to 16, wherein the presence or absence of the ESR1-NCD1 deletion is determined by analysing the previously amplified fragments of the ESR1 gene, by subjecting said fragments to pyrosequencing.

18. Method, as claimed in claim 17, wherein the previous amplification of the fragments of the ESR1 gene from the genomic DNA sample is performed using oligonucleotide ED-RW (SEQ ID NO:47) as the reverse primer that binds to the area of the ESR1 gene that is absent from the allele carrying the deletion, using oligonucleotide ED-F (SEQ ID NO:46) as the direct primer that binds to the area located at the 5'-end with respect to the deletion area, using oligonucleotide R2 (SEQ ID NO:44) as the reverse primer that binds to the area located at the 3'-end with respect to the deletion area, and using amplification conditions such that the normal allele leads to the amplification of only the fragment comprised between reverse primer ED-RW (SEQ ID NO:47) and direct primer ED-F (SEQ ID NO:46), and the pyrosequencing is performed using oligonucleotide ED-S (SEQ ID NO:48) as the primer.

19. Method, as claimed in claim 18, wherein oligonucleotides R2 (SEQ ID NO:44) and ED-W (SEQ ID NO:47) are marked with biotin at the 5'-end.

20. Method, as claimed in any of claims 14 to 16, wherein the presence or absence of the ESR1-NCD1 deletion is determined by analysing the previously amplified fragments of the ESR1 gene using amplification primers whereof at least one is bound to a marker, and subjecting them to capillary electrophoresis coupled with a procedure designed for size discrimination of the fragments by determining the time necessary to detect the signal of the marker coupled to the amplified fragments.

21. Method, as claimed in claim 20, wherein the fragments of the ESR1 gene have been previously amplified using amplification primers whereof at least one is bound to a fluorescent marker and the capillary electrophoresis is coupled with a procedure designed for size discrimination of the fragments by determining the time necessary to detect the signal of the fluorescent marker bound to the amplified fragments.

22. Method, as claimed in claim 21, wherein the fragments of the ESR1 gene have been previously amplified using oligonucleotide ED-RW (SEQ ID NO:47) as the reverse primer that binds to the area of the ESR1 gene that is absent from the allele carrying the deletion, using oligonucleotide ED-F (SEQ ID NO:46) as the direct primer that binds to the area located at the 5'-end with respect to the deletion area, using oligonucleotide R2 (SEQ ID NO:44) as the reverse primer that binds to the area located at the 3'-end with respect to the deletion area and using amplification conditions such that the normal allele leads to the amplification of only the fragment comprised between reverse primer ED-RW (SEQ ID NO:47) and direct primer ED-F (SEQ ID NO:46), and wherein oligonucleotide ED-F (SEQ ID NO:46) is marked with Cy5 at the 5'-end.

23. Method, as claimed in any of claims 1 to 22, wherein the predisposition to exhibit hypersensitivity to oestrogens and/or substances with oestrogenic activity is diagnosed in mammary tissue by determining, in the subject's isolated genomic DNA, in addition to the NCD1 deletion in the ESR1 gene, the presence or absence of the rs4986938 polymorphism of the ESR2 gene.

24. Method, as claimed in any of claims 1 to 22, wherein the predisposition to suffer from male idiopathic infertility is diagnosed by determining, in the subject's isolated genomic DNA, in addition to the NCD1 deletion in the ESR1 gene, the presence or absence of any of the following polymorphisms: rs851995, rs3020314 or rs910416.

25. An oligonucleotide selected from the group the sequence whereof is represented by SEQ ID NO:1, SEQ ID NO:2, SEQ ID NO:3, SEQ ID NO:4, SEQ ID NO:5, SEQ ID NO:6, SEQ ID NO:7, SEQ ID NO:8, SEQ ID NO:9, SEQ ID NO:10, SEQ ID NO:11, SEQ ID NO:12, SEQ ID NO:13, SEQ ID NO:14, SEQ ID NO:15, SEQ ID NO:16, SEQ ID NO:17, SEQ ID NO:18, SEQ ID NO:19, SEQ ID NO:20, SEQ ID NO:21 SEQ ID NO:22, SEQ ID NO:23, SEQ ID NO:24, SEQ ID NO:25, SEQ ID NO:26, SEQ ID NO:27, SEQ ID NO:28, SEQ ID NO:29, SEQ ID NO:30, SEQ ID NO:31, SEQ ID NO:32, SEQ ID NO:33, SEQ ID NO:34, SEQ ID NO:35, SEQ ID NO:36, SEQ ID NO:37 (A1), SEQ ID NO:38 (A3), SEQ ID NO:39 (A4), SEQ ID NO:40 (A2), SEQ ID NO:41 (F1), SEQ ID NO:42 (F2), SEQ ID NO:43 (R1), SEQ ID NO:44 (R2), SEQ ID NO:45(R3), SEQ ID NO:46 (ED-F), SEQ ID NO:47 (ED-RW), SEQ ID NO:48 (ED-S), SEQ ID NO:49, SEQ ID NO:50, SEQ ID NO:51, SEQ ID NO:52, SEQ ID NO:53, SEQ ID NO:54, SEQ ID NO:55, SEQ ID NO:56, SEQ ID NO:57, SEQ ID NO:58, SEQ ID N0:59, SEQ ID NO:60, SEQ ID NO:61 and SEQ ID NO:62, or a combination thereof.

26. Oligonucleotide, as claimed in claim 25, selected from the group the sequence whereof is represented by SEQ ID NO:1, SEQ ID NO:2, SEQ ID NO:13, SEQ ID NO:14, SEQ ID NO:27, SEQ ID NO:28, SEQ ID NO:37 (A1), SEQ ID NO:38 (A3), SEQ ID NO:39 (A4), SEQ ID NO:40 (A2), SEQ ID NO:41 (F1), SEQ ID NO:42 (F2), SEQ ID NO:43 (R1), SEQ ID NO:44 (R2), SEQ ID NO:45 (R3), SEQ ID NO:46 (ED-F), SEQ ID NO:47 (ED-RW) or SEQ ID NO:48 (ED-S), or a combination thereof.

27. A composition that comprises at least one oligonucleotide of claim 25.

28. An assay kit that comprises at least one of oligonucleotides A1 (SEQ ID NO:37), A3 (SEQ ID NO:38), A4 (SEQ ID NO:39), A2 (SEQ ID NO:40), F1 (SEQ ID NO:41), F2 (SEQ ID NO:42), R1 (SEQ ID NO:43), R2 (SEQ ID NO:44), R3 (SEQ ID NO:45), ED-F (SEQ ID NO:46), ED-RW (SEQ ID NO:47) or ED-S (SEQ ID NO:48), or combinations thereof.

29. Assay kit, as claimed in claim 28, that comprises at least one of the oligonucleotides from the group formed by A1 (SEQ ID NO:37), A4 (SEQ ID NO:39), F1 (SEQ ID NO:41), F2 (SEQ ID NO:42), ED-F (SEQ ID NO:46) and ED-S (SEQ ID NO:48), and/or at least one of the oligonucleotides from the group formed by A3 (SEQ ID NO:38), A2 (SEQ ID NO:40), R1 (SEQ ID NO:43), R2 (SEQ ID NO:44), R3 (SEQ ID NO:45) and ED-RW (SEQ ID NO:47).

30. Assay kit, as claimed in claim 29, that comprises at least one of the oligonucleotides from the group formed by A1 (SEQ ID NO:37), A4 (SEQ ID NO:39), F1 (SEQ ID NO:41), F2 (SEQ ID NO:42), ED-F (SEQ ID NO:46) and ED-S (SEQ ID NO:48), and/or at least one of the oligonucleotides from the group formed by A3 (SEQ ID NO:38), A2 (SEQ ID NO:40), R1 (SEQ ID NO:43), R2 (SEQ ID NO:44), R3 (SEQ ID NO:45) and ED-RW (SEQ ID NO:47).

31. Assay kit, as claimed in claim 30, that comprises at least one pair of oligonucleotides selected from the pairs:
a. A1 (SEQ ID NO:37) and R2 (SEQ ID NO:44);
b. A1 (SEQ ID NO:37) and R3 (SEQ ID NO:45);
c. A1 (SEQ ID NO:37) and A2 (SEQ ID NO:40);
d. ED-F (SEQ ID NO:46) and ED-RW (SEQ ID NO:47);
e. ED-F (SEQ ID NO:46) and R2 (SEQ ID NO:44);
f. ED-F (SEQ ID NO:46) and R3 (SEQ ID NO:45);
g. ED-F (SEQ ID NO:46) and A2 (SEQ ID NO:40);
or combinations thereof, and, optionally, oligonucleotide ED-S (SEQ ID NO:48).

32. Assay kit, as claimed in claim 31, that comprises at least the pair of oligonucleotides A1 (SEQ ID NO:37) and R2 (SEQ ID NO:44), and, optionally, dNTPs, a DNA polymerase I or the Klenow fragment thereof and a buffer, concentrated or at a concentration such that said polymerase may act therein.

33. Assay kit, as claimed in claim 31, that comprises at least the pair of oligonucleotides A1 (SEQ ID NO:37) and R2 (SEQ ID NO:44), and, optionally, dNTPs, a thermostable DNA polymerase and a buffer, concentrated or at a concentration such that said polymerase may act therein.

34. Assay kit, as claimed in claim 31, that comprises at least the set of oligonucleotides ED-F (SEQ ID NO:46), R2 (SEQ ID NO:44) and ED-RW (SEQ ID NO:47), and, optionally, dNTPs, a thermostable DNA polymerase, a buffer, concentrated or at a concentration such that said polymerase may act therein, and, also optionally, oligonucleotide ED-S (SEQ ID NO:48), a kit wherein at least one member of each of the oligonucleotide pairs is coupled to a marker at the 5'-end that allows for the detection thereof.

35. Assay kit, as claimed in claim 34, wherein oligonucleotides ED-RW (SEQ ID NO:47) and R2 (SEQ ID NO:44) are marked at the 5'-end.

36. Assay kit, as claimed in claim 35, wherein oligonucleotides ED-RW (SEQ ID NO:47) and R2 (SEQ ID NO:44) are marked with biotin at the 5'-end.

37. Assay kit, as claimed in claim 34, wherein oligonucleotide ED-F (SEQ ID NO:46) is marked at the 5'-end.

38. Assay kit, as claimed in claim 37, wherein oligonucleotide ED-F (SEQ ID NO:46) is marked with a fluorochrome.

39. Assay kit, as claimed in claim 38, wherein the fluorochrome is Cy5.

40. Kit, as claimed in any of claims 28 to 39, that additionally comprises one or more oligonucleotides from the group formed by SEQ ID NO:1, SEQ ID NO:2, SEQ ID NO:13, SEQ ID NO:14, SEQ ID NO:27 and SEQ ID NO:28.

41. Kit, as claimed in claim 40, that comprises at least one of the pairs selected from the group formed by:
a) SEQ ID NO: 1 and SEQ ID NO:2;
b) SEQ ID NO:13 and SEQ ID NO:14;
c) SEQ ID NO:27 and SEQ ID NO:28,
or combinations thereof.

42. Kit, as claimed in claim 41, wherein at least one of the oligonucleotides in each of the pairs present,
a) SEQ ID NO: 1 and SEQ ID NO: 2;
b) SEQ ID NO:13 and SEQ ID NO:14;
c) SEQ ID NO:27 and SEQ ID NO:28,
is bound to a marker at the 5'-end.

43. Kit, as claimed in claim 42, wherein the marker bound at the 5'-end to one of the oligonucleotides in each of the pairs present,
a) SEQ ID NO: 1 and SEQ ID NO: 2;
b) SEQ ID NO: 13 and SEQ ID NO: 14;
c) SEQ ID NO: 27 and SEQ ID NO:28,
is biotin.

44. Use of a variant of the human oestrogen receptor alpha (ESR1) that exhibits the ESR1-NCD1 deletion in intron 6 of said gene for the *in vitro* prognosis and/or diagnosis of oestrogen hypersensitivity, by determining the presence or absence of said variant independently, or by additionally determining the nucleotide present in the location pertaining to at least one polymorphism of the ESR1 gene selected from: rs851995, rs3020314 or rs910416, or the rs4986938 polymorphism of the ESR2 gene.


SEQUENCE LISTING

<110>  NEOCODEX, S.L.

<120>  METHOD FOR THE IN VITRO PROGNOSIS AND/OR DIAGNOSIS OF
       HYPERSENSITIVITY TO OESTROGENS OR SUBSTANCES WITH OESTROGENIC
       ACTIVITY

<130>  PCT-01735

<160>  81

<170>  PatentIn version 3.5

<210>  1
<211>  20
<212>  DNA
<213>  Artificial Sequence

<220>
<223>  PCR direct primer marked with biotin at 5´-end used for the
       genotyping of rs851995 polymorphism of ESR1 human gene

<400>  1

catttcgaat ccgtccaaca                                                  20


<210>  2
<211>  21
<212>  DNA
<213>  Artificial Sequence

<220>
<223>  PCR indirect primer used for the genotyping of rs851995
       polymorphism of ESR1 human gene

<400>  2

tgactgaagt tgaaggctgg a                                                21


<210>  3
<211>  22
<212>  DNA
<213>  Artificial Sequence

<220>
<223>  PCR direct primer marked with biotin at 5´-end used for the
       genotyping of rs1285057 polymorphism of ESR1 human gene

<400>  3

atttccctcc agaaatgtgt gc                                               22


<210>  4
<211>  22
<212>  DNA

<213>  Artificial Sequence

<220>
<223>  PCR indirect primer  used for the genotyping of rs1285057
       polymorphism of ESR1 human gene

<400>  4

tttggccagc acttaattgt cc                                              22


<210>  5
<211>  24
<212>  DNA
<213>  Artificial Sequence

<220>
<223>  PCR direct primer  used for the genotyping of rs3844508
       polymorphism of ESR1 human gene

<400>  5

tgaagaagaa agtaggaaca tgcc                                            24


<210>  6
<211>  24
<212>  DNA
<213>  Artificial Sequence

<220>
<223>  PCR indirect primer marked with biotin at 5´-end used for the
       genotyping of rs3844508 polymorphism of ESR1 human gene

<400>  6

ctagagtgta ctacctaaag tgtg                                            24


<210>  7
<211>  22
<212>  DNA
<213>  Artificial Sequence

<220>
<223>  PCR direct primer used for the genotyping of rs8179176
       polymorphism of ESR1 human gene

<400>  7

actgatatcc agggttatgt gg                                              22


<210>  8
<211>  22
<212>  DNA
<213>  Artificial Sequence

<220>

<223>  PCR indirect primer marked with biotin at 5´-end used for the
       genotyping of rs8179176 polymorphism of ESR1 human gene

<400>  8

cagaaccatt agagaccaat gc                                              22


<210>  9
<211>  22
<212>  DNA
<213>  Artificial Sequence

<220>
<223>  PCR direct primer used for the genotyping of rs1643821
       polymorphism of ESR1 human gene

<400>  9

attgtgttct gttgccttct cc                                              22


<210>  10
<211>  22
<212>  DNA
<213>  Artificial Sequence

<220>
<223>  PCR indirect primer marked with biotin at 5´-end used for the
       genotyping of rs1643821 polymorphism of ESR1 human gene

<400>  10

gaattgtcct taaccttcat gc                                              22


<210>  11
<211>  21
<212>  DNA
<213>  Artificial Sequence

<220>
<223>  PCR direct primer used for the genotyping of rs1643821
       polymorphism of ESR1 human gene

<400>  11

tgccagcaaa actctagaag a                                               21


<210>  12
<211>  21
<212>  DNA
<213>  Artificial Sequence

<220>
<223>  PCR indirect primer marked with biotin at 5´-end used for the
       genotyping of rs6927072 polymorphism of ESR1 human gene

<400>  12

tcatgtccag tgttttgtcc a                                                21


<210>  13
<211>  21
<212>  DNA
<213>  Artificial Sequence

<220>
<223>  PCR direct primer marked with biotin at 5´-end used for the
       genotyping of rs3024314 polymorphism of ESR1 human gene

<400>  13

aaccctgctc tcaaactgtt g                                                21


<210>  14
<211>  21
<212>  DNA
<213>  Artificial Sequence

<220>
<223>  PCR indirect primer used for the genotyping of rs2030314
       polymorphism of ESR1 human gene

<400>  14

aaagctgcat attgccagtc c                                                21


<210>  15
<211>  21
<212>  DNA
<213>  Artificial Sequence

<220>
<223>  PCR direct primer used for the genotyping of rs1643821
       polymorphism of ESR1 human gene

<400>  15

atttgagagc catctccgta g                                                21


<210>  16
<211>  20
<212>  DNA
<213>  Artificial Sequence

<220>
<223>  PCR indirect primer marked with biotin at 5´-end used for the
       genotyping of rs3020411 polymorphism of ESR1 human gene

<400>  16

cctcccaaag tgctgagatt                                                  20

<210>  17
<211>  22
<212>  DNA
<213>  Artificial Sequence

<220>
<223>  PCR direct primer marked with biotin at 5´-end used for the
       genotyping of rs926779 polymorphism of ESR1 human gene

<400>  17

gcattgacca gattcttcag tc                                              22


<210>  18
<211>  22
<212>  DNA
<213>  Artificial Sequence

<220>
<223>  PCR indirect primer used for the genotyping of rs926779
       polymorphism of ESR1 human gene

<400>  18

acgtgcgtag ttagcaaaca tc                                              22


<210>  19
<211>  24
<212>  DNA
<213>  Artificial Sequence

<220>
<223>  PCR direct primer marked with biotin at 5´-end used for the
       genotyping of rs3020364 polymorphism of ESR1 human gene

<400>  19

tcatttggga gactcttatt gtcc                                            24


<210>  20
<211>  24
<212>  DNA
<213>  Artificial Sequence

<220>
<223>  PCR indirect primer used for the genotyping of rs3020364
       polymorphism of ESR1 human gene

<400>  20

ctacaaaaca gaatcaacgt cctg                                            24


<210>  21

<211>  21
<212>  DNA
<213>  Artificial Sequence

<220>
<223>  PCR direct primer marked with biotin at 5´-end used for the
       genotyping of rs6932902 polymorphism of ESR1 human gene

<400>  21

ggatatatac ccagtagtgg g                                                21


<210>  22
<211>  21
<212>  DNA
<213>  Artificial Sequence

<220>
<223>  PCR indirect primer used for the genotyping of rs6932902
       polymorphism of ESR1 human gene

<400>  22

taaaagggtc ttgggcatgg a                                                21


<210>  23
<211>  21
<212>  DNA
<213>  Artificial Sequence

<220>
<223>  PCR direct primer used for the genotyping of rs3020371
       polymorphism of ESR1 human gene

<400>  23

ttccactcac gtgcattgtg c                                                21


<210>  24
<211>  21
<212>  DNA
<213>  Artificial Sequence

<220>
<223>  PCR indirect primer marked with biotin at 5´-end used for the
       genotyping of rs3020371 polymorphism of ESR1 human gene

<400>  24

catccactgt gtggatcata c                                                21


<210>  25
<211>  20
<212>  DNA
<213>  Artificial Sequence

<220>
<223>  PCR direct primer marked with biotin at 5´-end used for the
      genotyping of rs3020375 polymorphism of ESR1 human gene

<400>  25

taattctcag gagcgtgtgg                                                  20


<210>  26
<211>  20
<212>  DNA
<213>  Artificial Sequence

<220>
<223>  PCR indirect primer used for the genotyping of rs3020375
      polymorphism of ESR1 human gene

<400>  26

gtctctgcag cactcaatac                                                  20


<210>  27
<211>  19
<212>  DNA
<213>  Artificial Sequence

<220>
<223>  PCR direct primer marked with biotin at 5´-end used for the
      genotyping of rs910416 polymorphism of ESR1 human gene

<400>  27

aggaaccgtg tcgctgtct                                                   19


<210>  28
<211>  20
<212>  DNA
<213>  Artificial Sequence

<220>
<223>  PCR indirect primer used for the genotyping of rs910416
      polymorphism of ESR1 human gene

<400>  28

ctccaatttt ggcaccatca                                                  20


<210>  29
<211>  20
<212>  DNA
<213>  Artificial Sequence

<220>
<223>  PCR direct primer marked with Cy5 at 5´-end used to amplify the

D3S1358 region of the microsatellite marker

<400>  29

acagaagtct gggatgtgga                                                   20


<210>  30
<211>  20
<212>  DNA
<213>  Artificial Sequence

<220>
<223>  PCR indirect primer used to amplify the D3S1358 region of the
       microsatellite marker

<400>  30

gcccaaaaag acagacagaa                                                   20


<210>  31
<211>  20
<212>  DNA
<213>  Artificial Sequence

<220>
<223>  PCR direct primer marked with Cy5 at 5´-end used to amplify the
       CSF1PO region of the microsatellite marker

<400>  31

aacctgagtc tgccaaggac                                                   20


<210>  32
<211>  20
<212>  DNA
<213>  Artificial Sequence

<220>
<223>  PCR indirect primer used to amplify the CSF1PO region of the
       microsatellite marker

<400>  32

ttccacacac cactggccat                                                   20


<210>  33
<211>  20
<212>  DNA
<213>  Artificial Sequence

<220>
<223>  PCR direct primer marked with Cy5 at 5´-end used to amplify the
       D13S317 region of the microsatellite marker

<400>  33

acagaagtct gggatgtgga                                                   20


<210>  34
<211>  20
<212>  DNA
<213>  Artificial Sequence

<220>
<223>  PCR indirect primer used to amplify the D13S317 region of the
       microsatellite marker

<400>  34

gcccaaaaag acagacagaa                                                   20


<210>  35
<211>  21
<212>  DNA
<213>  Artificial Sequence

<220>
<223>  PCR direct primer marked with Cy5 at 5´-end used to amplify the
       D21S11 region of the microsatellite marker




<400>  35

gtgagtcaat tccccaagtg a                                                 21


<210>  36
<211>  23
<212>  DNA
<213>  Artificial Sequence

<220>
<223>  PCR indirect primer used to amplify the D21S11 region of the
       microsatellite marker

<400>  36

gttgtattag tcaatgttct cca                                               23


<210>  37
<211>  21
<212>  DNA
<213>  Artificial Sequence

<220>
<223>  PCR direct primer used for the amplify the ESR1-NCD1 deletion
       region and rs3798573 polymorphism of ESR1 human gene

<400>  37

cgcagtgctg acttaaaatg t                                                21


<210>  38
<211>  21
<212>  DNA
<213>  Artificial Sequence

<220>
<223>  PCR indirect primer used for the amplify the ESR1-NCD1 deletion
       region and rs3798573 polymorphism of ESR1 human gene

<400>  38

tcacagatgc ttatgaatgc c                                                21


<210>  39
<211>  20
<212>  DNA
<213>  Artificial Sequence

<220>
<223>  PCR direct primer used for the amplify the rs9397483,
       rs3778087,rs37778088 and rs3778089 regions of polymorphism of
       ESR1 human gene

<400>  39

ggagggttga tttgttgtga                                                  20



<210>  40
<211>  21
<212>  DNA
<213>  Artificial Sequence

<220>
<223>  PCR indirect primer used for the amplify the ESR1-NCD1 deletion
       region and rs39397483, rs3778087, rs3778088 and rs3778089 regions
       of polymorphism of ESR1 human gene

<400>  40

tttctgttgc agagaatctg g                                                21


<210>  41
<211>  21
<212>  DNA
<213>  Artificial Sequence

<220>
<223>  PCR direct primer used for the amplify the intron 6 regions next
       to rs3020375 polymorphism of the ESR1 human gene

<400>  41

tccacagatg gggtggtgta g                                                21


<210>  42
<211>  21
<212>  DNA
<213>  Artificial Sequence

<220>
<223>  PCR direct primer used for the amplify the intron 6 regions next
       to rs3020375 polymorphism of the ESR1 human gene

<400>  42

gtctcatcaa gtcactggac c                                                21


<210>  43
<211>  21
<212>  DNA
<213>  Artificial Sequence

<220>
<223>  PCR imdirect primer used for the amplify the intron 6 regions
       next to rs9397483 polymorphism of the ESR1 human gene

<400>  43

ggctaagtgc tgtgagtttg g                                                21


<210>  44
<211>  22
<212>  DNA
<213>  Artificial Sequence

<220>
<223>  PCR indirect primer used for the amplify the intron 6 regions
       next to rs9397483 polymorphism and to ESR1-NCD1region of the ESR1
       human gene

<400>  44

tttaccatca gatcatgagg tc                                               22


<210>  45
<211>  22
<212>  DNA
<213>  Artificial Sequence

<220>
<223>  PCR indirect primer used for the amplify the intron 6 region next
       to rs9397483 polymorphism of the ESR1 human gene

<400>  45

cacttacgtg tttaacagca tc                                                22

<210>  46
<211>  20
<212>  DNA
<213>  Artificial Sequence

<220>
<223>  PCR direct primer used for the amplify the ESR1-NCD1 deletion
       region of the ESR1 human gene

<400>  46

tgaaaactac tggcatcgac                                                    20

<210>  47
<211>  20
<212>  DNA
<213>  Artificial Sequence

<220>
<223>  PCR direct primer used for the amplify the ESR1 human gene do not
       exhibit the ESR1-NCD1 deletion region

<400>  47

gtctctgcag cactcaatac                                                    20

<210>  48
<211>  17
<212>  DNA
<213>  Artificial Sequence

<220>
<223>  Sequencing primer

<400>  48

actgtgccag gcactgc                                                       17

<210>  49
<211>  20
<212>  DNA
<213>  Artificial Sequence

<220>
<223>  Primer used for sequencing fragments containing rs851995
       nucleotide polymorphism

<400>  49

tttgtgctta atttaatctt                                                    20

<210>  50
<211>  18
<212>  DNA
<213>  Artificial Sequence

<220>
<223>  Primer used for sequencing fragments containing nucleotide
       polymorphism rs1285057

<400>  50

cagccagaat aatgagga                                                    18


<210>  51
<211>  15
<212>  DNA
<213>  Artificial Sequence

<220>
<223>  Primer used for sequencing fragments containing nucleotide
       polymorphism rs3844508

<400>  51

tggcctgagg tcttt                                                       15


<210>  52
<211>  15
<212>  DNA
<213>  Artificial Sequence

<220>
<223>  Primer used for sequencing fragments containing nucleotide
       polymorphism rs8179176

<400>  52

tccaaatgtc ccagc                                                       15


<210>  53
<211>  15
<212>  DNA
<213>  Artificial Sequence

<220>
<223>  Primer used for sequencing fragments containing nucleotide
       polymorphism rs1643821

<400>  53

ctggagttag tcctg                                                       15


<210>  54
<211>  16
<212>  DNA

<213>  Artificial Sequence

<220>
<223>  Primer used for sequencing fragments containing nucleotide
       polymorphism rs6927072

<400>  54

acaaggttaa aggatt                                                    16


<210>  55
<211>  15
<212>  DNA
<213>  Artificial Sequence

<220>
<223>  Primer used for sequencing fragments containing nucleotide
       polymorphism rs3020314

<400>  55

tgtccttaaa cagag                                                     15


<210>  56
<211>  18
<212>  DNA
<213>  Artificial Sequence

<220>
<223>  Primer used for sequencing fragments containing nucleotide
       polymorphism rs3020411

<400>  56
   ggttaaggca gttttttg                                                   18


<210>  57
<211>  20
<212>  DNA
<213>  Artificial Sequence

<220>
<223>  Primer used for sequencing fragments containing nucleotide
       polymorphism rs926779

<400>  57

tgctagtcgt tagtaaaaac                                                20


<210>  58
<211>  16
<212>  DNA
<213>  Artificial Sequence

<220>

<223>  Primer used for sequencing fragments containing nucleotide
       polymorphism rs3020364

<400>  58

gctggcctag atctca                                                    16


<210>  59
<211>  16
<212>  DNA
<213>  Artificial Sequence

<220>
<223>  Primer used for sequencing fragments containing nucleotide
       polymorphism rs6932902

<400>  59

gggagtcacc gttaag                                                    16


<210>  60
<211>  17
<212>  DNA
<213>  Artificial Sequence

<220>
<223>  Primer used for sequencing fragments containing nucleotide
       polymorphism rs3020371

<400>  60

tgaacctgtg ccttctt                                                   17


<210>  61
<211>  16
<212>  DNA
<213>  Artificial Sequence

<220>
<223>  Primer used for sequencing fragments containing nucleotide
       polymorphism rs3020375

<400>  61

gcacagttcc aagcat                                                    16


<210>  62
<211>  16
<212>  DNA
<213>  Artificial Sequence

<220>
<223>  Primer used for sequencing fragments containing nucleotide
       polymorphism rs910416

<400>  62

caaggcactg agacca                                                    16


<210>  63
<211>  3343
<212>  DNA
<213>  Homo sapiens


<220>
<221>  primer_bind
<222>  (1)..(21)
<223>  Binding site of the A1 primer (SEQ ID NO:37)

<220>
<221>  variation
<222>  (357)..(357)
<223>  rs3798573 polymorphism of ESR1 human gene: A=G

<220>
<221>  primer_bind
<222>  (861)..(880)
<223>  Binding site of the ED-F primer (SEQ ID NO:46)

<220>
<221>  variation
<222>  (963)..(963)
<223>  rs3020375 polymorphism of ESR1 human gene: A=C

<220>
<221>  primer_bind
<222>  (973)..(989)
<223>  Binding site of the ED-S primer (SEQ ID NO:48)

<220>
<221>  variation
<222>  (985)..(3228)
<223>  nucleotides absent in the allele carrying the ESD1-NCD1 deletion

<220>
<221>  primer_bind
<222>  (1101)..(1120)
<223>  Binding site of the ED-RW primer (SEQ ID NO:47)

<220>
<221>  primer_bind
<222>  (3322)..(3343)
<223>  Binding site of the R2 primer (SEQ ID NO:44)



<400>  63

cgcagtgctg acttaaaatg ttcttagtat gccaatgttg cttaaatatg aatgactgta   60

gcttctactt aaattggcaa ccgcaccaaa tataaactgc aaatgtttat agctcttatg  120

gaagtataaa agcaaaacaa attcttaaat taaatacaaa ctaagaggga aactgataat 180
aacaatgttt tgtgtaatta agttgctgtt tgcaacatgc ctggggcaga ctcctggcct 240
ctttgcatct gacatgagag gctaccaagg atgtggccac aactgggctc tcccagcact 300
tgcttgcgct cagaactgtg ccaaaacctt tgctcgccca gtgttctgca acgtccatca 360
tttggatttg gcaggaacac atcatttatg cattttgttt gcttctgtat ttagttgaga 420
tacttaaaat ataactgcta gattctaagg cattcataag catctgtgaa aaatgtgtta 480
cttaggaaac agttcagggt tccatggatt atctagattg gtgagtctta agactgacca 540
cacaattcat ctgggggtgt ggaggagggg agctttgaga aaaatctctg tatgagcacc 600
cacacccaga gattcctatg caattatccc agagtggtgc cttaatatca acatttgttt 660
aaaaccatcc cccaagacta aaatttgtga ccgggattta gaaccattga cttaattgat 720
gcagaacact ctgatattaa tgtcttcatg tcttttggaa tgtcattaag acaaaagcat 780
aaaattttaa aattcttaaa gacaatttaa agacctatga gaatccatct ttaattctca 840
ggagcgtgtg gaacctagtt tgaaaactac tggcatcgac aattgaattt ccactaaaat 900
aaaatagctc tctagtatat tacaaaacta cccattctgc aaactgcagg ggagctactg 960
ataatgcttg gaactgtgcc aggcactgcc tgcataaaaa tgagtaaggt ccacttcctc 1020
catggactgg gttgggtagg aggcaaagat aattaaccaa ttattttaat attatgagtt 1080
cagggttgta atgaaagtaa gtattgagtg ctgcagagac ccagcaaagg agtccttagc 1140
tgagaactga tcctttgctg agtgacgagg acattattga accgccaggc ttgatggcat 1200
gttgcatggt aacagaccag tacaccaaac agcaggagct gtagcagaga aagagtttaa 1260
taattgtgtg gcagccaaat aaggagacag aaggaaacct taaatccacc tcttgaaaaa 1320
gtctgggact aggcttttta agagaattct ggcaagaagg aggctgagga gctgggggta 1380
actgattggt taggacatag gagaggagac aataaggata tagaaactcc attcttgcac 1440
tgggtcagtt cctcggaggg ggtcctcact ctggctggca gcagtgaacc cattggaatg 1500
caggatctga aaaatatctc aaaatggcaa acttgagggg ttttttttag catcaaagat 1560
gttatctata gaagttagga cattgtgaca ggggctacgt gactttgaag tgttaagtgg 1620
ctgtcagaaa gtgagctatt gcagcggtcc ccaacctttt tggtaccagg gaccagcttc 1680
atgaaagaaa atttttccac agatggggtg gtgtagggtg gggtggggga tagcttcggg 1740
atgaaactga tccacctcag atcatcaggc attagttaga ttctcatgag gggcacacag 1800
cccagatccc tcacatgggc agttcacagt agggttcaca gccctatgag gttctaatgc 1860
cactgctgat ctgacaggtg gcagagctca ggcagtaatg cttccttgcc caccactcac 1920

ctcttgctgt gtggctcagt tcctgacaga ttacggactg gtaccagttt gcaacccagg 1980

cagctgggga cctctgggct gtagggtagg ctggttaatg cttagctgtg tttctattca 2040

aagcttatgc ttttgtttaa aacctagtaa tttggttttg ttaattttat gaagatgatt 2100

tcaaggatga gtaggattag ctggagtcag aaggacacaa gggaatttct ggcaacaaca 2160

ttaaactacc agtgaatgtc tcatcaagtc actggacctc taattctaag cagtactgtc 2220

aaccttatag gttatgatgt ataagatagt gttgtgatgt ataagatagt gcaaaaacct 2280

aagttaatat caccatttct gtagtactct ctatttcaaa ggaaaaaatc ttcagcagtc 2340

taatatccta ccaaaatttt gaagtcaaga cctccctaca ctaatcaaag aactagaggt 2400

gcaatcaggc catatatttg gaaaggccat aggtgccctc cctctgtcct tgttcccagc 2460

atactaaact atagtctaaa agactttaaa acttgaactg aagaaaacct taaggaaaca 2520

acaacaaaat tccatttatt tcctgagccc aaaagtagaa taattgtttt ttaatgaatt 2580

tttctttccc tgtaagacat actatgaatt gttcaatggg tattcaaagc atatgtaata 2640

gaatatgtat gctttcctga aaaattgaaa tggaacatca aactgttctg taaagttttg 2700

agtctagacc aaactcacag cacttagcca gaatatgtac atctgcatct gccactcatg 2760

gccccatcgc tccctccaac tgcttctctt gggctgaagg atggaaaagc aaactccctg 2820

cgttgagttc catcttgccc tgcatggcct cccccatggg gtcattgagc tccccaccag 2880

cttccacctc ttccagcact ttccgccaga gggcccgtgg ctgcttttgc agactgaaat 2940

tgattgtggt tgattcagct ccatttcctc tgagttttta ctttagatgt gctttttagg 3000

ttgcattttt cttctggcat ataagaaaga cagcaatatt ccatggaaca gagtgatgtc 3060

tgtggctttg tcaaccgttg tttgttagct tgatcaccat ttattgagtg tttactactt 3120

tccaggcatt gtgctaagtg gtagaaatac aaagttgcct ttacttgatt catctgtaaa 3180

atagagagag tagtagttcc tacagtgtgg tttatgtgag gcatagggac tgctccctga 3240

aacctaggga caactcccta gcagcgaccc agtgctcata tcaatagtgg taacaattag 3300

gaaggcagct cctacctcaa agacctcatg atctgatggt aaa 3343

<210> 64
<211> 260
<212> DNA
<213> Homo sapiens

<220>
<221> primer_bind
<222> (1)..(20)

<223>  Binding site of the ED-F primer (SEQ ID NO:46)

<220>
<221>  primer_bind
<222>  (113)..(129)
<223>  Binding site of the ED-S primer (SEQ ID NO:48)


<220>
<221>  misc_feature
<222>  (125)..(260)
<223>  absent nucleotides in the allele carrying the deletion

<220>
<221>  primer_bind
<222>  (241)..(260)
<223>  Binding site of the ED-RW primer (SEQ ID NO:47)

<400>  64

tgaaaactac tggcatcgac aattgaattt ccactaaaat aaaatagctc tctagtatat   60

tacaaaacta cccattctgc aaactgcagg ggagctactg ataatgcttg gaactgtgcc  120

aggcactgcc tgcataaaaa tgagtaaggt ccacttcctc catggactgg gttgggtagg  180

aggcaaagat aattaaccaa ttattttaat attatgagtt cagggttgta atgaaagtaa  240

gtattgagtg ctgcagagac                                              260


<210>  65
<211>  239
<212>  DNA
<213>  Homo sapiens


<220>
<221>  primer_bind
<222>  (1)..(20)
<223>  Binding site of the ED-F primer (SEQ ID NO:46)

<220>
<221>  primer_bind
<222>  (113)..(129)
<223>  Binding site of the ED-S primer (SEQ ID NO:48)

<220>
<221>  misc_feature
<222>  (125)..(239)
<223>  fragment located at 3'-end regarding the deletion

<220>
<221>  primer_bind
<222>  (218)..(239)
<223>  Binding site of the R2 primer (SEQ ID NO:44)

<400>  65

tgaaaactac tggcatcgac aattgaattt ccactaaaat aaaatagctc tctagtatat    60

tacaaaacta cccattctgc aaactgcagg ggagctactg ataatgcttg gaactgtgcc   120

aggcactgct ccctgaaacc tagggacaac tccctagcag cgacccagtg ctcatatcaa   180

tagtggtaac aattaggaag gcagctccta cctcaaagac ctcatgatct gatggtaaa    239

<210>  66
<211>  152
<212>  DNA
<213>  Homo sapiens

<220>
<221>  primer_bind
<222>  (1)..(20)
<223>  Binding site of the SEQ ID NO:1 primer

<220>
<221>  variation
<222>  (69)..(69)
<223>  rs8519195 polymorphism of ESR1 human gene: G=A

<220>
<221>  primer_bind
<222>  (71)..(90)
<223>  Binding site of the SEQ ID NO:49 primer

<220>
<221>  primer_bind
<222>  (132)..(152)
<223>  Binding site of the SEQ ID NO:2 primer

<400>  66

catttcgaat ccgtccaaca taccttcacc agattatgtt cccgattaat catcctcacc    60

gtgttactgc aagattaaat taagcacaaa ccacttacca tatccctgat cacccgtctc   120

cagccaacct ctccagcctt caacttcagt ca                                 152

<210>  67
<211>  183
<212>  DNA
<213>  Homo sapiens

<220>
<221>  primer_bind
<222>  (1)..(22)
<223>  Binding site of primer of the SEQ ID NO:3

<220>
<221>  variation
<222>  (60)..(60)
<223>  rs1285057 polymorphism of ESR1 human gene: T=C

<220>
<221>  primer_bind
<222>  (61)..(78)
<223>  Binding site of primer of the SEQ ID NO:50

<220>
<221>  primer_bind
<222>  (162)..(183)
<223>  Binding site of primer of the SEQ ID NO:4

<400>  67

atttccctcc agaaatgtgt gcacctattt aaatgtgtgt ggaggtgcac attttaacat   60

tcctcattat tctggctgcc ttctataaat caagctaccc tggtattatg gctctcctcc  120

agaaatctaa tcatttaaac acatgaagtg aatgtgtgaa tggacaatta agtgctggcc  180

aaa                                                                183


<210>  68
<211>  293
<212>  DNA
<213>  Homo sapiens


<220>
<221>  primer_bind
<222>  (1)..(24)
<223>  Binding site of the SEQ ID NO:5 primer´s

<220>
<221>  primer_bind
<222>  (139)..(153)
<223>  Binding site of the SEQ ID NO:151 primer´s

<220>
<221>  variation
<222>  (156)..(156)
<223>  rs3844508 polymorphism of ESR1 human gene: T=C

<220>
<221>  primer_bind
<222>  (270)..(293)
<223>  Binding site of the SEQ ID NO:6 primer´s

<400>  68

tgaagaagaa agtaggaaca tgccatttaa acatggctct ttcaacaaga gcttcttgga   60

gtaatagtta ggagactgat tggaaccaaa tacaagggct tgattgccac accatggaac  120

atacatgttt tctctgactg gcctgaggtc ttttatgcag aggaagaacc agatcaggtt  180

ggggagtata tatgatatat gtatattata tatatatatg tataccaggt atatatatgt  240

gtgtgtgtgt gtgtatatat gtgtatatac acactttagg tagtacactc tag        293

<210>  69
<211>  188
<212>  DNA
<213>  Homo sapiens


<220>
<221>  primer_bind
<222>  (1)..(22)
<223>  Binding site of the SEQ ID NO:7 primer´s

<220>
<221>  primer_bind
<222>  (93)..(107)
<223>  Binding site of the SEQ ID NO:52 primer´s

<220>
<221>  variation
<222>  (108)..(108)
<223>  rs8179176 polymorphism of ESR1 human gene: T=C

<220>
<221>  primer_bind
<222>  (167)..(188)
<223>  Binding site of the SEQ ID NO:8 primer´s

<400>  69

actgatatcc agggttatgt ggcaatgacg taaaaatttg aattgttatt tttttgacac   60

atgttctgtg ttgtccatca gttcatctga gttccaaatg tcccagctgt tttatgcttt  120

gtctctgttt cccagagacc ctgagtgtgg tctagagttg ggatgagcat tggtctctaa  180

tggttctg                                                            188


<210>  70
<211>  258
<212>  DNA
<213>  Homo sapiens


<220>
<221>  primer_bind
<222>  (1)..(22)
<223>  Binding site of the SEQ ID NO:9 primer´s

<220>
<221>  primer_bind
<222>  (136)..(150)
<223>  Binding site of the SEQ ID NO:53 primer´s

<220>
<221>  variation
<222>  (136)..(150)
<223>  rs1643821 polymorphism of ESR1 human gene: G=A

<220>
<221> primer_bind
<222> (237)..(258)
<223> Binding site of the SEQ ID NO:10 primer´s

<400> 70

attgtgttct gttgccttct ccaagggaaa ggagtgcttc cagaaaggcc attggaagtg 60

aggagtgtca cctgataata gctggctcct catcccacta ggttaacagg caacagatgg 120

gggccacaga gttggctgga gttagtcctg gttgccaagg gtaaatgggg ctgttaccat 180

gcaatggata gaaactagga tgctcctgtg tgtctcctta tatcctacca ggtgtagcat 240

gaaggttaag gacaattc 258

<210> 71
<211> 212
<212> DNA
<213> Homo sapiens

<220>
<221> primer_bind
<222> (1)..(21)
<223> Binding site of the SEQ ID NO:11 primer´s

<220>
<221> primer_bind
<222> (65)..(80)
<223> Binding site of the SEQ ID NO:54 primer´s

<220>
<221> variation
<222> (81)..(81)
<223> rs6927072 polymorphism of ESR1 human gene: T=G

<220>
<221> primer_bind
<222> (192)..(212)
<223> Binding site of the SEQ ID NO:12 primer´s

<400> 71

tgccagcaaa actctagaag aagaaactaa gaatttcaaa ttgacagttt gttaatgaga 60

aaagacaagg ttaaaggatt ttgttaaaca gatgaccaaa ataattattg gaaacttctg 120

cttctggcca aggtggaata ataagggcta catttacctt cccaccagaa aatataataa 180

caaacaaaaa ctggacaaaa cactggacat ga 212

<210> 72
<211> 231
<212> DNA

<213>  Homo sapiens


<220>
<221>  primer_bind
<222>  (1)..(21)
<223>  Binding site of the SEQ ID NO:13 primer´s

<220>
<221>  variation
<222>  (104)..(104)
<223>  rs3020314 polymorphism of ESR1 human gene: C=T

<220>
<221>  primer_bind
<222>  (105)..(119)
<223>  Binding site of the SEQ ID NO:55 primer´s

<220>
<221>  primer_bind
<222>  (211)..(231)
<223>  Binding site of the SEQ ID NO:14 primer´s

<400>  72

aaccctgctc tcaaactgtt gtggtgtcca gcatgctttg gcaaggtaat gaaagataac   60

atgacatgga catatgggtg acatcctgga gagatgacag aagcctctgt ttaaggacaa  120

gatttgccat ttagattttg cacccactgt ataataagag ccttaggatt gggctggaat  180

cgccctagca ggcatgatgg cagcccctct ggactggcaa tatgcagctt t           231


<210>  73
<211>  310
<212>  DNA
<213>  Homo sapiens


<220>
<221>  primer_bind
<222>  (1)..(22)
<223>  Binding site of the SEQ ID NO:17 primer´s

<220>
<221>  variation
<222>  (136)..(136)
<223>  rs926779 polymorphism of ESR1 human gene: G=A

<220>
<221>  primer_bind
<222>  (137)..(156)
<223>  Binding site of the SEQ ID NO:57 primer´s

<220>
<221>  primer_bind
<222>  (289)..(310)
<223>  Binding site of the SEQ ID NO:18 primer´s

<400>  73

gcattgacca gattcttcag tctttttaat tcattctgaa ataactgggc tacagtttca   60

tctgctttgt ggacagaagt gccacaaaga gccgaattgt cagtgcagac ccacatgaat  120

catagatctt aacgaggttt ttactaacga ctagcaaagg atacaagcta aaaatgggta  180

caagcaaaca cagcatcatt catcactgta aagactctga actatcacat ggaacttcaa  240

aaggattctt cttctttgct gcaatgtgtt ttgatttttg gagtgataga tgtttgctaa  300

ctacgcacgt                                                          310


<210>  74
<211>  221
<212>  DNA
<213>  Homo sapiens


<220>
<221>  primer_bind
<222>  (1)..(24)
<223>  Binding site of the SEQ ID NO:19 primer´s

<220>
<221>  variation
<222>  (74)..(74)
<223>  rs3020364 polymorphism of ESR1 human gene: A=G

<220>
<221>  primer_bind
<222>  (77)..(92)
<223>  Binding site of the SEQ ID NO:58 primer´s

<220>
<221>  primer_bind
<222>  (198)..(221)
<223>  Binding site of the SEQ ID NO:20 primer´s

<400>  74

tcatttggga gactcttatt gtcctatttg cactgaaaaa gtcactgaat cattatttta   60

gaactggaat aacacctgag atctaggcca gcactttgca agttgtgctc tatgggactt  120

ttcatggaag tggctgagga gttgccttga aggaaggcag agggagtggg tcttgggaca  180

cccttccagt tataaaacag gacgttgatt ctgttttgta g                       221


<210>  75
<211>  349
<212>  DNA
<213>  Homo sapiens

<220>
<221>  primer_bind
<222>  (1)..(21)
<223>  Binding site of the SEQ ID NO:21 primer´s

<220>
<221>  primer_bind
<222>  (204)..(219)
<223>  Binding site of the SEQ ID NO:59 primer´s

<220>
<221>  variation
<222>  (204)..(219)
<223>  rs6932902 polymorphism of ESR1 human gene: G=A

<220>
<221>  primer_bind
<222>  (329)..(349)
<223>  Binding site of the SEQ ID NO:22 primer´s

<400>  75

ggatatatac ccagtagtgg gattattgga tcaaatggta attatatttc taattttggg   60

cagggtcctc tgtagtagct ccataatggt tgtagtaatt tacatatcca accaagcacg  120

ccagggttca cttttcctca tgtcctcgcc gacaagcctg atattcttat ttgcctctta  180

gcgcttcagc ctttccctcg tgacttaacg gtgactccct tgagactact tgaaataata  240

agtttggatg gcaaggaaat acccttctgc tgtcaccctt tgccataaga ctgagttact  300

ttgtaaacaa agaagattta cttggtcttc catgcccaag accctttta              349


<210>  76
<211>  317
<212>  DNA
<213>  Homo sapiens


<220>
<221>  primer_bind
<222>  (1)..(21)
<223>  Binding site of the SEQ ID NO:23 primer´s

<220>
<221>  primer_bind
<222>  (133)..(149)
<223>  Binding site of the SEQ ID NO:60 primer´s

<220>
<221>  primer_bind
<222>  (150)..(150)
<223>  rs3020371 polymorphism of ESR1 human gene: C=T

<220>
<221>  primer_bind
<222>  (297)..(317)

<223>  Binding site of the SEQ ID NO:24 primer´s

<400>  76

ttccactcac gtgcattgtg cttttcattt cagtcgtttg tcctgtacat tgtagggtcc   60

agatcccaca atggctcttt attggatgag agttctggga gcagtgccac tcagctacat  120

ggtgccaggt cctgaacctg tgccttcttc ggtggagggc tggcacgtgc tgacagcttt  180

catgtgggca atctgggaac ttcagagaag gcaggcctat taagtgttaa gactccccac  240

cccgaacttt tactgagaaa aagtacccca gacaggaagt aaaattagcc agagttgtat  300

gatccacaca gtggatg                                                  317


<210>  77
<211>  289
<212>  DNA
<213>  Homo sapiens


<220>
<221>  protein_bind
<222>  (1)..(20)
<223>  Binding site of the SEQ ID NO:25 primer´s

<220>
<221>  variation
<222>  (132)..(132)
<223>  rs3020375 polymorphism of ESR1 human gene: A=C

<220>
<221>  protein_bind
<222>  (133)..(148)
<223>  Binding site of the SEQ ID NO:61 primer´s

<220>
<221>  protein_bind
<222>  (270)..(289)
<223>  Binding site of the SEQ ID NO:26 primer´s

<400>  77

taattctcag gagcgtgtgg aacctagttt gaaaactact ggcatcgaca attgaatttc   60

cactaaaata aaatagctct ctagtatatt acaaaactac ccattctgca aactgcaggg  120

gagctactga taatgcttgg aactgtgcca ggcactgcct gcataaaaat gagtaaggtc  180

cacttcctcc atggactggg ttgggtagga ggcaaagata attaaccaat tattttaata  240

ttatgagttc agggttgtaa tgaaagtaag tattgagtgc tgcagagac              289


<210>  78
<211>  149
<212>  DNA

<213>  Homo sapiens


<220>
<221>  primer_bind
<222>  (1)..(19)
<223>  Binding site of the SEQ ID NO:27 primer´s

<220>
<221>  variation
<222>  (36)..(36)
<223>  rs910416 polymorphism of ESR1 human gene: C=T

<220>
<221>  primer_bind
<222>  (38)..(53)
<223>  Binding site of the SEQ ID NO:62 primer´s

<220>
<221>  primer_bind
<222>  (130)..(149)
<223>  Binding site of the SEQ ID NO:28 primer´s

<400>  78

aggaaccgtg tcgctgtctc ggtagctgct ttacacgtgg tctcagtgcc ttgaggacgc   60

caggcgcaga ccagttttgg ggctcaaccc cagcaaggga agcatttcct tccaattgga  120

gggaagggct gatggtgcca aaattggag                                    149


<210>  79
<211>  469
<212>  DNA
<213>  Homo sapiens


<220>
<221>  primer_bind
<222>  (1)..(21)
<223>  Binding site of the A1 primer (SEQ ID NO:37)

<220>
<221>  variation
<222>  (357)..(357)
<223>  rs3798573 polymorphism of ESR1 human gene: A=G

<220>
<221>  primer_bind
<222>  (449)..(469)
<223>  Binding site of the A31 primer (SEQ ID NO:38)

<400>  79

cgcagtgctg acttaaaatg ttcttagtat gccaatgttg cttaaatatg aatgactgta   60

gcttctactt aaattggcaa ccgcaccaaa tataaactgc aaatgtttat agctcttatg  120

gaagtataaa agcaaaacaa attcttaaat taaatacaaa ctaagaggga aactgataat 180

aacaatgttt tgtgtaatta agttgctgtt tgcaacatgc ctggggcaga ctcctggcct 240

ctttgcatct gacatgagag gctaccaagg atgtggccac aactgggctc tcccagcact 300

tgcttgcgct cagaactgtg ccaaaacctt tgctcgccca gtgttctgca acgtccatca 360

tttggatttg gcaggaacac atcatttatg cattttgttt gcttctgtat ttagttgaga 420

tacttaaaat ataactgcta gattctaagg cattcataag catctgtga 469

<210> 80
<211> 289
<212> DNA
<213> Homo sapiens

<220>
<221> primer_bind
<222> (1)..(20)
<223> Binding site of the A4 primer (SEQ ID NO:39)

<220>
<221> variation
<222> (134)..(134)
<223> rs9397483 polymorphism of ESR1 human gene: A=G

<220>
<221> variation
<222> (191)..(191)
<223> rs3778087 polymorphism of ESR1 human gene: G=T

<220>
<221> variation
<222> (205)..(205)
<223> rs3778088 polymorphism of ESR1 human gene: G=A

<220>
<221> variation
<222> (245)..(245)
<223> rs3778089 polymorphism of ESR1 human gene: G=A

<220>
<221> primer_bind
<222> (269)..(289)
<223> Binding site of the A2 primer (SEQ ID NO:40)

<400> 80

ggagggttga tttgttgtga gtgtatctct ctttaaaaac ttttctaggg aaggacaacc 60

tcttccaaag actttgtttg ctttgtgtga ttggcatgtc cttccctggt cagtaatgag 120

cttgaaggaa gctactggac acgggactga acatggggtt ttgctatcac tctagctgtg 180

cctttctcta gtgatcaagg agatgttggt tcagaaagtg ccctgtctaa tcccactaag 240

gcccgaagga ccaagtgctg tctactctcc agattctctg caacagaaa          289

<210>  81
<211>  4800
<212>  DNA
<213>  Homo sapiens

<220>
<221>  primer_bind
<222>  (1)..(21)
<223>  Binding site of the A1 primer (SEQ ID NO:37)

<220>
<221>  variation
<222>  (357)..(357)
<223>  rs3798573 polymorphism of ESR1 human gene: A=G

<220>
<221>  primer_bind
<222>  (861)..(880)
<223>  Binding site of the ED-F primer (SEQ ID NO:46)

<220>
<221>  variation
<222>  (963)..(963)
<223>  rs3020375 polymorphism of ESR1 human gene: A=C

<220>
<221>  misc_feature
<222>  (963)..(3228)
<223>  absent nucleotides in the allele carrying the ESR1-NCD1 deletion

<220>
<221>  primer_bind
<222>  (973)..(989)
<223>  Binding site of the ED-S primer (SEQ ID NO:48)

<220>
<221>  primer_bind
<222>  (1101)..(1120)
<223>  Binding site of the ED-RW primer (SEQ ID NO:47)

<220>
<221>  primer_bind
<222>  (1696)..(1716)
<223>  Binding site of the F1 primer (SEQ ID NO:41)

<220>
<221>  primer_bind
<222>  (2178)..(2198)
<223>  Binding site of the F2 primer (SEQ ID NO:42)

<220>
<221>  primer_bind
<222>  (2709)..(2729)
<223>  Binding site of the R1 primer (SEQ ID NO:43)

<220>
<221>  primer_bind
<222>  (3322)..(3343)
<223>  Binding site of the R2 primer (SEQ ID NO:44)

<220>
<221>  primer_bind
<222>  (3892)..(3913)
<223>  Binding site of the R3 primer (SEQ ID NO:45)

<220>
<221>  primer_bind
<222>  (4512)..(4531)
<223>  Binding site of the A4 primer (SEQ ID NO:39)

<220>
<221>  variation
<222>  (4645)..(4645)
<223>  rs9397483 polymorphism of ESR1 human gene: A=G

<220>
<221>  variation
<222>  (4702)..(4702)
<223>  rs3778087 polymorphism of ESR1 human gene: G=T

<220>
<221>  variation
<222>  (4716)..(4716)
<223>  rs3778087 polymorphism of ESR1 human gene: G=A

<220>
<221>  variation
<222>  (4756)..(4756)
<223>  rs3778089 polymorphism of ESR1 human gene: G=A

<220>
<221>  primer_bind
<222>  (4780)..(4800)
<223>  Binding site of the A2 primer (SEQ ID NO:40)

<400>  81

cgcagtgctg acttaaaatg ttcttagtat gccaatgttg cttaaatatg aatgactgta   60

gcttctactt aaattggcaa ccgcaccaaa tataaactgc aaatgtttat agctcttatg  120

gaagtataaa agcaaaacaa attcttaaat taaatacaaa ctaagaggga aactgataat  180

aacaatgttt tgtgtaatta agttgctgtt tgcaacatgc ctggggcaga ctcctggcct  240

ctttgcatct gacatgagag gctaccaagg atgtggccac aactgggctc tcccagcact  300

tgcttgcgct cagaactgtg ccaaaacctt tgctcgccca gtgttctgca acgtccatca  360

tttggatttg gcaggaacac atcatttatg cattttgttt gcttctgtat ttagttgaga  420

tacttaaaat ataactgcta gattctaagg cattcataag catctgtgaa aaatgtgtta  480

cttaggaaac agttcagggt tccatggatt atctagattg gtgagtctta agactgacca  540

cacaattcat ctgggggtgt ggaggagggg agctttgaga aaaatctctg tatgagcacc 600

cacacccaga gattcctatg caattatccc agagtggtgc cttaatatca acatttgttt 660

aaaaccatcc cccaagacta aaatttgtga ccgggattta gaaccattga cttaattgat 720

gcagaacact ctgatattaa tgtcttcatg tcttttggaa tgtcattaag acaaaagcat 780

aaaattttaa aattcttaaa gacaatttaa agacctatga gaatccatct ttaattctca 840

ggagcgtgtg gaacctagtt tgaaaactac tggcatcgac aattgaattt ccactaaaat 900

aaaatagctc tctagtatat tacaaaacta cccattctgc aaactgcagg ggagctactg 960

ataatgcttg gaactgtgcc aggcactgcc tgcataaaaa tgagtaaggt ccacttcctc 1020

catggactgg gttgggtagg aggcaaagat aattaaccaa ttattttaat attatgagtt 1080

cagggttgta atgaaagtaa gtattgagtg ctgcagagac ccagcaaagg agtccttagc 1140

tgagaactga tcctttgctg agtgacgagg acattattga accgccaggc ttgatggcat 1200

gttgcatggt aacagaccag tacaccaaac agcaggagct gtagcagaga aagagtttaa 1260

taattgtgtg gcagccaaat aaggagacag aaggaaacct taaatccacc tcttgaaaaa 1320

gtctgggact aggcttttta agagaattct ggcaagaagg aggctgagga gctgggggta 1380

actgattggt taggacatag gagaggagac aataaggata tagaaactcc attcttgcac 1440

tgggtcagtt cctcggaggg ggtcctcact ctggctggca gcagtgaacc cattggaatg 1500

caggatctga aaaatatctc aaaatggcaa acttgagggg ttttttttag catcaaagat 1560

gttatctata gaagttagga cattgtgaca ggggctacgt gactttgaag tgttaagtgg 1620

ctgtcagaaa gtgagctatt gcagcggtcc ccaacctttt tggtaccagg gaccagcttc 1680

atgaaagaaa atttttccac agatggggtg gtgtagggtg gggtggggga tagcttcggg 1740

atgaaactga tccacctcag atcatcaggc attagttaga ttctcatgag gggcacacag 1800

cccagatccc tcacatgggc agttcacagt agggttcaca gccctatgag gttctaatgc 1860

cactgctgat ctgacaggtg gcagagctca ggcagtaatg cttccttgcc caccactcac 1920

ctcttgctgt gtggctcagt tcctgacaga ttacggactg gtaccagttt gcaacccagg 1980

cagctgggga cctctgggct gtagggtagg ctggttaatg cttagctgtg tttctattca 2040

aagcttatgc ttttgtttaa aacctagtaa tttggttttg ttaattttat gaagatgatt 2100

tcaaggatga gtaggattag ctggagtcag aaggacacaa gggaatttct ggcaacaaca 2160

ttaaactacc agtgaatgtc tcatcaagtc actggacctc taattctaag cagtactgtc 2220

aaccttatag gttatgatgt ataagatagt gttgtgatgt ataagatagt gcaaaaacct 2280

aagttaatat caccatttct gtagtactct ctatttcaaa ggaaaaaatc ttcagcagtc 2340

taatatccta ccaaaatttt gaagtcaaga cctccctaca ctaatcaaag aactagaggt 2400

gcaatcaggc catatatttg gaaaggccat aggtgccctc cctctgtcct tgttcccagc 2460

atactaaact atagtctaaa agactttaaa acttgaactg aagaaaacct taaggaaaca 2520

acaacaaaat tccatttatt tcctgagccc aaaagtagaa taattgtttt ttaatgaatt 2580

tttctttccc tgtaagacat actatgaatt gttcaatggg tattcaaagc atatgtaata 2640

gaatatgtat gctttcctga aaaattgaaa tggaacatca aactgttctg taaagttttg 2700

agtctagacc aaactcacag cacttagcca gaatatgtac atctgcatct gccactcatg 2760

gccccatcgc tccctccaac tgcttctctt gggctgaagg atggaaaagc aaactccctg 2820

cgttgagttc catcttgccc tgcatggcct cccccatggg gtcattgagc tccccaccag 2880

cttccacctc ttccagcact ttccgccaga gggcccgtgg ctgcttttgc agactgaaat 2940

tgattgtggt tgattcagct ccatttcctc tgagttttta ctttagatgt gctttttagg 3000

ttgcattttt cttctggcat ataagaaaga cagcaatatt ccatggaaca gagtgatgtc 3060

tgtggctttg tcaaccgttg tttgttagct tgatcaccat ttattgagtg tttactactt 3120

tccaggcatt gtgctaagtg gtagaaatac aaagttgcct ttacttgatt catctgtaaa 3180

atagagagag tagtagttcc tacagtgtgg tttatgtgag gcatagggac tgctccctga 3240

aacctaggga caactcccta gcagcgaccc agtgctcata tcaatagtgg taacaattag 3300

gaaggcagct cctacctcaa agacctcatg atctgatggt aaagacagat ataacaagaa 3360

aattattctt tttaaatata aaattgtgat ataaaaatag ttttcactaa attggatttt 3420

tatgaaattt acagaaacaa aataataaat taaggaaata taaaaccacc tgacattagc 3480

caaccatgtt gatgttctga ttatgttact gcaaaggagt gtagggttaa gaacgaggga 3540

ctttgattcc aagatcaaat ccctttctgc tatttcttag ctgtctgact ttgggcaagt 3600

tgcttaaacc ctttgtgctt ctgtgtgctt gcccatgaga tggagattat aatagcgtct 3660

acctcatagg gctattttta atacaagtta gctcttagaa caatacctac ttaaactaac 3720

aatttgtatt aggtactgtt ttattctcca tccgtactgt gtatgcatat attcatattt 3780

atatttacat attctaatta ggattatata catgtacttt ctttaaaaat aagattttaa 3840

ataactatat aatcagtcat gatgatacaa catgatttat tcaacaaatc tgatgctgtt 3900

aaacacgtaa gtgatttctc tttcaggatt ataaataaca tttgttttgc atttttttca 3960

tacatctttt gctcactttt cattgttact ttgtatatgt tgctagaaat tgatttgctg 4020

gtgaaaaggg tataaacatt tttaaggctc ttgctacata ttgcctgctc ttcatacata 4080

ttgcctactg ccccaggaaa ctatcaatta cacttttacc aagagtgtat gaaagcattc 4140

acatgaatgt aaccttatca tgcaatgcgg gccatgagac agaggagagt tatggagggt 4200

agacggcttc cccgctgctc agccactgcc ctctcctcta aaagagccac agtcaggaat 4260

ttctacatag atcctattat aaacatctcc ttttgaaaca aattatcttc tctcagagca 4320

ttttcttcat tttagtatct atataggtcc ttgttatgtt cattttaagt tttgtgacct 4380

gcactgacac aatgctgatg aactgcttat tccaggcagt ctgagttctc aaagcatttg 4440

cattttatgt cttatcctct gttttgggct ctttgccact gatttttttt taagtgatat 4500

tttaagaaag aggagggttg atttgttgtg agtgtatctc tctttaaaaa cttttctagg 4560

gaaggacaac ctcttccaaa gactttgttt gctttgtgtg attggcatgt ccttccctgg 4620

tcagtaatga gcttgaagga agctactgga cacgggactg aacatggggt tttgctatca 4680

ctctagctgt gcctttctct agtgatcaag gagatgttgg ttcagaaagt gccctgtcta 4740

atcccactaa ggcccgaagg accaagtgct gtctactctc cagattctct gcaacagaaa 4800